# EUROPEAN PATENT SPECIFICATION

(11) **EP 3 292 205 B1**
(45) Date of publication and mention of the grant of the patent: **02.08.2023**
(21) Application number: 16725663.5
(22) Date of filing: 06.05.2016
(51) Int. Cl.: C12N 15/82

(54) **METHODS AND COMPOSITIONS FOR THE PRODUCTION OF UNREDUCED, NON-RECOMBINED GAMETES AND CLONAL OFFSPRING**
VERFAHREN UND ZUSAMMENSETZUNGEN ZUR HERSTELLUNG VON NICHTREDUZIERTEN, NICHTREKOMBINIERTEN GAMETEN UND KLONALEN NACHKOMMEN
PROCÉDÉS ET COMPOSITIONS DE PRODUCTION DE GAMÈTES NON RÉDUITS, NON RECOMBINÉS ET DESCENDANCE CLONALE

(30) Priority: 06.05.2015 US 201562157687 P
(43) Date of publication of application: 14.03.2018
(73) Proprietor: Pioneer Hi-Bred International, Inc., Johnston, Iowa 50131-1014 (US); Universität Zürich, 8006 Zürich (CH); Corteva Agriscience LLC, Indianapolis, IN 46268 (US)
(72) Inventor: FOX, Tim, W., Des Moines, IA 50301 (US); ALBERTSEN, Marc, C., Grimes, IA 50111 (US); WILLIAMS, Mark, E., Newark, DE 19711 (US); LAWIT, Shai, J., Urbandale, IA 50323 (US); CHAMBERLIN, Mark, A., Windsor Heights, IA 50324 (US); GROSSNIKLAUS, Ueli, 8126 Zumikon (CH); BRUNNER, Gion, Arco, 8810 Horgen (CH); CHUMAK, Nina, 8134 Adliswil (CH); DE ASIS, Joana, Bernardes, 4127 Birsfelden (CH); PASQUER, Frederique, 8810 Horgen (CH)
(74) Representative: J A Kemp LLP
(86) International application number: PCT/US2016/031271
(87) International publication number: WO 2016/179522

(56) References cited:
- EP-A1- 2 208 790
- WO-A1-2013/065045
- WO-A1-2013/065046
- JP-A- 2010 154 848
- US-A1- 2004 123 343
- US-A1- 2007 020 621
- US-A1- 2007 044 171
- US-A1- 2007 209 085
- US-A1- 2009 094 717
- US-A1- 2013 117 886
- L. BROWNFIELD ET AL: "Unreduced gamete formation in plants: mechanisms and prospects", JOURNAL OF EXPERIMENTAL BOTANY, vol. 62, no. 5, 25 November 2010 (2010-11-25), pages 1659-1668, XP55228862, GB ISSN: 0022-0957, DOI: 10.1093/jxb/erq371
- M. L. HAND ET AL: "The Genetic Control of Apomixis: Asexual Seed Formation", GENETICS, vol. 197, no. 2, 1 June 2014 (2014-06-01), pages 441-450, XP55285206, US ISSN: 0016-6731, DOI: 10.1534/genetics.114.163105

## Description

### FIELD OF THE DISCLOSURE

The present disclosure relates to the field of plant molecular biology, more particularly to plant female reproductive biology, and methods and compositions of altering reduction and recombination during female sporogenesis (meiosis in the ovule).

### BACKGROUND OF THE DISCLOSURE

Apomixis refers to asexual reproduction leading to the production of seeds without fertilization, that is to offspring which is genetically identical to the mother plant (Koltunow, et al., (1995) Plant Physiol. 108:1345-1352; Koltunow and Grossniklaus (2003) Ann. Rev. Plant Devl. 54: 547-74; Ravi, et al., (2008) Nature 451:1121-4). Apomixis is thus a reproductive process that bypasses female meiosis and syngamy to produce embryos identical to the maternal parent. Apomixis increases the opportunity for developing superior gene combinations and facilitates the rapid incorporation of desirable traits. Apomixis not only provides reproductive assurance, but also avoids a loss of heterozygosity in the offspring because the offspring maintains the parental genotype. Apomixis therefore avoids the effects of loss of vigor due to inbreeding and may additionally confer some advantages because of the heterosis effects.

At the species level, apomixis occurs in less than 1% of the species. Apomixis occurs in many wild species and in a few agronomically important species such as citrus and mango, but not in any of the major cereal crops (Eckhardt, (2003) Plant Cell 15:1449-1501). One form of apomixis is adventitious embryony, where embryos are formed directly out of somatic tissues within the ovules outside the embryo sac. Adventitious embryony usually occurs in parallel to normal sexual reproduction. A second form of apomixis is diplospory, which displaces sexual reproduction. In diplospory, an non-reduced egg cell is formed which then goes through a process called parthenogenesis (embryogenesis without fertilization) to form an embryo. A third form of apomixis is apopsory, which like adventitious embryony takes place in tissues outside the sexual embryo sac. Apospory, involves the formation of an asexual, non-reduced and non-recombined embryo sac that - as in diplospory - goes through parthenogenesis to form a clonal embryo. All three forms of apomixis rely on the production of an embryo without fertilization (parthenogenesis). Because apomixis offers the promise of the fixation and indefinite propagation of a desired genotype, there is a great deal of interest in engineering this ability to produce clonal seeds into crops, especially cereals (Spillane, et al., (2001) Sex. Plant Reprod. 14: 179-87; Spillane, et al., (2004) Nat. Biotechnol. 22:687-91).

A molecular approach to engineer apomixis in commercial plant lines is highly desirable as a self-reproducing hybrid (SRH) system can result in a significant reduction in hybrid seed production costs. In other crops, such as soybean, it can result in the production, increase and sale of hybrid seeds, something which has not been done. There are three key components to apomixis: (1) avoidance of meiosis; (2) parthenogenic development of the embryo; and (3) achieving functional endosperm formation (Grossniklaus et al., (1998), in The Flowering of Apomixis: from Mechanisms to Genetic Engineering, eds. Savidan, Carman, Dresselhaus (CIMMYT, IRD, European Commission DG VI (FAIR), Mexico, DF), pp. 168-211; Grossniklaus, (2001), in Advances in Hybrid Rice Technology. Proceedings of the 3rd International Symposium on Hybrid Rice 1996, eds. Virmani, Siddiq, Muralidharan (International Rice Research Institute, Manila), pp. 187-211).

### BRIEF SUMMARY OF THE DISCLOSURE

The development of self-reproducing hybrids (SRH), or synthetic apomixis without reduction in meiosis by modifying *Non-reductive in female4 (Nrf4)* activity is disclosed herein.

The invention provides a method of producing a plant, the method comprising:
disrupting by genome editing, transposon tagging, or mutagenizing an endogenous *Nrf4* gene in a plant or plant cell, wherein the plant cell is an ovule primordia, ovule, female gametophyte, or female gamete, thereby producing viable non-reduced, or viable non-reduced and non-recombined, gametes; and
regenerating a plant having the disruption of the endogenous *Nrf4* gene:
   (A) wherein the endogenous *Nrf4* gene comprises a polynucleotide selected from the group consisting of:
      (a) a polynucleotide having at least 80% sequence identity, as determined by the GAP algorithm under default parameters, to the full length sequence of a polynucleotide selected from the group consisting of SEQ ID NOs: 3, 4, 5, 6, 7, 8, 10, 12, 13, 17, 18, 22, 28, and 29, and wherein the polynucleotide encodes a polypeptide that has the function of reducing, or reducing and recombining, female sporocytes, female gametophytes, or female gametes during meiosis;
      (b) a polynucleotide having at least 90% sequence identity, as determined by the GAP algorithm under default parameters, to the full length sequence of a polynucleotide selected from the group consisting of SEQ ID NOs: 3, 4, 5, 6, 7, 8, 10, 12, 13, 17, 18, 22, 28, and 29, and wherein the polynucleotide encodes a polypeptide that has the function of reducing, or reducing and recombining, female sporocytes, female gametophytes, or female gametes during meiosis;
      (c) a polynucleotide selected from the group consisting of SEQ ID NOs: 3, 4, 5, 6, 7, 8, 10, 12, 13, 17, 18, 22, 28, and 29;
      (d) a polynucleotide that is a fragment comprising at least 450 contiguous nucleotides of the polynucleotide of (a), (b), or (c), and wherein the polynucleotide encodes a polypeptide that has the function of reducing, or reducing and recombining, female sporocytes, female gametophytes, or female gametes during meiosis; or
(B) wherein the endogenous *Nrf4* gene encodes for a Nrf4 polypeptide selected from the group consisting of:
   (a) a polypeptide comprising an amino acid sequence being identical to or having at least 80% identity with SEQ ID NOs: 1, 2, 9, 14, 16, 20, 24, 25 or 27, or an ortholog thereof, and wherein the polypeptide has the function of reducing, or reducing and recombining, female sporocytes, female gametophytes, or female gametes during meiosis;
   (b) a polypeptide comprising an amino acid sequence being identical to or having at least 90% identity with SEQ ID NOs: 1, 2, 9, 14, 16, 20, 24, 25 or 27, or an ortholog thereof, and wherein the polypeptide has the function of reducing, or reducing and recombining, female sporocytes, female gametophytes, or female gametes during meiosis;
   (c) a polypeptide comprising an amino acid sequence selected from SEQ ID NOs: 1, 2, 9, 14, 16, 20, 24, 25 or 27, or an ortholog thereof; and
   (d) a polypeptide comprising an amino acid sequence which is a fragment comprising at least 150 contiguous amino acids of the amino acid sequence of (a), (b), or (c), and wherein the polypeptide has the function of reducing, or reducing and recombining, female sporocytes, female gametophytes, or female gametes during meiosis.

The invention further provides a nucleic acid construct comprising a nucleotide sequence encoding an element that silences *Nrf4* activity in a plant cell selected from the group consisting of an ovule primordia, ovule, female gametophyte, or female gamete; wherein:
(A) the element comprises at least 19 nucleotides of any one of SEQ ID NOs: 3, 4, 5, 6, 7, 8, 10, 12, 13, 17, 18, 22, 28, and 29 or a complement thereof; or
(B) the element comprises a nucleotide sequence that hybridizes under stringent conditions to a full length complement of a nucleotide sequence of any one of SEQ ID NOs: 3, 4, 5, 6, 7, 8, 10, 12, 13, 17, 18, 22, 28, and 29;
wherein the construct further comprises a heterologous promoter functional in plants operably linked to the element.

Yet further provided by the invention is a plant cell transformed with the construct as described above; and a plant comprising said plant cell. In some embodiments of the invention, the plant comprises viable non-reduced, or non-reduced and non-recombined, gametes compared to a control plant; or the plant is Arabidopsis, maize, wheat, rice, sorghum, barley, oat, lawn grass, rye, soybean, Brassica, sunflower, millet, sugarcane, cotton, safflower, tobacco, or alfalfa. The invention also provides a seed from said plant.

The invention further provides a method of decreasing expression of one or more *Nrf4* genes in the plant by introducing into the plant by transformation the nucleic acid construct as described above, wherein the decreased expression of the one or more *Nrf4* genes is in an ovule primordia, ovule, female sporocyte, female gametophyte, or female gamete of the plant.

Yet further provided by the invention is an expression cassette comprising in operable linkage to a heterologous nucleotide sequence of interest a nucleic acid molecule comprising a polynucleotide selected from the group consisting of:
(a) a polynucleotide sequence comprising the polynucleotide sequence SEQ ID NOs: 11, 15, 19, 21, 23 and 26;
(b) a polynucleotide sequence comprising a fragment comprising at least 16 contiguous nucleotides of the polynucleotide sequence of SEQ ID NOs: 11, 15, 19, 21, 23 and 26, wherein the polynucleotide sequence initiates transcription in a plant cell;
(c) a polynucleotide which is complementary to the polynucleotide of (a) or (b); and
(d) a polynucleotide that hybridizes under stringent conditions to the polynucleotide of (a), (b) or (c);
wherein said polynucleotide initiates transcription in a cell of an ovule primordia, ovule, female sporocyte, female gametophyte, or female gamete.

The invention further provides a plant comprising the expression cassette as described above.

Yet further provided by the invention is a method for expressing a polynucleotide sequence in a plant or a plant cell, said method comprising introducing into the plant or the plant cell by transformation an expression cassette comprising a promoter operably linked to a polynucleotide sequence of interest, wherein said promoter comprises any of the polynucleotide sequences as described above.

The invention further provides an expression cassette comprising a heterologous promoter operably linked to a polynucleotide comprising a member selected from the group consisting of:
(a) a polynucleotide that encodes the polypeptide of SEQ ID NOs: 1, 2, 9, 14, 16, 20, 24, 25 or 27;
(b) a polynucleotide comprising the sequence set forth in SEQ ID NOs: 3, 4, 5, 6, 7, 8, 10, 12, 13, 17, 18, 22, 28 or 29;
(c) a polynucleotide comprising at least 300 nucleotides in length which hybridizes under stringent conditions to a polynucleotide of (a) or (b), wherein the conditions include hybridization in 40 to 45% formamide, 1 M NaCl, 1% SDS at 37°C and a wash in 0.5 X to 1 X SSC at 55 to 60°C;
(d) a polynucleotide having at least 80% sequence identity to SEQ ID NOs: 3, 4, 5, 6, 7, 8, 10, 12, 13, 17, 18, 22, 28 or 29, wherein the % sequence identity is based on the entire coding region and is determined by BLAST 2.0 under default parameters, wherein the polynucleotide encodes a polypeptide that confers the function of reducing, or reducing and recombining, female sporocytes, female gametophytes, or female gametes during meiosis; and
(e) a polynucleotide fully complementary to a polynucleotide of any one of (a) to (d).

Yet further provided by the invention is an isolated polypeptide selected from the group consisting of:
(a) a polypeptide comprising any one of SEQ ID NOs: 9, 14, 16, 20, 24, 25 or 27, wherein said polypeptide confers the function of reducing, or reducing and recombining, female sporocytes, female gametophytes, or female gametes during meiosis;
(b) a polypeptide that is at least 80% identical to the amino acid sequence of any of SEQ ID NOs: 9, 14, 16, 20, 24, 25 or 27, wherein said polypeptide confers the function of reducing, or reducing and recombining, female sporocytes, female gametophytes, or female gametes during meiosis;
(c) a polypeptide that is encoded by a nucleic acid molecule comprising a nucleotide sequence that is at least 80% identical to any one of SEQ ID NOs: 8, 10, 12, 17, 18, 28 or 29 or a complement thereof, wherein said polypeptide confers the function of reducing, or reducing and recombining, female sporocytes, female gametophytes, or female gametes during meiosis;
(d) a polypeptide that is encoded by a nucleic acid molecule that hybridizes with a nucleic acid probe consisting of the nucleotide sequence of any of SEQ ID NOs: 8, 10, 12, 17, 18, 28 or 29, or a complement thereof following at least one wash in 0.2X SSC at 55°C for 20 minutes, wherein said polypeptide confers the function of reducing, or reducing and recombining, female sporocytes, female gametophytes, or female gametes during meiosis; and
(e) a fragment comprising at least 150 consecutive amino acids of any of SEQ ID NOs: 9, 14, 16, 20, 24, 25 or 27, wherein said polypeptide confers the function of reducing, or reducing and recombining, female sporocytes, female gametophytes, or female gametes during meiosis.

The invention further provides a plant cell transformed with the expression cassette as described above, and a plant regenerated from said plant cell, wherein said plant comprises the expression cassette. In some embodiments of the invention, the plant is maize, wheat, rice, sorghum, barley, oat, lawn grass, rye, soybean, Brassica, sunflower, millet, sugarcane, cotton, safflower, tobacco, or alfalfa. The invention also provides a seed from said plant.

Yet further provided by the invention is a method of increasing the expression of one or more *Nrf4* genes in a plant cell by introducing into the plant by transformation:
(A) a polynucleotide comprising a member selected from the group consisting of:
   (a) a polynucleotide that encodes the polypeptide of SEQ ID NOs: 1, 2, 9, 14, 16, 20, 24, 25 or 27;
   (b) a polynucleotide comprising the sequence set forth in SEQ ID NOs: 3, 4, 5, 6, 7, 8, 10, 12, 13, 17, 18, 22, 28 or 29;
   (c) a polynucleotide comprising at least 300 nucleotides in length which hybridizes under stringent conditions to a polynucleotide of (a) or (b), wherein the conditions include hybridization in 40 to 45% formamide, 1 M NaCl, 1% SDS at 37°C and a wash in 0.5 X to 1 X SSC at 55 to 60°C;
   (d) a polynucleotide having at least 80% sequence identity to SEQ ID NOs: 3, 4, 5, 6, 7, 8, 10, 12, 13, 17, 18, 22, 28 or 29, wherein the % sequence identity is based on the entire coding region and is determined by BLAST 2.0 under default parameters, wherein the polynucleotide encodes a polypeptide that confers the function of reducing, or reducing and recombining, female sporocytes, female gametophytes, or female gametes during meiosis; and
   (e) a polynucleotide fully complementary to a polynucleotide of any one of (a) to (d);
(B) any of the polypeptides as described above; or
(C) the expression cassette as described above.

Further aspects and preferred embodiments are defined in the dependent claims. Any aspects, embodiments and examples of the present disclosure which do not fall under the scope of the appended claims do not form part of the invention and are merely provided for illustrative purposes.

### SUMMARY OF TECHNICAL TEACHINGS

Disclosed herein are methods of producing viable non-reduced, or viable non-reduced and non-recombined, gametes. The method comprises disrupting an endogenous *Nrf4* gene. In an aspect, the method comprises disrupting an endogenous *Nrf4* gene.in a plant or plant cell, wherein the plant cell is an ovule primordia, ovule, female gametophyte, or female gamete, thereby producing viable non-reduced, or viable non-reduced and non-recombined, gametes.

Provided herein is a nucleic acid construct that has a nucleotide sequence with an element that silences *Nrf4* activity. Methods of decreasing *Nrf4* activity in a plant is described. In some aspects of the disclosure, the method includes decreasing the expression of one or more *Nrf4* genes in a plant cell, for example, in an ovule primordia, ovule, female gametophyte, or female gamete. In some aspects of the disclosure, the methods include disrupting an endogenous *Nrf4* gene in a plant.

In other aspects of the disclosure, methods of producing viable non-reduced, non-recombinant gametes are provided. A non-naturally occurring plant that has a disruption in an endogenous *Nrf4* gene and develops ovules that include non-reduced, recombined gametes and/or non-reduced, non-recombined clonal gametes are described herein as well as seeds obtained from such a plant.

A further aspects of the disclosure includes methods of maintaining heterozygosity in a progeny plant that has the same genotype as the parent plant by regenerating the progeny plant from a parent plant that has non-reduced, non-recombinant clonal gametes. In some examples, the endogenous *Nrf4* gene is disrupted in the parent and progeny plant.

Also described herein are methods of expressing a sequence of interest in a plant or a plant cell. In some aspects of the disclosure, the nucleotide sequence is expressed in an ovule-preferred manner in a plant. In some aspects of the disclosure, the nucleotide sequence is expressed by a *Nrf4* promoter, variant or fragment thereof.

### BRIEF DESCRIPTION OF THE DRAWINGS

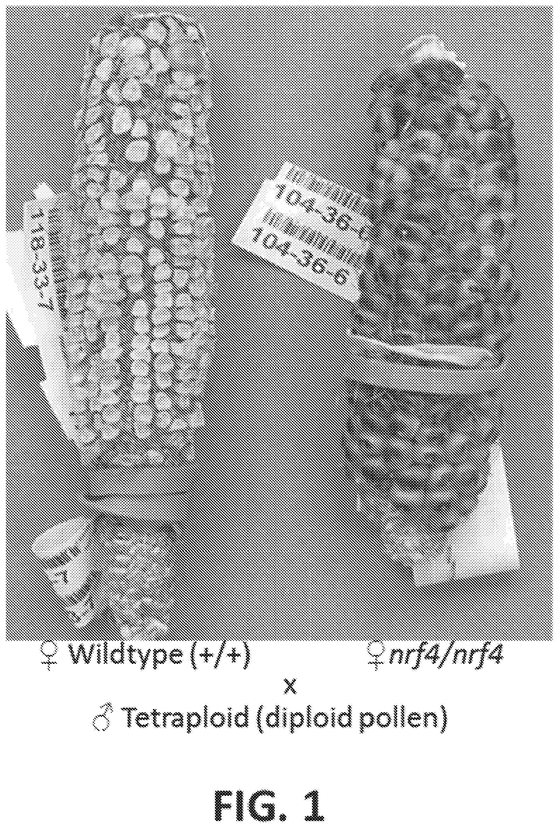

**FIG. 1****.** Ear phenotype of wild-type and *nrf4*/*nrf4* mutant females crossed with a tetraploid male that produces diploid pollen. Plump kernels result from unreduced and shrunken kernels from reduced female gametophytes, respectively.

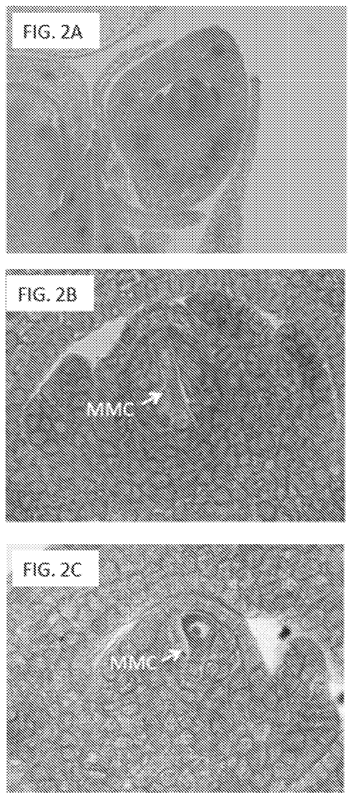

**FIG. 2****.** Localization of *Nrf4* transcripts in wild-type ovules of inbred line W22 determined by *in situ* hybridization. (Fig. 2A) *Nrf4* transcript is abundant in the nucellus and integuments of early stage ovules. In the developing female sporocyte (megaspore mother cells - MMC) the *Nrf4* signal was detected either at an intermediate (FIG. 2B) or high level (FIG. 2C). Images were taken with 10x magnification (FIG. 2A) or 40x magnification (FIG. 2B and FIG. 2C).

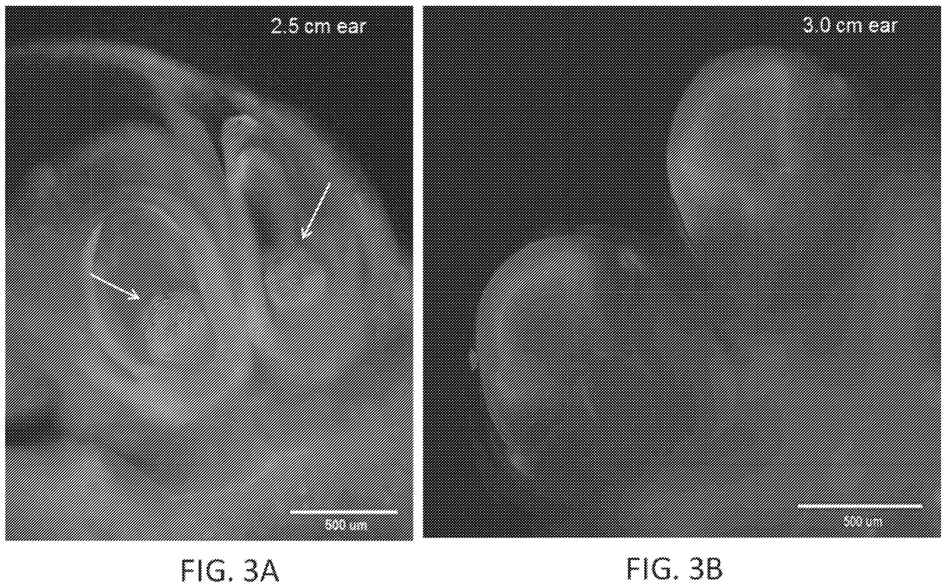

**FIG. 3****.** Fluorescence image of a pair of maize florets of a young pre-fertilization ear (2.5 cm long) (FIG. 3A) and a young pre-fertilization ear (3.0 cm long) (FIG. 3B). These are T1 transgenic plants expressing the ZsGreen fluorescent protein (php56985) under the control of the *ZmNRF4* promoter. (FIG. 3A) ZsGreen-positive expression is observed in the pre-meiotic ovules (arrows). Weak ZsGreen expression is also noted in the palea and lemma surrounding the ovule primordia, but not the glumes. (FIG. 3B) ZsGreen-positive expression is observed in the pre-meiotic ovules, their nucellus and surrounding ovular tissues. No expression was noted in the palea, lemma or glumes surrounding these slightly older ovules (compare with A).

### DETAILED DESCRIPTION

The invention is defined in the appended claims. Any aspects, embodiments and examples of the present disclosure which do not fall under the scope of the appended claims do not form part of the invention and are merely provided for illustrative purposes.

The present disclosure now will be described more fully hereinafter with reference to the accompanying drawings, in which some, but not all embodiments of the disclosure are shown.

### I. Overview

Compositions and methods for preventing or decreasing meiotic reduction in a female gametophyte or female gamete by modulating *Nrf4* expression or activity is described herein. As used herein, *"Nrf4* activity" refers to one or more of the following activities: (i) causing the reduction in meiosis during plant reproduction, for example, in an ovule, female sporocyte, female gametophyte, or female gamete, (ii) the recombination during the reduction in meiosis in a plant ovule, for example, in a female sporocyte. *Nrf4* activity may occur *in vitro* or *in vivo.*

Manipulating *Nrf4* expression and/or activity provides an opportunity to alter chromosome reduction during meiosis. Decreasing *Nrf4* expression and/or activity in a plant may be used to create a plant with a non-reduced gamete, i.e. where the gamete retains a full chromosome set and does not have its genetic material reduced in half. Moreover, decreasing *Nrf4* expression and/or activity in a plant may be used prevent recombination during sporogenesis. Provided herein are methods and compositions for producing plants having viable non-reduced, or non-reduced and non-recombined, gametes as well methods and compositions for maintaining heterozygosity in a progeny plant.

### II. Various Methods of Use

Methods and compositions for decreasing *Nrf4* activity are provided. Disclosed herein are methods and compositions for decreasing *Nrf4* activity in a female sporocyte, female gametophyte or female gamete in a plant. The method can include decreasing the expression level of one or more *Nrf4* sequences, for example, gene, cDNA, polypeptide, in the female plant germline, for instance a female sporocyte, female gametophyte or female gamete. In some aspects of the disclosure, methods and compositions are provided which employ a silencing element that decreases the level of expression and/or activity of *Nrf4.* Targets for *Nrf4* polynucleotides include wild type *Nrf4* polynucleotide or Nrf4 polypeptide sequences, *Nrf4* variant polynucleotides, Nrf4 variant polypeptides, cognate promoter sequences, ortholog sequences, variants or fragments thereof. Silencing elements that decrease the expression of one or more of the *Nrf4* target sequences and thereby decrease or inhibit the reduction and recombination in meiosis, e.g. *Nrf4* activity, can be designed in view of these target polynucleotides.

Assays and techniques that measure reduction or recombination in meiosis include microscopic examination of spores, flow cytometry, crossing plants of various ploidy, and marker analysis, see, for example, Example 1 herein.

### III. Target Sequences

As used herein, a "target sequence" or "target polynucleotide" comprises any sequence that one desires to decrease the level of expression. In specific aspects of the disclosure, decreasing the level of the target sequence in the female sporocyte, the female gametophyte or female gamete prevents or decreases reduction and/or recombination in meiosis. Non-limiting examples of target sequences include those in Table 1 and set forth in the sequence listing, any wild type *Nrf4* polynucleotide or Nrf4 polypeptide sequences, *Nrf4* variant polynucleotides, Nrf4 variant polypeptides, cognate promoter sequences, ortholog sequences, variants or fragments thereof. As exemplified elsewhere herein, decreasing the level of expression of one or more of these target sequences results in the non-reduction and non-recombination during meiosis in the plant female sporocyte, female gametophyte or female gamete.

### IV. Silencing Elements

By "silencing element" is intended a polynucleotide that is capable of reducing or eliminating the level or expression of a target polynucleotide or the polypeptide encoded thereby. The silencing element employed can decrease or eliminate the expression level of the target sequence by influencing the level of the target RNA transcript or, alternatively, by influencing translation and thereby affecting the level of the encoded polypeptide. Methods to assay for functional silencing elements that are capable of decreasing or eliminating the level of a sequence of interest are disclosed elsewhere herein. A single polynucleotide employed in the methods can comprise one or more silencing elements to the same or different target polynucleotides. The silencing element can be produced *in vivo* (i.e., in a host cell such as a plant or microorganism) or *in vitro.*

In specific aspects of the disclosure, the target sequence is endogenous to the plant. In other aspects of the disclosure, while the silencing element regulates non-reduction and non-recombination of meiosis, preferably the silencing element has no effect on the parts of the plant that do not constitute the female germline (female sporocyte, female gametophyte, and female gamete).

As discussed in further detail below, silencing elements can include, but are not limited to, a sense suppression element, an antisense suppression element, a double stranded RNA, a siRNA, an amiRNA, a miRNA, or a hairpin suppression element. Non-limiting examples of silencing elements that can be employed to decrease expression of these target sequences or additionally sequences targeting genes involved in recombination comprise fragments and variants of the sense or antisense sequence or consists of the sense or antisense sequence of any of the sequences in Table 1, set forth in the sequence listing, wild type *Nrf4* polynucleotide or Nrf4 polypeptide sequences, *Nrf4* variant polynucleotides, Nrf4 variant polypeptides, cognate promoter sequences, ortholog sequences, variants or fragments thereof. The silencing element can further comprise additional sequences that advantageously effect transcription and/or the stability of a resulting transcript. For example, the silencing elements can comprise at least one thymine residue at the 3' end. This can aid in stabilization. Thus, the silencing elements can have at least 1, 2, 3, 4, 5, 6, 7, 8, 9, 10 or more thymine residues at the 3' end. As discussed in further detail below, enhancer suppressor elements can also be employed in conjunction with the silencing elements disclosed herein.

By "decreases" or "decreasing" the expression level of a polynucleotide or a polypeptide encoded thereby is intended to mean, the polynucleotide or polypeptide level of the target sequence is lower than the polynucleotide level or polypeptide level of the same target sequence in an appropriate control in which the silencing element is not introduced. In particular aspects of the disclosure, decreasing the polynucleotide level and/or the polypeptide level of the target sequence results in less than 95%, less than 90%, less than 80%, less than 70%, less than 60%, less than 50%, less than 40%, less than 30%, less than 20%, less than 10%, or less than 5% of the polynucleotide level, or the level of the polypeptide encoded thereby, of the same target sequence in an appropriate control. Methods to assay for the level of the RNA transcript, the level of the encoded polypeptide, or the activity of the polynucleotide or polypeptide are discussed elsewhere herein.

### A. Sense Suppression/Cosuppression

In some aspects of the disclosure of the disclosure, decreasing expression of a Nrf4 polypeptide may be obtained by sense suppression or cosuppression. For cosuppression, an expression cassette is designed to express an RNA molecule corresponding to all or part of a messenger RNA encoding a polypeptide in the "sense" orientation. Over expression of the RNA molecule may result in decreased expression of the native gene. Accordingly, multiple plant lines transformed with the cosuppression expression cassette are screened to identify those that show the desired degree of inhibition of polypeptide expression.

Typically, a sense suppression element has substantial sequence identity to the target polynucleotide, typically greater than about 65% sequence identity, greater than about 85% sequence identity, about 90%, 91%, 92%, 93%, 94%, 95%, 96%, 97%, 98% or 99% sequence identity. See, U.S. Pat. Nos. 5,283,184 and 5,034,323. The sense suppression element can be any length so long as it allows for the suppression of the targeted sequence. The sense suppression element can be, for example, 15, 16, 17, 18 19, 20, 22, 25, 30, 50, 100, 150, 200, 250, 300, 350, 400, 450, 500, 600, 700, 900, 1000, 1100, 1200, 1300 nucleotides or longer of the target polynucleotides set forth in any of SEQ ID NO:3 - 8, 10, 23, 24, 17, 18, 22, 28 or 29. In other aspects of the disclosure, the sense suppression element can be, for example, about 15-25, 25-100, 100-150, 150-200, 200-250, 250-300, 300-350, 350-400, 450-500, 500-550, 550-600, 600-650, 650-700, 700-750, 750-800, 800-850, 850-900, 900-950, 950-1000, 1000-1050, 1050-1100, 1100-1200, 1200-1300, 1300-1400, 1400-1500, 1500-1600, 1600-1700, 1700-1800 nucleotides or longer of the target polynucleotides set forth in any of SEQ ID NO:3 - 8, 10, 23, 24, 17, 18, 22, 28 or 29.

The polynucleotide used for cosuppression may correspond to all or part of the sequence encoding the polypeptide, all or part of the 5' and/or 3' untranslated region of a polypeptide transcript or all or part of both the coding sequence and the untranslated regions of a transcript encoding a polypeptide. In some aspects of the disclosure where the polynucleotide comprises all or part of the coding region for the polypeptide, the expression cassette is designed to eliminate the start codon of the polynucleotide so that no protein product will be translated.

Cosuppression may be used to inhibit the expression of plant genes to produce plants having undetectable protein levels for the proteins encoded by these genes. See, for example, Broin, et al., (2002) Plant Cell 14:1417-1432. Cosuppression may also be used to inhibit the expression of multiple proteins in the same plant. See, for example, US Patent Number 5,942,657. Methods for using cosuppression to inhibit the expression of endogenous genes in plants are described in Flavell, et al., (1994) Proc. Natl. Acad. Sci. USA 91:3490-3496; Jorgensen, et al., (1996) Plant Mol. Biol. 31:957-973; Johansen and Carrington, (2001) Plant Physiol. 126:930-938; Broin, et al., (2002) Plant Cell 14:1417-1432; Stoutjesdijk, et al., (2002) Plant Physiol. 129:1723-1731; Yu, et al., (2003) Phytochemistry 63:753-763 and US Patent Numbers 5,034,323, 5,283,184 and 5,942,657. The efficiency of cosuppression may be increased by including a poly-dT region in the expression cassette at a position 3' to the sense sequence and 5' of the polyadenylation signal. See, US Patent Application Publication Number 2002/0048814. Typically, such a nucleotide sequence has substantial sequence identity to the sequence of the transcript of the endogenous gene, optimally greater than about 65% sequence identity, more optimally greater than about 85% sequence identity, most optimally greater than about 95% sequence identity. See US Patent Numbers 5,283,184 and 5,034,323.

### B. Antisense Suppression

In some aspects of the disclosure, inhibition of the expression of the polypeptide may be obtained by antisense suppression. For antisense suppression, the expression cassette is designed to express an RNA molecule complementary to all or part of a messenger RNA encoding the polypeptide. Over expression of the antisense RNA molecule may result in decreased expression of the target gene. Accordingly, multiple plant lines transformed with the antisense suppression expression cassette are screened to identify those that show the desired degree of inhibition of polypeptide expression.

The polynucleotide for use in antisense suppression may correspond to all or part of the complement of the sequence encoding the polypeptide, all or part of the complement of the 5' and/or 3' untranslated region of the target transcript or all or part of the complement of both the coding sequence and the untranslated regions of a transcript encoding the polypeptide. In addition, the antisense polynucleotide may be fully complementary (i.e., 100% identical to the complement of the target sequence) or partially complementary (i.e., less than 100% identical to the complement of the target sequence) to the target sequence. In addition, the antisense suppression element may be fully complementary (i.e., 100% identical to the complement of the target sequence) or partially complementary (i.e., less than 100% identical to the complement of the target sequence) to the target polynucleotide. In specific aspects of the disclosure, the antisense suppression element comprises at least 85%, 90%, 91%, 92%, 93%, 94%, 95%, 96%, 97%, 98%, or 99% sequence complementarity to the target polynucleotide. Antisense suppression may be used to inhibit the expression of multiple proteins in the same plant. See, for example, U.S. Pat. No. 5,942,657. Furthermore, the antisense suppression element can be complementary to a portion of the target polynucleotide. Generally, sequences of at least 15, 20, 22, 25, 50, 100, 200, 300, 400, 450 nucleotides or greater of the sequence set forth in any of SEQ ID NO:3 - 8, 10, 23, 24, 17, 18, 22, 28 or 29may be used. Methods for using antisense suppression to inhibit the expression of endogenous genes in plants are described, for example, in Liu et at (2002) Plant Physiol. 129:1732-1743 and U.S. Pat. Nos. 5,759,829 and 5,942,657. Efficiency of antisense suppression may be increased by including a poly-dT region in the expression cassette at a position 3' to the antisense sequence and 5' of the polyadenylation signal. See, US Patent Application Publication Number 2002/0048814.

### C. Double-Stranded RNA Interference

In some aspects of the disclosure, inhibition of the expression of a polypeptide may be obtained by double-stranded RNA (dsRNA) interference. A "double stranded RNA silencing element" or "dsRNA" comprises at least one transcript that is capable of forming a dsRNA. Thus, a "dsRNA silencing element" includes a dsRNA, a transcript or polyribonucleotide capable of forming a dsRNA or more than one transcript or polyribonucleotide capable of forming a dsRNA. "Double stranded RNA" or "dsRNA" refers to a polyribonucleotide structure formed either by a single self-complementary RNA molecule or a polyribonucleotide structure formed by the expression of least two distinct RNA strands. The dsRNA molecule(s) employed in the methods and compositions mediate the decrease of expression of a target sequence, for example, by mediating RNA interference "RNAi" or gene silencing in a sequence-specific manner. The dsRNA is capable of decreasing or eliminating the level or expression of a target polynucleotide or the polypeptide, for example, *Nrf4.*

The dsRNA can decrease or eliminate the expression level of the target sequence by influencing the level of the target RNA transcript, by influencing translation and thereby affecting the level of the encoded polypeptide, or by influencing expression at the pre-transcriptional level (i.e., via the modulation of chromatin structure, methylation pattern, etc., to alter gene expression). See, for example, Verdel et al., (2004) Science 303:672-676; Pal-Bhadra et al., (2004) Science 303:669-672; Allshire (2002) Science 297:1818-1819; Volpe et al., (2002) Science 297:1833-1837; Jenuwein (2002) Science 297:2215-2218; and Hall et al., (2002) Science 297:2232-2237. Methods to assay for functional dsRNA that are capable of decreasing or eliminating the level of a sequence of interest are disclosed elsewhere herein. Accordingly, as used herein, the term "dsRNA" is meant to encompass other terms used to describe nucleic acid molecules that are capable of mediating RNA interference or gene silencing, including, for example, short-interfering RNA (siRNA), double-stranded RNA (dsRNA), micro-RNA (miRNA), hairpin RNA, short hairpin RNA (shRNA), post-transcriptional gene silencing RNA (ptgsRNA), and others.

For dsRNA interference, a sense RNA molecule like that described above for cosuppression and an antisense RNA molecule that is fully or partially complementary to the sense RNA molecule are expressed in the same cell, resulting in inhibition of the expression of the corresponding endogenous messenger RNA.

Expression of the sense and antisense molecules may be accomplished by designing the expression cassette to comprise both a sense sequence and an antisense sequence. Alternatively, separate expression cassettes may be used for the sense and antisense sequences. Multiple plant lines transformed with the dsRNA interference expression cassette or expression cassettes are then screened to identify plant lines that show the desired degree of inhibition of polypeptide expression. Methods for using dsRNA interference to inhibit the expression of endogenous plant genes are described in Waterhouse, et al., (1998) Proc. Natl. Acad. Sci. USA 95:13959-13964, Liu, et al., (2002) Plant Physiol. 129:1732-1743 and WO 1999/49029, WO 1999/53050, WO 1999/61631 and WO 2000/49035.

### D. Hairpin RNA Interference and Intron-Containing Hairpin RNA Interference

In some aspects of the disclosure, inhibition of the expression of a polypeptide may be obtained by hairpin RNA (hpRNA) interference or intron-containing hairpin RNA (ihpRNA) interference. These methods are highly efficient at inhibiting the expression of endogenous genes. See, Waterhouse and Helliwell, (2003) Nat. Rev. Genet. 4:29-38, and the references cited therein.

For hpRNA interference, the expression cassette is designed to express an RNA molecule that hybridizes with itself to form a hairpin structure that comprises a single-stranded loop region and a base-paired stem. The base-paired stem region comprises a sense sequence corresponding to all or part of the endogenous messenger RNA encoding the gene whose expression is to be inhibited, and an antisense sequence that is fully or partially complementary to the sense sequence. Alternatively, the base-paired stem region may correspond to a portion of a promoter sequence controlling expression of the gene whose expression is to be inhibited. Thus, the base-paired stem region of the molecule generally determines the specificity of the RNA interference. hpRNA molecules are highly efficient at inhibiting the expression of endogenous genes and the RNA interference they induce is inherited by subsequent generations of plants. See, for example, Chuang and Meyerowitz, (2000) Proc. Natl. Acad. Sci. USA 97:4985-4990; Stoutjesdijk, et al., (2002) Plant Physiol. 129:1723-1731; and Waterhouse and Helliwell, (2003) Nat. Rev. Genet. 4:29-38. Methods for using hpRNA interference to inhibit or silence the expression of genes are described, for example, in Chuang and Meyerowitz, (2000) Proc. Natl. Acad. Sci. USA 97:4985-4990; Stoutjesdijk, etal., (2002) Plant Physiol. 129:1723-1731; Waterhouse and Helliwell, (2003) Nat. Rev. Genet. 4:29-38; Pandolfini et al., BMC Biotechnology 3:7; and US Patent Application Publication Number 2003/0175965. A transient assay for the efficiency of hpRNA constructs to silence gene expression *in vivo* has been described by Panstruga, et al., (2003)Mol. Biol. Rep. 30:135-140.

For ihpRNA, the interfering molecules have the same general structure as for hpRNA, but the RNA molecule additionally comprises an intron that is capable of being spliced in the cell in which the ihpRNA is expressed. The use of an intron minimizes the size of the loop in the hairpin RNA molecule following splicing, and this increases the efficiency of interference. See, for example, Smith, et al., (2000) Nature 407:319-320. In fact, Smith, *et al.,* show 100% suppression of endogenous gene expression using ihpRNA-mediated interference. Methods for using ihpRNA interference to inhibit the expression of endogenous plant genes are described, for example, in Smith, et al., (2000) Nature 407:319-320; Wesley, et al., (2001) Plant J. 27:581-590; Wang and Waterhouse, (2001) Curr. Opin. Plant Biol. 5:146-150; Waterhouse and Helliwell, (2003) Nat. Rev. Genet. 4:29-38; Helliwell and Waterhouse, (2003) Methods 30:289-295; and US Patent Application Publication Number 2003/0180945.

Any region of the target polynucleotide can be used to design the domain of the silencing element that shares sufficient sequence identity to allow expression of the hairpin transcript to decrease the level of the target polynucleotide. For instance, the domain can be designed to share sequence identity to the 5' untranslated region of the target polynucleotide(s), the 3' untranslated region of the target polynucleotide(s), exonic regions of the target polynucleotide(s), intronic regions of the target polynucleotide(s), and any combination thereof. In specific aspects of the disclosure, a domain of the silencing element shares sufficient homology to at least about 15, 16, 17, 18, 19, 20, 22, 25 or 30 consecutive nucleotides from about nucleotides 1-50, 25-75, 75-125, 50-100, 125-175, 175-225, 100-150, 150-200, 200-250, 225-275, 275-325, 250-300, 325-375, 375-425, 300-350, 350-400, 425-475, 400-450, 475-525, 450-500, 525-575, 575-625, 550-600, 625-675, 675-725, 600-650, 625-675, 675-725, 650-700, 725-825, 825-875, 750-800, 875-925, 925-975, 850-900, 925-975, 975-1025, 950-1000, 1000-1050, 1025-1075, 1075-1125, 1050-1100, 1125-1175, 1100-1200, 1175-1225, 1225-1275, 1200-1300, 1325-1375, 1375-1425, 1300-1400, 1425-1475, 1475-1525, 1400-1500, 1525-1575, 1575-1625, 1625-1675, 1675-1725, 1725-1775, 1775-1825, 1825-1875, 1875-1925, 1925-1975, 1975-2025, 2025-2075, 2075-2125, 2125-2175, 2175-2225, 1500-1600, 1600-1700, 1700-1800, 1800-1900, 1900-2000 of the target sequence. In some instances to optimize the siRNA sequences employed in the hairpin, the synthetic oligodeoxyribonucleotide/RNAse H method can be used to determine sites on the target mRNA that are in a conformation that is susceptible to RNA silencing. See, for example, Vickers et al., (2003) J. Biol. Chem. 278:7108-7118 and Yang et al., (2002) Proc. Natl. Acad. Sci. USA 99:9442-9447. These studies indicate that there is a significant correlation between the RNase-H-sensitive sites and sites that promote efficient siRNA-directed mRNA degradation.

The expression cassette for hpRNA interference may also be designed such that the sense sequence and the antisense sequence do not correspond to an endogenous RNA. In this aspect of the disclosure, the sense and antisense sequence flank a loop sequence that comprises a nucleotide sequence corresponding to all or part of the endogenous messenger RNA of the target gene. Thus, it is the loop region that determines the specificity of the RNA interference. See, for example, WO 2002/00904; Mette, et al., (2000) EMBO J 19:5194-5201; Matzke, et al., (2001) Curr. Opin. Genet. Devel. 11:221-227; Scheid, et al., (2002) Proc. Natl. Acad. Sci. USA 99: 13659-13662; Aufsaftz, et al., (2002) Proc. Natl. Acad. Sci. 99:16499-16506; Sijen, et al., Curr. Biol. (2001) 11:436-440.

In addition, transcriptional gene silencing (TGS) may be accomplished through use of a hairpin suppression element where the inverted repeat of the hairpin shares sequence identity with the promoter region of a target polynucleotide to be silenced. See, for example, Aufsatz et al., (2002) Proc. Natl. Acad. Sci. 99:16499-16506 and Mette et al., (2000) EMBO J. 19:5194-5201.

### E. AmpliconMediated Interference

Amplicon expression cassettes comprise a plant virus-derived sequence that contains all or part of the target gene but generally not all of the genes of the native virus. The viral sequences present in the transcription product of the expression cassette allow the transcription product to direct its own replication. The transcripts produced by the amplicon may be either sense or antisense relative to the target sequence (i.e., the messenger RNA for the polypeptide). Methods of using amplicons to inhibit the expression of endogenous plant genes are described, for example, in Angell and Baulcombe, (1997) EMBO J. 16:3675-3684; Angell and Baulcombe, (1999) Plant J 20:357-362; and US Patent Number 6,646,805.

### F. Ribozymes

In some aspects of the disclosure, the polynucleotide expressed by the expression cassette of the disclosure is a catalytic RNA or has ribozyme activity specific for the messenger RNA of the polypeptide. Thus, the polynucleotide causes the degradation of the endogenous messenger RNA, resulting in decreased expression of the polypeptide. This method is described, for example, in US Patent Number 4,987,071.

### G. Small Interfering RNA or Micro RNA

In some aspects of the disclosure, inhibition of the expression of a polypeptide may be obtained by RNA interference by expression of a gene encoding a micro RNA (miRNA) or short-interfering RNA (siRNA) (Meister and Tuschl (2004) Nature 431:343-349 and Bonetta et al., (2004) Nature Methods 1:79-86). miRNAs are regulatory agents consisting of about 22 ribonucleotides. miRNA are highly efficient at inhibiting the expression of endogenous genes. See, for example Palatnik et al., (2003) Nature 425:257-263. The miRNA can be an "artificial miRNA" or "amiRNA" which comprises a miRNA sequence that is synthetically designed to silence a target sequence.

For miRNA interference, the expression cassette is designed to express an RNA molecule that is modeled on an endogenous miRNA gene. For example, the miRNA gene encodes an RNA that forms a hairpin structure containing a 22-nucleotide sequence that is complementary to another endogenous gene (target sequence). In some aspects of the disclosure, the 22-nucleotide sequence is selected from a transcript sequence from a *Nrf4* gene and contains 22 nucleotides of the *Nrf4* gene in sense orientation and 21 nucleotides of a corresponding antisense sequence that is complementary to the sense sequence. In some aspects of the disclosure, in addition to targeting *Nrf4,* genes involved in recombination may also be targeted. Accordingly, in some aspects of the disclosure, the 22-nucleotide sequence is selected from a transcript sequence from a gene involved in recombination and contains 22 nucleotides of the gene involved in recombination in sense orientation and 21 nucleotides of a corresponding antisense sequence that is complementary to the sense sequence. miRNA molecules are highly efficient at inhibiting the expression of endogenous genes, and the RNA interference they induce is inherited by subsequent generations of plants.

The heterologous polynucleotide being expressed need not form the dsRNA by itself, but can interact with other sequences in the plant cell to allow the formation of the dsRNA. For example, a chimeric polynucleotide that can selectively silence the target polynucleotide can be generated by expressing a chimeric construct comprising the target sequence for a miRNA or siRNA to a sequence corresponding to all or part of the gene or genes to be silenced. In this aspect of the disclosure, the dsRNA is "formed" when the target for the miRNA or siRNA interacts with the miRNA present in the cell. The resulting dsRNA can then decrease the level of expression of the gene or genes to be silenced. See, for example, US Application Publication 2007-0130653, entitled "Methods and Compositions for Gene Silencing". The construct can be designed to have a target for an endogenous miRNA or, alternatively, a target for a heterologous and/or synthetic miRNA can be employed in the construct. If a heterologous and/or synthetic miRNA is employed, it can be introduced into the cell on the same nucleotide construct as the chimeric polynucleotide or on a separate construct. As discussed elsewhere herein, any method can be used to introduce the construct comprising the heterologous miRNA.

While the various target sequences disclosed herein can be used to design any silencing element that encodes a miRNA, non-limiting examples of such miRNA constructs include SEQ ID NOS:33 - 37; or active variants or fragments thereof.

### H. Polypeptide-Based Inhibition of Gene Expression

In one aspect of the disclosure, the polynucleotide encodes a zinc finger protein that binds to a gene encoding a polypeptide, resulting in decreased expression of the gene. In other aspects of the disclosure, the zinc finger protein binds to a messenger RNA encoding a polypeptide and prevents its translation. Methods of selecting sites for targeting by zinc finger proteins have been described, for example, in US Patent Number 6,453,242, and methods for using zinc finger proteins to inhibit the expression of genes in plants are described, for example, in US Patent Application Publication Number 2003/0037355.

### I. Polypeptide-Based Inhibition of Protein Activity

In some aspects of the disclosure, the polynucleotide encodes an antibody that binds to at least one polypeptide and decreases the activity of the polypeptide. In another aspect of the disclosure, the binding of the antibody results in increased turnover of the antibody complex by cellular quality control mechanisms. The expression of antibodies in plant cells and the inhibition of molecular pathways by expression and binding of antibodies to proteins in plant cells are well known in the art. See, for example, Conrad and Sonnewald, (2003) Nature Biotech. 21:35-36.

### V. Gene Disruption

In some aspects of the present disclosure, the activity of a polypeptide is decreased or eliminated by disrupting the gene encoding the polypeptide. The gene encoding the polypeptide may be disrupted by any method known in the art, for example, by genome editing, transposon tagging or mutagenizing plants using random or targeted mutagenesis and selecting for plants that have decreased activity.

### A. Genome Editing and Induced Mutagenesis

In some aspects of the disclosure, the target, for example, *Nrf4* can be modified using gene editing technology, including without limitation double-strand-break-inducing agent, such as but not limited to a CRISPR-Cas guideRNA or other polynucleotide-guided double strand break reagent, a Zinc Finger endonuclease, a meganuclease, or a TALEN endonuclease.

CRISPR loci (Clustered Regularly Interspaced Short Palindromic Repeats) (also known as SPIDRs-SPacer Interspersed Direct Repeats) constitute a family of recently described DNA loci. CRISPR loci consist of short and highly conserved DNA repeats (typically 24 to 40 bp, repeated from 1 to 140 times, also referred to as CRISPR-repeats) which are partially palindromic. The repeated sequences (usually specific to a species) are interspaced by variable sequences of constant length (typically 20 to 58 bp depending on the CRISPR locus (WO2007/025097 published March 1, 2007).

CRISPR loci were first recognized in *Escherichia coli* (Ishino et al., (1987) J. Bacteriol. 169:5429-5433; Nakata et al., (1989) J. Bacteriol. 171:3553-3556). Similar interspersed short sequence repeats have been identified in *Haloferax mediterranei, Streptococcus pyogenes, Anabaena,* and *Mycobacterium tuberculosis* (Groenen et al., (1993) Mol. Microbiol. 10:1057-1065; Hoe et al., (1999) Emerg. Infect. Dis. 5:254-263; Masepohl et al., (1996) Biochim. Biophys. Acta 1307:26-30; Mojica et al. (1995) Mol. Microbiol. 17:85-93). The CRISPR loci differ from other simple sequence repeats (SSRs) by the structure of the repeats, which have been termed short regularly spaced repeats (SRSRs) (Janssen etal., (2002) J. Integ. Biol. 6:23-33; Mojica etal., (2000) Mol. Microbiol. 36:244-246). The repeats are short elements that occur in clusters, which are always regularly spaced by variable sequences of constant length (Mojica et al., (2000) Mol. Microbiol. 36:244-246).

*Cas* gene relates to a gene that is generally coupled, associated or close to, or in the vicinity of flanking CRISPR loci. The terms *"Cas* gene", "CRISPR-associated (Cas) gene" are used interchangeably herein. A comprehensive review of the Cas protein family is presented in Haft *et al.,* (2005) *PLoS Comput Biol* 1: e60.. As described therein, 41 CRISPR-associated (*Cas*) gene families are described, in addition to the four previously known gene families. It shows that CRISPR systems belong to different classes, with different repeat patterns, sets of genes, and species ranges. The number of *Cas* genes at a given CRISPR locus can vary between species.

Cas endonuclease relates to a Cas protein encoded by a *Cas* gene, wherein said Cas protein is capable of introducing a double strand break into a DNA target sequence. The Cas endonuclease is guided by a guide polynucleotide to recognize and optionally introduce a double strand break at a specific target site into the genome of a cell (U.S. Provisional Application No. 62/023239, filed July 11, 2014). The guide polynucleotide/Cas endonuclease system includes a complex of a Cas endonuclease and a guide polynucleotide that is capable of introducing a double strand break into a DNA target sequence. The Cas endonuclease unwinds the DNA duplex in close proximity of the genomic target site and cleaves both DNA strands upon recognition of a target sequence by a guide RNA if a correct protospacer-adjacent motif (PAM) is approximately oriented at the 3' end of the target sequence.

The *Cas* endonuclease gene can encode Cas9 endonuclease, or a functional fragment thereof, such as but not limited to the *Cas9* genes listed in SEQ ID NOs: 462, 474, 489, 494, 499, 505, and 518 of WO2007/025097, published March 1, 2007. The *Cas* endonuclease gene can be a plant, maize or soybean optimized Cas9 endonuclease, such as but not limited to a plant codon optimized *Streptococcus pyogenes Cas9* gene that can recognize any genomic sequence of the form N(12-30)NGG. The Cas endonuclease can be introduced directly into a cell by any method known in the art, for example, but not limited to, transient introduction methods, transfection, and/or topical application.

As used herein, the term "guide RNA" relates to a synthetic fusion of two RNA molecules, a crRNA (CRISPR RNA) comprising a variable targeting domain, and a tracrRNA. In one aspect of the disclosure, the guide RNA comprises a variable targeting domain of 12 to 30 nucleotide sequences and a RNA fragment that can interact with a Cas endonuclease.

As used herein, the term "guide polynucleotide" relates to a polynucleotide sequence that can form a complex with a Cas endonuclease and enables the Cas endonuclease to recognize and optionally cleave a DNA target site (U.S. Provisional Application No. 62/023239, filed July 11, 2014). The guide polynucleotide can be a single molecule or a double molecule. The guide polynucleotide sequence can be a RNA sequence, a DNA sequence, or a combination thereof (a RNA-DNA combination sequence). Optionally, the guide polynucleotide can comprise at least one nucleotide, phosphodiester bond or linkage modification such as, but not limited to, Locked Nucleic Acid (LNA), 5-methyl dC, 2,6-Diaminopurine, 2'-Fluoro A, 2'-Fluoro U, 2'-O-Methyl RNA, phosphorothioate bond, linkage to a cholesterol molecule, linkage to a polyethylene glycol molecule, linkage to a spacer 18 (hexaethylene glycol chain) molecule, or 5' to 3' covalent linkage resulting in circularization. A guide polynucleotide that solely comprises ribonucleic acids is also referred to as a "guide RNA".

The guide polynucleotide can be a double molecule (also referred to as duplex guide polynucleotide), comprising a first nucleotide sequence domain (referred to as Variable Targeting domain or VT domain) that is complementary to a nucleotide sequence in a target DNA and a second nucleotide sequence domain (referred to as Cas endonuclease recognition domain or CER domain) that interacts with a Cas endonuclease polypeptide. The CER domain of the double molecule guide polynucleotide comprises two separate molecules that are hybridized along a region of complementarity. The two separate molecules can be RNA, DNA, and/or RNA-DNA-combination sequences. In some aspects of the disclosure, the first molecule of the duplex guide polynucleotide comprising a VT domain linked to a CER domain is referred to as "crDNA" (when composed of a contiguous stretch of DNA nucleotides) or "crRNA" (when composed of a contiguous stretch of RNA nucleotides), or "crDNA-RNA" (when composed of a combination of DNA and RNA nucleotides). The crNucleotide can comprise a fragment of the cRNA naturally occurring in Bacteria and Archaea. In one aspect of the disclosure, the size of the fragment of the cRNA naturally occurring in Bacteria and Archaea that is present in a crNucleotide disclosed herein can range from, but is not limited to, 2, 3, 4, 5, 6, 7, 8, 9,10, 11, 12, 13, 14, 15, 16, 17, 18, 19, 20 or more nucleotides. In some aspects of the disclosure, the second molecule of the duplex guide polynucleotide comprising a CER domain is referred to as "tracrRNA" (when composed of a contiguous stretch of RNA nucleotides) or "tracrDNA" (when composed of a contiguous stretch of DNA nucleotides) or "tracrDNA-RNA" (when composed of a combination of DNA and RNA nucleotides. In one aspect of the disclosure, the RNA that guides the RNA/Cas9 endonuclease complex is a duplexed RNA comprising a duplex crRNA-tracrRNA.

The guide polynucleotide can also be a single molecule comprising a first nucleotide sequence domain (referred to as Variable Targeting domain or VT domain) that is complementary to a nucleotide sequence in a target DNA and a second nucleotide domain (referred to as Cas endonuclease recognition domain or CER domain) that interacts with a Cas endonuclease polypeptide. By "domain" it is meant a contiguous stretch of nucleotides that can be RNA, DNA, and/or RNA-DNA-combination sequence. The VT domain and/or the CER domain of a single guide polynucleotide can comprise a RNA sequence, a DNA sequence, or a RNA-DNA-combination sequence. In some aspects of the disclosure, the single guide polynucleotide comprises a crNucleotide (comprising a VT domain linked to a CER domain) linked to a tracrNucleotide (comprising a CER domain), wherein the linkage is a nucleotide sequence comprising a RNA sequence, a DNA sequence, or a RNA-DNA combination sequence. The single guide polynucleotide being comprised of sequences from the crNucleotide and tracrNucleotide may be referred to as "single guide RNA" (when composed of a contiguous stretch of RNA nucleotides) or "single guide DNA" (when composed of a contiguous stretch of DNA nucleotides) or "single guide RNA-DNA" (when composed of a combination of RNA and DNA nucleotides). In one aspect of the disclosure, the single guide RNA comprises a cRNA or cRNA fragment and a tracrRNA or tracrRNA fragment of the type II CRISPR/Cas system that can form a complex with a type II Cas endonuclease, wherein said guide RNA/Cas endonuclease complex can direct the Cas endonuclease to a plant genomic target site, enabling the Cas endonuclease to introduce a double strand break into the genomic target site. One aspect of using a single guide polynucleotide versus a duplex guide polynucleotide is that only one expression cassette needs to be made to express the single guide polynucleotide.

The term "variable targeting domain" or "VT domain" is used interchangeably herein and includes a nucleotide sequence that is complementary to one strand (nucleotide sequence) of a double strand DNA target site. The % complementation between the first nucleotide sequence domain (VT domain) and the target sequence can be at least 50%, 51%, 52%, 53%, 54%, 55%, 56%, 57%, 58%, 59%, 60%, 61%, 62%, 63%, 63%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100%. The variable target domain can be at least 12, 13, 14, 15, 16, 17, 18, 19, 20, 21, 22, 23, 24, 25, 26, 27, 28, 29 or 30 nucleotides in length. In some aspects of the disclosure, the variable targeting domain comprises a contiguous stretch of 12 to 30 nucleotides. The variable targeting domain can be composed of a DNA sequence, a RNA sequence, a modified DNA sequence, a modified RNA sequence, or any combination thereof.

The term "Cas endonuclease recognition domain" or "CER domain" of a guide polynucleotide is used interchangeably herein and includes a nucleotide sequence (such as a second nucleotide sequence domain of a guide polynucleotide) that interacts with a Cas endonuclease polypeptide. The CER domain can be composed of a DNA sequence, a RNA sequence, a modified DNA sequence, a modified RNA sequence (see for example modifications described herein), or any combination thereof.

The nucleotide sequence linking the crNucleotide and the tracrNucleotide of a single guide polynucleotide can comprise a RNA sequence, a DNA sequence, or a RNA-DNA combination sequence. In one aspect of the disclosure, the nucleotide sequence linking the crNucleotide and the tracrNucleotide of a single guide polynucleotide can be at least 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 78, 79, 80, 81, 82, 83, 84, 85, 86, 87, 88, 89, 90, 91, 92, 93, 94, 95, 96, 97, 98, 99 or 100 nucleotides in length. In another aspect of the disclosure, the nucleotide sequence linking the crNucleotide and the tracrNucleotide of a single guide polynucleotide can comprise a tetraloop sequence, such as, but not limiting to, a GAAA tetraloop seqence.

Nucleotide sequence modification of the guide polynucleotide, VT domain and/or CER domain can be selected from, but not limited to, the group consisting of a 5' cap, a 3' polyadenylated tail, a riboswitch sequence, a stability control sequence, a sequence that forms a dsRNA duplex, a modification or sequence that targets the guide polynucleotide to a subcellular location, a modification or sequence that provides for tracking, a modification or sequence that provides a binding site for proteins, a Locked Nucleic Acid (LNA), a 5-methyl dC nucleotide, a 2,6-Diaminopurine nucleotide, a 2'-Fluoro A nucleotide, a 2'-Fluoro U nucleotide, a 2'-O-Methyl RNA nucleotide, a phosphorothioate bond, linkage to a cholesterol molecule, linkage to a polyethylene glycol molecule, linkage to a spacer 18 molecule, a 5' to 3' covalent linkage, or any combination thereof. These modifications can result in at least one additional beneficial feature, wherein the additional beneficial feature is selected from the group of a modified or regulated stability, a subcellular targeting, tracking, a fluorescent label, a binding site for a protein or protein complex, modified binding affinity to complementary target sequence, modified resistance to cellular degradation, and increased cellular permeability.

### B. Transposon Tagging

In one aspect of the disclosure, transposon tagging is used to decrease or eliminate the activity of one or more polypeptides. Transposon tagging comprises inserting a transposon within an endogenous gene in the pathway to decrease or eliminate expression of the polypeptide.

In this aspect of the disclosure, the expression of one or more polypeptides is decreased or eliminated by inserting a transposon within a regulatory region or coding region of the gene encoding the polypeptide. A transposon that is within an exon, intron, 5' or 3' untranslated sequence, a promoter or any other regulatory sequence of a gene may be used to decrease or eliminate the expression and/or activity of the encoded polypeptide.

Methods for the transposon tagging of specific genes in plants are well known in the art. See, for example, Maes, etal., (1999) Trends Plant Sci. 4:90-96; Dharmapuri and Sonti, (1999) FEMS Microbiol. Lett. 179:53-59; Meissner et al., (2000) Plant J. 22:265-274; Phogat, et al., (2000) J. Biosci. 25:57-63; Walbot, (2000) Curr. Opin. Plant Biol. 2:103-107; Gai et al., (2000) Nucleic Acids Res. 28:94-96; Fitzmaurice et al., (1999) Genetics 153:1919-1928. In addition, the TUSC process for selecting *Mu* insertions in selected genes has been described in Bensen et al., (1995) Plant Cell 7:75-84; Mena et al., (1996) Science 274:1537-1540; and US Patent Number 5,962,764.

### C. Mutant Plants with Decreased Activity

Additional methods for decreasing or eliminating the expression of endogenous genes in plants are also known in the art and may be similarly applied to the instant disclosure. These methods include other forms of mutagenesis, such as ethyl methanesulfonate-induced mutagenesis, deletion mutagenesis, and fast neutron deletion mutagenesis used in a reverse genetics sense (with PCR) to identify plant lines, in which the endogenous gene has been mutated or deleted. For examples of these methods see Ohshima et al., (1998) Virology 243:472-481; Okubara et al., (1994) Genetics 137:867-874; and Quesada et al., (2000) Genetics 154:421-436. In addition, a fast and automatable method for screening for chemically induced mutations, TILLING (Targeting Induced Local Lesions In Genomes), using denaturing HPLC or selective endonuclease digestion of selected PCR products is also applicable to the instant disclosure. See McCallum et al., (2000) Nat. Biotechnol. 18:455-457.

Mutations may impact gene expression or interfere with the activity of an encoded Nrf4 protein. Insertional mutations in gene exons usually result in null-mutants. Mutations in conserved residues are particularly effective in inhibiting the activity of the encoded protein. Conserved residues of plant polypeptides suitable for mutagenesis with the goal to eliminate activity have been described. Such mutants may be isolated according to well-known procedures and mutations in different *Nrf4* loci may be stacked by genetic crossing. See, for example, Gruis et al., (2002) Plant Cell 14:2863-2882.

In another aspect of this disclosure, dominant mutants may be used to trigger RNA silencing due to gene inversion and recombination of a duplicated gene locus. See, for example, Kusaba et al., (2003) Plant Cell 15:1455-1467.

The disclosure encompasses additional methods for decreasing or eliminating the activity of one or more target polypeptides. Examples of other methods for altering or mutating a genomic nucleotide sequence in a plant are known in the art and include, but are not limited to, the use of RNA:DNA vectors, RNA:DNA mutational vectors, RNA:DNA repair vectors, mixed-duplex oligonucleotides, self-complementary RNA:DNA oligonucleotides, and recombinogenic oligonucleobases. Such vectors and methods of use are known in the art. See, for example, US Patent Numbers 5,565,350; 5,731,181; 5,756,325; 5,760,012; 5,795,972; and 5,871,984. See also WO 1998/49350, WO 1999/07865, WO 1999/25821, and Beetham et al., (1999) Proc. Natl. Acad. Sci. USA 96:8774-8778.

### VI. Expression Constructs

Methods and compositions are provided to modify the level of expression or activity of a Nrf4 polypeptide in a plant cell, for example, in an ovule primordia, ovule, female sporocyte, female gametophyte, or female gamete. In some aspects of the disclosure, a plant cell is transformed with a DNA construct or expression cassette for expression of at least one silencing element. In specific aspects of the disclosure, modulation of *Nrf4* expression level and/or activity of the Nrf4 polypeptide promotes non-reduction, or non-reduction and non-recombination, during meiosis resulting in the production of non-reduced, or non-reduced and non-recombined, female gametes. Such methods and compositions can employ an expression construct comprising an element that when expressed decreases *Nrf4* polynucleotide and/or Nrf4 polypeptide expression level or activity and is operably linked to a promoter functional in a plant cell. In certain aspects of the disclosure, the promoter is a female sporogenesis-related promoter, in particular a promoter expressing in ovule primordia or ovule tissue, including but not limited to an *Nrf4* promoter.

The expression cassette can include 5' and 3' regulatory sequences operably linked to a polynucleotide of interest, e.g. a silencing element, or an active variant or fragment thereof. "Operably linked" is intended to mean a functional linkage between two or more elements. For example, an operable linkage between a polynucleotide of interest and a regulatory sequence (i.e., a promoter) is a functional link that allows for expression of the polynucleotide of interest, for example, a silencing element. Operably linked elements may be contiguous or non-contiguous. When used to refer to the joining of two protein coding regions, by operably linked is intended that the coding regions are in the same reading frame. The cassette may additionally contain at least one additional gene to be cotransformed into the organism. Alternatively, the additional polynucleotides can be provided on multiple expression cassettes. Such an expression cassette is provided with a plurality of restriction sites and/or recombination sites for insertion of the element to be under the transcriptional regulation of the promoter. The expression cassette may additionally contain selectable marker genes.

In some aspects of the disclosure, the expression cassette will include in the 5'-3' direction of transcription an ovule-specific promoters, ovule-preferred promoters, female-gametophyte specific promoters, female-gametophyte preferred promoters, female-gamete-specific promoters, female-gamete-preferred promoters, a female sporogenesis-related promoter or an active variant or fragment thereof, a silencing element and a transcriptional and translational termination region (i.e., termination region) functional in the host cell (i.e., the plant). The regulatory regions and/or the silencing elements may be heterologous to the host cell, e.g. plant cell, or to each other.

As used herein, "heterologous" in reference to a sequence is a sequence that originates from a foreign species, or, if from the same species, is substantially modified from its native form in composition and/or genomic locus by deliberate human intervention. For example, a promoter operably linked to a heterologous polynucleotide is from a species different from the species from which the polynucleotide was derived, or, if from the same/analogous species, one or both are substantially modified from their original form and/or genomic locus, or the promoter is not the native promoter for the operably linked polynucleotide. As used herein, a chimeric gene comprises a coding sequence operably linked to a transcription initiation region that is heterologous to the coding sequence. In an aspect, the promoter is a heterologous promoter.

The termination region may be native with the transcriptional initiation region, may be native with the operably linked silencing element or with the ovule tissue-preferred promoter sequences, may be native with the plant host, or may be derived from another source (i.e., foreign or heterologous) to the promoter, the silencing element, the plant host, or any combination thereof. Convenient termination regions are available from the Tiplasmid *of Agrobacterium tumefaciens,* such as the octopine synthase and nopaline synthase gene termination regions. See also Guerineau et al., (1991) Mol. Gen. Genet. 262:141-144; Proudfoot, (1991) Cell 64:671-674; Sanfacon, et al., (1991) Genes Dev. 5:141-149; Mogen, et al., (1990) Plant Cell 2:1261-1272; Munroe, et al., (1990) Gene 91:151-158; Ballas, et al., (1989) Nucleic Acids Res. 17:7891-7903; and Joshi, et al., (1987) Nucleic Acids Res. 15:9627-9639.

Where appropriate, the polynucleotides may be optimized for increased expression in the transformed plant. That is, the polynucleotides can be synthesized using plant-preferred codons for improved expression. See, for example, Campbell and Gowri, (1990) Plant Physiol. 92:1-11 for a discussion of host-preferred codon usage. Methods are available in the art for synthesizing plant-preferred genes. See, for example, US Patent Numbers 5,380,831 and 5,436,391, and Murray, et al., (1989) Nucleic Acids Res. 17:477-498.

Additional sequence modifications are known to enhance gene expression in a cellular host. These include elimination of sequences encoding spurious polyadenylation signals, exon-intron splice site signals, transposon-like repeats and other such well-characterized sequences that may be deleterious to gene expression. The G-C content of the sequence may be adjusted to levels average for a given cellular host, as calculated by reference to known genes expressed in the host cell. When possible, the sequence is modified to avoid predicted hairpin secondary mRNA structures.

The constructs or expression cassettes may additionally contain 5' leader sequences. Such leader sequences can act to enhance translation. Translation leaders are known in the art and include: picornavirus leaders, for example, EMCV leader (Encephalomyocarditis Virus 5' noncoding region) (Elroy-Stein et al., (1989) Proc. Natl. Acad. Sci. USA 86:6126-6130); potyvirus leaders, for example, TEV leader (Tobacco Etch Virus) (Gallie et al., (1995) Gene 165:233-238), MDMV leader (Maize Dwarf Mosaic Virus) (Johnson et al., (1986) Virology 154:9-20), and human immunoglobulin heavy-chain binding protein (BiP) (Macej ak et al., (1991) Nature 353:90-94); untranslated leader from the coat protein mRNA of Alfalfa Mosaic Virus (AMV RNA 4) (Jobling et al., (1987) Nature 325:622-625); tobacco mosaic virus (TMV) leader (Gallie et al., (1989) in Molecular Biology of RNA, ed. Cech (Liss, New York), pp. 237-256), and Maize Chlorotic Mottle Virus (MCMV) leader (Lommel et al., (1991) Virology 81:382-385). See also Della-Cioppa et al., (1987) Plant Physiol. 84:965-968. Other methods known to enhance translation can also be utilized, for example, and introns.

In preparing the expression cassette, the various DNA fragments may be manipulated, so as to provide for the DNA sequences in the proper orientation and, as appropriate, in the proper reading frame. Toward this end, adapters or linkers may be employed to join the DNA fragments or other manipulations may be involved to provide for convenient restriction sites, removal of superfluous DNA, or removal of restriction sites. For this purpose, *in vitro* mutagenesis, primer repair, restriction, annealing, substitutions, e.g., transitions and transversions, may be involved.

The expression cassette can also comprise a selectable marker gene for the selection of transformed cells. Selectable marker genes are utilized for the selection of transformed cells or tissues. Marker genes include genes encoding antibiotic resistance, such as those encoding neomycin phosphotransferase II (NEO) and hygromycin phosphotransferase (HPT), as well as genes conferring resistance to herbicidal compounds, such as glufosinate ammonium, bromoxynil, imidazolinones, and 2,4-dichlorophenoxyacetate (2,4-D). Additional selectable markers include phenotypic markers, such as β-galactosidase and fluorescent proteins, for example green fluorescent protein (GFP) (Su et al., (2004) BiotechnolBioeng 85:610-9 and Fetter et al., (2004) Plant Cell 16:215-28), cyan florescent protein (CYP) (Bolte et al., (2004) J. Cell Science 117:943-54 and Kato et al., (2002) Plant Physiol 129:913-42), yellow florescent protein (PhiYFP^{™} from Evrogen, see Bolte et al., (2004) J. Cell Science 117:943-54), and red fluorescent protein (DsRED, see Baird et al., (2000) Proc. Natl. Acad. Sci. USA. 97:11984-11989). For additional selectable markers, see, generally, Yarranton, (1992) Curr. Opin. Biotech 3:506-511; Christopherson et al., (1992) Proc. Natl. Acad. Sci. USA 89:6314-6318; Yao et al., (1992) Cell 71:63-72; Reznikoff, (1992) Mol. Microbiol. 6:2419-2422; Barkley et al., (1980) in The Operon, pp. 177-220; Hu et al., (1987) Cell 48:555-566; Brown et al., (1987) Cell 49:603-612; Figge et al., (1988) Cell 52:713-722; Deuschle et al., (1989) Proc. Natl. Acad. Aci. USA 86:5400-5404; Fuerst et al., (1989) Proc. Natl. Acad. Sci. USA 86:2549-2553; Deuschle et al., (1990) Science 248:480-483; Gossen, (1993) Ph.D. Thesis, University of Heidelberg; Reines et al., (1993) Proc. Natl. Acad. Sci. USA 90:1917-1921; Labow et al., (1990) Mol. Cell. Biol. 10:3343-3356; Zambretti et al., (1992) Proc. Natl. Acad. Sci. USA 89:3952-3956; Bairn et al., (1991) Proc. Natl. Acad. Sci. USA 88:5072-5076; Wyborski et al., (1991) Nucleic Acids Res. 19:4647-4653; Hillenand-Wissman, (1989) Topics Mol. Struc. Biol. 10:143-162; Degenkolb et al., (1991) Antimicrob Agents Chemother 35:1591-1595; Kleinschnidt et al., (1988) Biochemistry 27:1094-1104; Bonin, (1993) Ph.D. Thesis, University of Heidelberg; Gossen et al., (1992) Proc. Natl. Acad. Sci. USA 89:5547-5551; Oliva et al., (1992) Antimicrob Agents Chemother 36:913-919; Hlavka et al., (1985) Handbook of Experimental Pharmacology, Vol. 78 (Springer-Verlag, Berlin); Gill et al., (1988) Nature 334:721-724. The above list of selectable marker genes is not meant to be limiting. Any selectable marker gene can be used.

It is further recognized that various expression constructs other than the *Nrf4* silencing expression construct are described herein. For example, expression constructs having silencing elements for genes involved in recombination and/or sequences encoding marker sequences are also described herein. One of skill will understand how to apply the language discussed above, to any expression construct.

### VII. Nrf4 Sequences

Disclosed herein in Table 1 below and elsewhere are isolated or substantially purified nucleic acid molecules or protein compositions of wild type *Nrf4* polynucleotide or Nrf4 polypeptide sequences, *Nrf4* variant polynucleotides, Nrf4 variant polypeptides, cognate promoter sequences, ortholog sequences, variants or fragments thereof that can be modified to effect non-reduction and non-recombination during meiosis in a plant female sporocyte, female gametophyte, or female gamete.

**Table 1.**

| **SEQ ID.** | **NAME** | **DESCRIPTIO N** | **SPECIES** | **POLYNUCLEOTID E/ POLYPEPTIDE (PN/PP)** |
|---|---|---|---|---|
| SEQ ID NO: 1 | NRF4 | protein | *Zea mays* | PP |
| SEQ ID NO: 2 | NRF4-ALT | Protein - Alternative splicing | *Zea mays* | PP |
| SEQ ID NO: 3 | *NRF4* | mRNA | *Zea mays* | PN |
| SEQ ID NO: 4 | *NRF4* | CDS | *Zea mays* | PN |
| SEQ ID NO: 5 | *NRF4-ALT* | CDS - alternative splicing | *Zea mays* | PN |
| SEQ ID NO: 6 | *NRF4* | 1625 bp 5' of transcription start (promoter) | *Zea mays* | PN |
| SEQ ID NO: 7 | *NRF4* | genomic | *Zea mays* | PN |
| SEQ ID NO:8 | *Bradi1g19285. 1* | CDS | *Brachypodium distachyon* | PN |
| SEQ ID NO: 9 | Bradi1g19285. 1 | protein | *Brachypodium distachyon* | PP |
| SEQ ID NO:10 | *Bradi1g19285. 1* | genomic | *Brachypodium distachyon* | PN |
| SEQ ID NO: 11 | *Bradi1g19285. 1* | promoter | *Brachypodium distachyon* | PN |
| SEQ ID NO: 12 | *Sobic 002G400700.1* | CDS | *Sorghum bicolor* | PN |
| SEQ ID NO:13 | *Sobic 002G400700* | genomic | *Sorghum bicolor* | PN |
| SEQ ID NO: 14 | Sobic 002G400700.1 | protein | *Sorghum bicolor* | PP |
| SEQ ID NO: 15 | *Sobic 002G400700.1* | promoter | *Sorghum bicolor* | PN |
| SEQ ID NO:16 | Pavirv0000987 8m | protein | *Panicum virgatum* | PP |
| SEQ ID NO:17 | *Pavirv0000987 8m* | CDS | *Panicum virgatum* | PN |
| SEQ ID NO: 18 | *Pavirv0000987 8m* | genomic | *Panicum virgatum* | PN |
| SEQ ID NO:19 | *Pavirv0000987 8m* | promoter | *Panicum virgatum* | PN |
| SEQ ID NO:20 | Si030723 | protein | *Setaria italica* | PP |
| SEQ ID NO:21 | *Si030723* | promoter (1.7kb) | *Setaria italica* | PN |
| SEQ ID NO:22 | *NRF4 orthologue* | CDS with introns from AP005184 and AP005182 | *Oryza sativa* | PN |
| SEQ ID NO:23 | *NRF4 orthologue promoter* Chr7.fgenesh. mRNA.4040 | Promoter (1.7kb): from AP005184 and AP005182 | *Oryza sativa* | PN |
| SEQ ID NO:24 | protein | protein | *Oryza sativa* | PP |
| SEQ ID NO:25 | Protein - putative from Indica | protein | *Oryza sativa* | PP |
| SEQ ID NO:26 | *Taes 2DS* DE 9475523 | Promoter (954bp) | *Triticum aestivum* | PN |
| SEQ ID NO:27 | Taes_2DS_DE 9475523 | protein | *Triticum aestivum* | PP |
| SEQ ID NO:28 | scaffold:IWGS P1:IWGSC_C SS_2DS_scaff _5389501:195 8:5178:-1 | Genomic | *Triticum aestivum* | PN |
| SEQ ID NO:29 | scaffold:IWGS P1:IWGSC_C SS_2DS_scaff _5389501:195 8:5178:-1 | CDS | *Triticum aestivum* | PN |
| SEQ ID NO: 30 | PHN130859-Muint26 | oligonucleotide | Artificial sequence | PN |
| SEQ ID NO: 31 | PHN130859-Muint26-PE2.0 | oligonucleotide | Artificial sequence | PN |
| SEQ ID NO: 32 | PE1.0 | oligonucleotide | Artificial sequence | PN |
| SEQ ID NO: 33 | ZM MIRNA SEND PRECURSOR 396H | oligonucleotide | Artificial sequence | PN |
| SEQ ID NO: 34 | ZM-NRF4A | oligonucleotide | Artificial sequence | PN |
| SEQ ID NO: 35 | ZM MRNA PRECURSOR 396H | oligonucleotide | Artificial sequence | PN |
| SEQ ID NO: 36 | ZM-NRF4A 396H STAR | oligonucleotide | Artificial sequence | PN |
| SEQ ID NO: 37 | ZM MIRNA SEND PRECURSOR 396H | oligonucleotide | Artificial sequence | PN |

As used herein, an "isolated" or "purified" polynucleotide or polypeptide or biologically active portion thereof, is substantially or essentially free from components that normally accompany or interact with the polynucleotide or polypeptide as found in its naturally occurring environment. Thus, an isolated or purified polynucleotide or polypeptide is substantially free of other cellular material or culture medium when produced by recombinant techniques or substantially free of chemical precursors or other chemicals when chemically synthesized. Optimally, an "isolated" polynucleotide is free of sequences (optimally protein encoding sequences) that naturally flank the polynucleotide (i.e., sequences located at the 5' and 3' ends of the polynucleotide) in the genomic DNA of the organism from which the polynucleotide is derived. For example, in various aspects of the disclosure, the isolated polynucleotide can contain less than about 5 kb, 4 kb, 3 kb, 2 kb, 1 kb, 0.5 kb or 0.1 kb of nucleotide sequence that naturally flank the polynucleotide in genomic DNA of the cell from which the polynucleotide is derived. A polypeptide that is substantially free of cellular material includes preparations of polypeptides having less than about 30%, 20%, 10%, 5% or 1% (by dry weight) of contaminating protein. When the polypeptide of the disclosure or biologically active portion thereof is recombinantly produced, optimally culture medium represents less than about 30%, 20%, 10%, 5% or 1% (by dry weight) of chemical precursors or non-protein-of-interest chemicals.

As used herein, polynucleotide or polypeptide is "recombinant" when it is artificial or engineered, or derived from an artificial or engineered protein or nucleic acid. For example, a polynucleotide that is inserted into a vector or any other heterologous location, e.g., in a genome of a recombinant organism, such that it is not associated with nucleotide sequences that normally flank the polynucleotide as it is found in nature is a recombinant polynucleotide. A polypeptide expressed *in vitro* or *in vivo* from a recombinant polynucleotide is an example of a recombinant polypeptide. Likewise, a polynucleotide sequence that does not appear in nature, for example, a variant of a naturally occurring gene is recombinant.

A "control" or "control plant" or "control plant cell" provides a reference point for measuring changes in phenotype of the subject plant or plant cell, and may be any suitable plant or plant cell. A control plant or plant cell may comprise, for example: (a) a wild-type or native plant or cell, i.e., of the same genotype as the starting material for the genetic alteration which resulted in the subject plant or cell; (b) a plant or plant cell of the same genotype as the starting material but which has been transformed with a null construct (i.e., with a construct which has no known effect on the trait of interest, such as a construct comprising a marker gene); (c) a plant or plant cell which is a non-transformed segregant among progeny of a subject plant or plant cell; (d) a plant or plant cell which is genetically identical to the subject plant or plant cell but which is not exposed to the same treatment (e.g., herbicide treatment) as the subject plant or plant cell; (e) the subject plant or plant cell itself, under conditions in which the gene of interest is not expressed or (f) a plant or plant cell which is a non-transformed with a construct containing a silencing element.

### VIII. Sequence Identity

The following terms are used to describe the sequence relationships between two or more nucleic acids or polynucleotides: (a) "reference sequence", (b) "comparison window", (c) "sequence identity", (d) "percentage of sequence identity" and (e) "substantial identity".

As used herein, "reference sequence" is a defined sequence used as a basis for sequence comparison. A reference sequence may be a subset or the entirety of a specified sequence; for example, as a segment of a full-length cDNA or gene sequence or the complete cDNA or gene sequence.

As used herein, "comparison window" makes reference to a contiguous and specified segment of a polynucleotide sequence, wherein the polynucleotide sequence in the comparison window may comprise additions or deletions (i.e., gaps) compared to the reference sequence (which does not comprise additions or deletions) for optimal alignment of the two sequences. Generally, the comparison window is at least 20 contiguous nucleotides in length, and optionally can be 30, 40, 50, 100 or longer. Those of skill in the art understand that to avoid a high similarity to a reference sequence due to inclusion of gaps in the polynucleotide sequence, a gap penalty is typically introduced and is subtracted from the number of matches.

Methods of alignment of sequences for comparison are well known in the art. Thus, the determination of percent sequence identity between any two sequences can be accomplished using a mathematical algorithm. Non-limiting examples of such mathematical algorithms are the algorithm of Myers and Miller, (1988) CABIOS 4:11-17; the algorithm of Smith et al., (1981) Adv. Appl. Math. 2:482; the algorithm of Needleman and Wunsch, (1970) J. Mol. Biol. 48:443-453; the algorithm of Pearson and Lipman, (1988) Proc. Natl. Acad. Sci. 85:2444-2448; the algorithm of Karlin and Altschul, (1990) Proc. Natl. Acad. Sci. USA 872:264, modified as in Karlin and Altschul, (1993) Proc. Natl. Acad. Sci. USA 90:5873-5877.

Computer implementations of these mathematical algorithms can be utilized for comparison of sequences to determine sequence identity. Such implementations include, but are not limited to: CLUSTAL in the PC/Gene program (available from Intelligenetics, Mountain View, Calif.); the ALIGN program (Version 2.0) and GAP, BESTFIT, BLAST, FASTA and TFASTA in the GCG Wisconsin Genetics Software Package^{®}, Version 10 (available from Accelrys Inc., 9685 Scranton Road, San Diego, Calif., USA). Alignments using these programs can be performed using the default parameters. The CLUSTAL program is well described by Higgins etal., (1988) Gene 73:237-244 (1988); Higgins et al., (1989) CABIOS 5:151-153; Corpet et al., (1988) Nucleic Acids Res. 16:10881-90; Huang et al., (1992) CABIOS 8:155-65 and Pearson, et al. (1994) Meth. Mol. Biol. 24:307-331. The ALIGN program is based on the algorithm of Myers and Miller, (1988) *supra.* A PAM120 weight residue table, a gap length penalty of 12, and a gap penalty of 4 can be used with the ALIGN program when comparing amino acid sequences. The BLAST programs of Altschul et al., (1990) J. Mol. Biol. 215:403 are based on the algorithm of Karlin and Altschul, (1990) *supra.* BLAST nucleotide searches can be performed with the BLASTN program, score=100, word length=12, to obtain nucleotide sequences homologous to a nucleotide sequence encoding a protein of the disclosure. BLAST protein searches can be performed with the BLASTX program, score=50, word length=3, to obtain amino acid sequences homologous to a protein or polypeptide of the disclosure. To obtain gapped alignments for comparison purposes, Gapped BLAST (in BLAST 2.0) can be utilized as described in Altschul et al., (1997) Nucleic Acids Res. 25:3389. Alternatively, PSI-BLAST (in BLAST 2.0) can be used to perform an iterated search that detects distant relationships between molecules. See, Altschul *et al.,* (1997) *supra.* When utilizing BLAST, Gapped BLAST and PSI-BLAST, the default parameters of the respective programs (e.g., BLASTN for nucleotide sequences, BLASTX for proteins) can be used. See the web site of the National Center for Biotechnology Information on the World Wide Web at ncbi.nlm.nih.gov. Alignment may also be performed manually by inspection.

Unless otherwise stated, sequence identity/similarity values provided herein refer to the value obtained using GAP Version 10 using the following parameters: % identity and % similarity for a nucleotide sequence using GAP Weight of 50 and Length Weight of 3, and the nwsgapdna.cmp scoring matrix; % identity and % similarity for an amino acid sequence using GAP Weight of 8 and Length Weight of 2, and the BLOSUM62 scoring matrix; or any equivalent program thereof. As used herein, "equivalent program" is any sequence comparison program that, for any two sequences in question, generates an alignment having identical nucleotide or amino acid residue matches and an identical percent sequence identity when compared to the corresponding alignment generated by GAP Version 10.

The GAP program uses the algorithm of Needleman and Wunsch, *supra,* to find the alignment of two complete sequences that maximizes the number of matches and minimizes the number of gaps. GAP considers all possible alignments and gap positions and creates the alignment with the largest number of matched bases and the fewest gaps. It allows for the provision of a gap creation penalty and a gap extension penalty in units of matched bases. GAP must make a profit of gap creation penalty number of matches for each gap it inserts. If a gap extension penalty greater than zero is chosen, GAP must, in addition, make a profit for each gap inserted of the length of the gap times the gap extension penalty. Default gap creation penalty values and gap extension penalty values in Version 10 of the GCG Wisconsin Genetics Software Package^{®} for protein sequences are 8 and 2, respectively. For nucleotide sequences the default gap creation penalty is 50 while the default gap extension penalty is 3. The gap creation and gap extension penalties can be expressed as an integer selected from the group of integers consisting of from 0 to 200. Thus, for example, the gap creation and gap extension penalties can be 0, 1, 2, 3, 4, 5, 6, 7, 8, 9, 10, 15, 20, 25, 30, 35, 40, 45, 50, 55, 60, 65 or greater.

GAP presents one member of the family of best alignments. There may be many members of this family, but no other member has a better quality. GAP displays four figures of merit for alignments: Quality, Ratio, Identity, and Similarity. The Quality is the metric maximized in order to align the sequences. Ratio is the quality divided by the number of bases in the shorter segment. Percent Identity is the percent of the symbols that actually match. Percent Similarity is the percent of the symbols that are similar. Symbols that are across from gaps are ignored. A similarity is scored when the scoring matrix value for a pair of symbols is greater than or equal to 0.50, the similarity threshold. The scoring matrix used in Version 10 of the GCG Wisconsin Genetics Software Package^{®} is BLOSUM62 (see Henikoff and Henikoff, (1989) Proc. Natl. Acad. Sci. USA 89:10915).

As used herein, "sequence identity" or "identity" in the context of two nucleic acid or polypeptide sequences makes reference to the residues in the two sequences that are the same when aligned for maximum correspondence over a specified comparison window. When percentage of sequence identity is used in reference to proteins it is recognized that residue positions which are not identical often differ by conservative amino acid substitutions, where amino acid residues are substituted for other amino acid residues with similar chemical properties (e.g., charge or hydrophobicity) and, therefore, do not change the functional properties of the molecule. When sequences differ in conservative substitutions, the percent sequence identity may be adjusted upwards to correct for the conservative nature of the substitution. Sequences that differ by such conservative substitutions are said to have "sequence similarity" or "similarity". Means for making this adjustment are well known to those of skill in the art. Typically, this involves scoring a conservative substitution as a partial rather than a full mismatch, thereby increasing the percentage sequence identity. Thus, for example, where an identical amino acid is given a score of one and a non-conservative substitution is given a score of zero, a conservative substitution is given a score between zero and one. The scoring of conservative substitutions is calculated, e.g., as implemented in the program PC/GENE (Intelligenetics, Mountain View, Calif.).

As used herein, "percentage of sequence identity" means the value determined by comparing two optimally aligned sequences over a comparison window, wherein the portion of the polynucleotide sequence in the comparison window may comprise additions or deletions (i.e., gaps) as compared to the reference sequence (which does not comprise additions or deletions) for optimal alignment of the two sequences. The percentage is calculated by determining the number of positions at which the identical nucleic acid base or amino acid residue occurs in both sequences to yield the number of matched positions, dividing the number of matched positions by the total number of positions in the window of comparison, and multiplying the result by 100 to yield the percentage of sequence identity.

The term "substantial identity" of polynucleotide sequences means that a polynucleotide comprises a sequence that has at least 70% sequence identity, optimally at least 80%, more optimally at least 90% and most optimally at least 95%, compared to a reference sequence using an alignment program using standard parameters. One of skill in the art will recognize that these values can be appropriately adjusted to determine corresponding identity of proteins encoded by two nucleotide sequences by taking into account codon degeneracy, amino acid similarity, and reading frame positioning. Substantial identity of amino acid sequences for these purposes normally means sequence identity of at least 60%, 70%, 80%, 90% and at least 95%.

### IX. Promoters

Various types of promoters can be employed in the methods and compositions provided herein. Promoters can drive expression in a manner that is cell-type-preferred, cell-type-specific, tissue-preferred or tissue-specific. Examples of promoters under developmental control include promoters that preferentially initiate transcription in certain tissues, such as leaves, roots, seeds or ovules. Such promoters are referred to as "tissue preferred". Promoters which initiate transcription only in certain tissue are referred to as "tissue specific". A "cell type" preferred promoter primarily drives expression in certain cell types in one or more organs, for example, vascular cells in roots, leaves or ovules. An "inducible" or "repressible" promoter is a promoter which is under environmental or chemical control. Examples of environmental conditions that may affect transcription by inducible promoters include anaerobic conditions or the presence of light. Tissue specific, tissue preferred, cell type specific, cell type preferred and inducible promoters constitute the class of "non-constitutive" promoters. A "constitutive" promoter is a promoter that is active under most environmental conditions and in all tissues throughout development.

Non-limiting examples of constitutive promoters include, for example, the core promoter of the *Rsyn7* promoter and other constitutive promoters disclosed in WO 1999/43838 and US Patent Number 6,072,050; the core CaMV *35S* promoter (Odell et al., (1985) Nature 313:810-812); the rice actin promoter (McElroy et al., (1990) Plant Cell 2:163-171); he ubiquitin promoter (Christensen et al., (1989) Plant Mol. Biol. 12:619-632 and Christensen et al., (1992) Plant Mol. Biol. 18:675-689); the *pEMU* promoter (Last et al., (1991) Theor. Appl. Genet. 81:581-588); the *MAS* promoter (Velten et al., (1984) EMBO J. 3:2723-2730); and the *ALS* promoter (US Patent Number 5,659,026). Other constitutive promoters include, for example, US Patent Numbers 5,608,149; 5,608,144; 5,604,121; 5,569,597; 5,466,785; 5,399,680; 5,268,463; 5,608,142 and 6,177,611.

One of skill will recognize that the sequences encoding elements or polypeptides can be placed into an expression cassette. Expression cassettes are discussed elsewhere herein. Any promoter of interest can be operably linked to the sequence encoding the elements or polypeptides, including, for example, constitutive promoters, tissue-preferred promoters, tissue-specific promoters, female sporocyte-specific promoters, female sporocyte-preferred promoters, female gametophyte-specific promoters, female gametophyte-preferred promoters, female gamete-specific promoters, female gamete-preferred promoters, ovule tissue-preferred promoters, an ovule tissue-preferred promoter that is active in at least one non-gametophyte tissue in a plant ovule, seed-preferred, embryo-preferred and/or endosperm preferred promoters. Many such promoters have been described elsewhere herein or are known in the art.

By "promoter" is intended a regulatory region of DNA usually comprising a TATA box capable of directing RNA polymerase II to initiate RNA synthesis at the appropriate transcription initiation site for a particular polynucleotide sequence. A promoter may additionally comprise other recognition sequences generally positioned upstream or 5' to the TATA box, referred to as upstream promoter elements, which influence the transcription initiation rate. The promoter sequences disclosed herein regulate (i.e., activate) transcription from the promoter region.

In some aspects of the disclosure, the methods and compositions include isolated polynucleotides comprising *Nrf4* promoters. The *Nrf4* promoter nucleotide sequences include those set forth in SEQ ID NOS: 11, 15, 19, 21, 23 and 26, active variants and fragments thereof. In one aspect of the disclosure, an expression construct includes any of the polynucleotides set forth in SEQ ID NOS: 11, 15, 19, 21, 23 and 26 operably linked to the polynucleotide of interest or any polynucleotide having at least 95%, 96%, 97%, 98% or 99% sequence identity to the sequence set forth in SEQ ID NO: 11, 15, 19, 21, 23 and 26, wherein said polynucleotide retains the ability to direct expression of an operably linked polynucleotide in ovule developmental tissue prior to the initiation of sporogenesis and/or prior to or during meiosis.

Fragments and variants of each of the promoter nucleotide sequences set forth in SEQ ID NOS: 11, 15, 19, 21, 23 and 26 are further provided herein. Fragments of a promoter polynucleotide may retain biological activity and, hence, retain transcriptional regulatory activity. Thus, fragments of a promoter nucleotide sequence may range from at least about 20 nucleotides, about 50 nucleotides, about 100 nucleotides, and up to the full-length polynucleotide of the disclosure. Thus, a fragment of promoter that drives expression during female sporogenesis, a female sporogenesis-related promoter, such as a promoter that expresses in ovule developmental tissue prior to and/or during meiosis, may encode a biologically active portion of a female sporogenesis-related promoter. A biologically active portion of a female sporogenesis-related promoter polynucleotide can be prepared by isolating a portion of one of the female sporogenesis-related promoter polynucleotides, and assessing the activity of the portion of the megasporogenesis-related promoter. Polynucleotides that are fragments of a female sporogenesis-related promoter polynucleotide comprise at least 16, 20, 50, 75, 100, 150, 200, 250, 300, 350, 400, 450, 500, 550, 600, 650, 700, 800, 900, 1,000, 1,100, 1,200, 1,300, 1,400, 1,500, 1,600, 1,700, 1,800, 1,900, 2000, nucleotides or up to the number of nucleotides present in a full-length female sporogenesis-related promoter polynucleotide disclosed herein.

For a promoter polynucleotide, a variant comprises a deletion and/or addition of one or more nucleotides at one or more internal sites within the native polynucleotide and/or a substitution of one or more nucleotides at one or more sites in the native polynucleotide. Generally, variants of a particular ovule tissue-preferred promoter will have at least about 40%, 45%, 50%, 55%, 60%, 65%, 70%, 75%, 80%, 85%, 90%, 91%, 92%, 93%, 94%, 95%, 96%, 97%, 98%, 99% or more sequence identity to that particular polynucleotide as determined by sequence alignment programs and parameters described elsewhere herein.

Thus, any of the *Nrf4* promoters, variants, and fragments thereof may be utilized to regulate a number of genes and developmental processes during female sporogenesis. The *Nrf4* promoter may be used to ectopically express an RNA resulting in protein production, silencing, gene modification, modification of other RNA(s), or catalysis of a reaction.

As discussed herein, various promoters can be employed in the methods and compositions provided herein, including: promoters to express sequences encoding elements that silence or reduce *Nrf4* activity and/or recombination activity. An *Nrf 4* promoter, variant, or fragment thereof can be operably linked to any of the sequences encoding silencing elements or polypeptides disclosed herein or known in the art. In one aspect of the disclosure, the *Nrf4* promoter may be utilized in constructs designed to modify or alter the meiotic process. In such an aspect of the disclosure, the construct may be used to express silencing elements that target *TAM, OSD1, SPO11, PRD1, PRD2, PRD3, DFO1, REC8, AM1, AM2, PAM1, PAM2, AS1, DSY1, DY1, ST1, EL1, DV1, VA1, VA2* and/or *PO1.*

It is recognized that additional domains can be added to the promoter sequences disclosed herein and thereby modulate the level of expression, the developmental timing of expression, or the tissue type that expression occurs in. See, particularly, Australian Patent Number AU-A-77751/94 and US Patent Numbers 5,466,785 and 5,635,618.

Any of the promoter sequences employed herein can be modified to provide for a range of expression levels of the heterologous nucleotide sequence. Thus, less than the entire promoter region may be utilized and the ability to drive expression of the nucleotide sequence of interest retained. It is recognized that expression levels of the mRNA may be altered in different ways with deletions of portions of the promoter sequences. The mRNA expression levels may be decreased, or alternatively, expression may be increased as a result of promoter deletions if, for example, there is a negative regulatory element (for a repressor) that is removed during the truncation process. Generally, at least about 20 nucleotides of an isolated promoter sequence will be used to drive expression of a nucleotide sequence.

Variant polynucleotides also encompass sequences derived from a mutagenic and recombinogenic procedure such as DNA shuffling. With such a procedure, one or more different promoter sequences can be manipulated to create a new female sporogenesis-related or ovule tissue-preferred promoter possessing the desired properties. Strategies for such DNA shuffling are described elsewhere herein.

Methods are available in the art for determining if a promoter sequence retains the ability to regulate transcription in the desired temporal and spatial pattern. Such activity can be measured by Northern blot analysis. See, for example, Sambrook et al., (1989) Molecular Cloning: A Laboratory Manual (2d ed., Cold Spring Harbor Laboratory Press, Plainview, New York). Alternatively, biological activity of the promoter can be measured using assays specifically designed for measuring the activity and/or level of the polypeptide being expressed from the promoter. Such assays are known in the art.

It is recognized that to increase transcription levels, enhancers may be utilized in combination with the promoter disclosed herein. Enhancers are nucleotide sequences that act to increase the expression of a promoter region. Enhancers are known in the art and include the SV40 enhancer region, and the 35S enhancer element. Some enhancers are also known to alter normal promoter expression patterns, for example, by causing a promoter to be expressed constitutively when without the enhancer, the same promoter is expressed only in one specific tissue or a few specific tissues.

Modifications of the promoters disclosed herein can provide for a range of expression of the heterologous nucleotide sequence. Thus, they may be modified to be weak promoters or strong promoters. Generally, a "weak promoter" means a promoter that drives expression of a coding sequence at a low level. A "low level" of expression is intended to mean expression at levels of about 1/10,000 transcripts to about 1/100,000 transcripts to about 1/500,000 transcripts. Conversely, a strong promoter drives expression of a coding sequence at a high level or at about 1/10 transcripts to about 1/100 transcripts to about 1/1,000 transcripts.

### X. Plants and Methods of Making

The methods disclosed herein involve introducing a polypeptide or polynucleotide into a plant. "Introducing" is intended to mean presenting to the plant the polynucleotide or polypeptide in such a manner that the sequence gains access to the interior of a cell of the plant. The methods disclosed herein do not depend on a particular method for introducing a sequence into a plant, only that the polynucleotide or polypeptides gains access to the interior of at least one cell of the plant. Methods for introducing polynucleotide or polypeptides into plants are known in the art including, but not limited to, stable transformation methods, transient transformation methods, and virus-mediated methods.

"Stable transformation" is intended to mean that the nucleotide construct introduced into a plant integrates into the genome of the plant and is capable of being inherited by the progeny thereof. "Transient transformation" is intended to mean that a polynucleotide is introduced into the plant and does not integrate into the genome of the plant or a polypeptide is introduced into a plant.

Transformation protocols as well as protocols for introducing polypeptides or polynucleotide sequences into plants may vary depending on the type of plant or plant cell, i.e., monocot or dicot, targeted for transformation. Suitable methods of introducing polypeptides and polynucleotides into plant cells include microinjection (Crossway et al., (1986) Biotechniques 4:320-334), electroporation (Riggs et al., (1986) Proc. Natl. Acad. Sci. USA 83:5602-5606, *Agrobacterium-*mediated transformation (US Patent Number 5,563,055 and US Patent Number 5,981,840), direct gene transfer (Paszkowski etal., (1984) EMBO J. 3:2717-2722), and ballistic particle acceleration (see, for example, US Patent Number 4,945,050; US Patent Number 5,879,918; US Patent Numbers 5,886,244 and 5,932,782; Tomes et al., (1995) in Plant Cell, Tissue, and Organ Culture: Fundamental Methods, ed. Gamborg and Phillips, (Springer-Verlag, Berlin); McCabe et al., (1988) Biotechnology 6:923-926), and Lec1 transformation (WO 2000/28058). Also see Weissinger et al., (1988) Ann. Rev. Genet. 22:421-477; Sanford et al., (1987) Particulate Science and Technology 5:27-37 (onion); Christou et al., (1988) Plant Physiol. 87:671-674 (soybean); McCabe et al., (1988) Bio/Technology 6:923-926 (soybean); Finer and McMullen, (1991) In Vitro Cell Dev. Biol. 27P:175-182 (soybean); Singh et al., (1998) Theor. Appl. Genet. 96:319-324 (soybean); Datta et al., (1990) Biotechnology 8:736-740 (rice); Klein et al., (1988) Proc. Natl. Acad. Sci. USA 85:4305-4309 (maize); Klein et al., (1988) Biotechnology 6:559-563 (maize); US Patent Numbers 5,240,855; 5,322,783 and 5,324,646; Klein et al., (1988) Plant Physiol. 91:440-444 (maize); Fromm et al., (1990) Biotechnology 8:833-839 (maize); Hooykaas-Van Slogteren et al., (1984) Nature 311:763-764; US Patent Number 5,736,369 (cereals); Bytebier et al., (1987) Proc. Natl. Acad. Sci. USA 84:5345-5349 (Liliaceae); De Wet et al., (1985) in The Experimental Manipulation of Ovule Tissues, ed. Chapman, et al., (Longman, New York), pp. 197-209 (pollen); Kaeppler et al., (1990) Plant Cell Reports 9:415-418 and Kaeppler et al., (1992) Theor. Appl. Genet. 84:560-566 (whisker-mediated transformation); D'Halluin et al., (1992) Plant Cell 4:1495-1505 (electroporation); Li et al., (1993) Plant Cell Reports 12:250-255 and Christou and Ford, (1995) Annals of Botany 75:407-413 (rice); Osjoda et al., (1996) Nature Biotechnology 14:745-750 (maize via *Agrobacterium tumefaciens*)*.*

In specific aspects of the disclosure, the various sequences employed in the methods and compositions disclosed herein (e.g., the silencing elements or constructs, *Nrf4* promoters or variants and fragments thereof) can be provided to a plant using a variety of transient transformation methods. Such transient transformation methods include, but are not limited to, the introduction of the various sequences employed in the methods and compositions disclosed herein (e.g., the silencing elements or constructs, *Nrf4* promoters or variants and fragments thereof) directly into the plant or the introduction of the transcript into the plant. Such methods include, for example, microinjection or particle bombardment. See, for example, Crossway et al., (1986) Mol Gen. Genet. 202:179-185; Nomura et al., (1986) Plant Sci. 44:53-58; Hepler et al., (1994) Proc. Natl. Acad. Sci. 91: 2176-2180, and Hush et al., (1994) Journal of Cell Science 107:775-784.

Alternatively, the various sequences employed in the methods and compositions disclosed herein (e.g., the silencing elements or constructs, *Nrf4* promoters or variants and fragments thereof) can be transiently transformed into the plant using techniques known in the art. Such techniques include viral vector system and the precipitation of the polynucleotide in a manner that precludes subsequent release of the DNA. Thus, the transcription from the particle-bound DNA can occur, but the frequency with which it is released to become integrated into the genome is greatly reduced. Such methods include the use particles coated with polyethyleneimine (PEI; Sigma #P3143).

In other aspects of the disclosure, the polynucleotide of the disclosure may be introduced into plants by contacting plants with a virus or viral nucleic acids. Generally, such methods involve incorporating a nucleotide construct of the disclosure within a viral DNA or RNA molecule. It is recognized that the various sequences employed in the methods and compositions disclosed herein (e.g., the silencing elements or constructs, *Nrf4* promoters or variants and fragments thereof) may be initially synthesized as part of a viral polyprotein, which later may be processed by proteolysis *in vivo* or *in vitro* to produce the desired recombinant protein. Further, it is recognized that promoters disclosed herein also encompass promoters utilized for transcription by viral RNA polymerases. Methods for introducing polynucleotides into plants and expressing a protein encoded therein, involving viral DNA or RNA molecules, are known in the art. See, for example, US Patent Numbers 5,889,191, 5,889,190, 5,866,785, 5,589,367, 5,316,931 and Porta et al., (1996) Molecular Biotechnology 5:209-221.

Methods are known in the art for the targeted insertion of a polynucleotide at a specific location in the plant genome. In one aspect of the disclosure, the insertion of the polynucleotide at a desired genomic location is achieved using a site-specific recombination system. See, for example, WO 1999/25821, WO 1999/25854, WO 1999/25840, WO 1999/25855, and WO 1999/25853. Briefly, the polynucleotide of the disclosure can be contained in a transfer cassette flanked by two non-recombinogenic recombination sites. The transfer cassette is introduced into a plant having stably incorporated into its genome a target site which is flanked by two non-recombinogenic recombination sites that correspond to the sites of the transfer cassette. An appropriate recombinase is provided and the transfer cassette is integrated at the target site. The polynucleotide of interest is thereby integrated at a specific chromosomal position in the plant genome.

Additional methods for targeted mutagenesis *in vivo* are known. For example, a DNA sequence having the desired sequence alteration can be flanked by sequences homologous to the genomic target. One can then select or screen for a successful homologous recombination event. See US Patent Number 5,527,695. Generally, such a vector construct is designed having two regions of homology to the genomic target which flank a polynucleotide having the desired sequence. Introduction of the vector into a plant cell will allow homologous recombination to occur and to produce an exchange of sequences between the homologous regions at the target site.

Such methods of homologous recombination can further be combined with agents that induce site-specific genomic double-stranded breaks in plant cells. Such double strand break agents can be engineered to produce the break at a targeted site and thereby enhance the homologous recombination events. See, for example, Puchta et al., (1996) Proc Natl Acad Sci USA 93:5055-5060; US Patent Application Publication Number 2005/0172365A1; US Patent Application Publication Number 2006/0282914, WO 2005/028942; WO 2004/067736 published August 12, 2004; US Patent Number 5,792,632; US Patent Number 6,610,545; Chevalier et al., (2002) Mol Cell 10:895-905; Chevalier et al., (2001) Nucleic Acids Res 29:3757-3774; Seligman et al., (2002) Nucleic Acids Res 30:3870-3879; US Patent Application Publication Number 2009/0133152, and WO 2005/049842.

The cells that have been transformed may be grown into plants in accordance with conventional ways. See, for example, McCormick et al., (1986) Plant Cell Reports 5:81-84. These plants may then be grown, and either pollinated with the same transformed strain or different strains, and the resulting progeny having constitutive expression of the desired phenotypic characteristic identified. Two or more generations may be grown to ensure that expression of the desired phenotypic characteristic is stably maintained and inherited and then seeds harvested to ensure expression of the desired phenotypic characteristic has been achieved. In this manner, the present disclosure provides transformed seed (also referred to as "transgenic seed") having a polynucleotide of the disclosure, for example, an expression cassette of the disclosure, stably incorporated into their genome.

As used herein, the term plant includes plant cells, plant protoplasts, plant cell tissue cultures from which plants can be regenerated, plant calli, plant clumps, and plant cells that are intact in plants or parts of plants, such as embryos, pollen, ovules, seeds, leaves, flowers, branches, fruits, kernels, ears, cobs, husks, stalks, roots, root tips, and anthers. Grain is intended to mean the mature seed produced by commercial growers for purposes other than growing or reproducing the species. Progeny, variants, and mutants of the regenerated plants are also included within the scope of the disclosure, provided that these parts comprise the introduced polynucleotides.

The methods and compositions disclosed herein may be used for transformation of any plant species, including, but not limited to, monocots and dicots. Examples of plant species of interest include, but are not limited to, corn (*Zea mays*)*, Brassica* sp. (e.g., *B. napus, B. rapa, B. juncea),* particularly those *Brassica* species useful as sources of seed oil, alfalfa (*Medicago sativa*)*,* rice (*Oryza sativa*)*,* rye (*Secale cereale*)*,* sorghum (*Sorghum bicolor, Sorghum vulgare*)*,* millet (e.g., pearl millet (*Pennisetum glaucum*)*,* proso millet (*Panicum miliaceum*)*,* foxtail millet (*Setaria italica*)*,* finger millet (*Eleusine coracana*))*,* sunflower (*Helianthus annuus*)*,* safflower (*Carthamus tinctorius*)*,* wheat (*Triticum aestivum*), soybean (*Glycine max*), tobacco (*Nicotiana tabacum*)*,* potato (*Solanum tuberosum*), peanuts (*Arachis hypogaea*)*,* cotton (*Gossypium barbadense, Gossypium hirsutum*)*,* sweet potato (*Ipomoea batatus*), cassava (*Manihot esculenta*)*,* coffee (*Coffea* spp.), coconut (*Cocos nucifera*)*,* pineapple (*Ananas comosus*), citrus trees (*Citrus* spp.), cocoa (*Theobroma cacao*)*,* tea (*Camellia sinensis*), banana (*Musa* spp.), avocado (*Persea americana*), fig (*Ficus casica*)*,* guava (*Psidium guajava*)*,* mango (*Mangifera indica*), olive (*Olea europaea*), papaya (*Caricapapaya*)*,* cashew (*Anacardium occidental*), macadamia (*Macadamia integrifolia*)*,* almond (*Prunus amygdalus*)*,* sugar beets (*Beta vulgaris*), sugarcane (*Saccharum* spp.), oats, barley, vegetables, ornamentals, and conifers.

Vegetables include tomatoes (*Lycopersicon esculentum*), lettuce (e.g., *Lactuca sativa*), green beans (*Phaseolus vulgaris*), lima beans (*Phaseolus limensis*), peas (*Lathyrus* spp.), and members of the genus *Cucumis,* such as cucumber (C. *sativus*)*,* cantaloupe (C. *cantalupensis*)*,* and musk melon (C. *melo*). Ornamentals include azalea (*Rhododendron* spp.), hydrangea (*Macrophylla hydrangea*), hibiscus (*Hibiscus rosasanensis*)*,* roses (*Rosa* spp.), tulips (*Tulipa* spp.), daffodils (*Narcissus* spp.), petunias (*Petunia hybrida*), carnation (*Dianthus caryophyllus*), poinsettia (*Euphorbia pulcherrima*)*,* and chrysanthemum.

Conifers that may be employed in practicing the present disclosure include, for example, pines such as loblolly pine (*Pinus taeda*)*,* slash pine (*Pinus elliotii*), ponderosa pine (*Pinus ponderosa*)*,* lodgepole pine (*Pinus contorta*)*,* Monterey pine (*Pinus radiata*), Douglas fir (*Pseudotsuga menziesii*), Western hemlock (*Tsuga canadensis*), Sitka spruce (*Picea glauca*)*,* redwood (*Sequoia sempervirens*)*,* true firs, such as silver fir (*Abies amabilis*) and balsam fir (*Abies balsamea*)*,* and cedars, such as Western red cedar (*Thuja plicata*) and Alaska yellow cedar (*Chamaecyparis nootkatensis*). In specific aspects of the disclosure, plants of the present disclosure are crop plants (for example, corn, alfalfa, sunflower, *Brassica sp.,* soybean, cotton, safflower, peanut, sorghum, wheat, millet, tobacco, etc.). In other aspects of the disclosure, corn and soybean plants are optimal, and in yet other aspects of the disclosure corn plants are optimal.

Other plants of interest include grain plants that provide seeds of interest, oil-seed plants, and leguminous plants. Seeds of interest include grain seeds, such as corn, wheat, barley, rice, sorghum, rye, etc. Oil-seed plants include cotton, soybean, safflower, sunflower, *Brassica sp.,* maize, alfalfa, palm, coconut, etc. Leguminous plants include beans and peas. Beans include guar, locust bean, fenugreek, soybean, garden beans, cowpea, mungbean, lima bean, fava bean, lentils, chickpea, etc.

### XI. Methods of Use

Also described are methods for producing viable non-reduced, or non-reduced and non-recombined, gametes as well as and methods of maintaining heterozygosity in offspring. In some aspects of the disclosure, the method comprises introducing into a plant a composition comprising a silencing element that reduces the level of a *Nrf4* target polynucleotide, thereby producing viable non-reduced, or non-reduced and non-recombined, gametes. In other aspects of the disclosure, the method comprises modifying endogenous *Nrf4* target polynucleotides, for example, *Nrf4* genes, using gene editing technologies, such as endonucleases, megenucleases, CRISPR-Cas guideRNA's or other polynucleotide guided double strand break reagentz or combinations thereof, so that the level or activity of a *Nrf4* target polynucleotide or Nrf4 polypeptide are reduced, thereby producing viable non-reduced, or non-reduced and non-recombined, gametes.

As described elsewhere herein, the silencing element can be introduced in a variety of ways. The silencing element can be expressed in a specific manner, for example, using inducible or tissue-preferred or developmentally regulated promoters that are discussed elsewhere herein. In one aspect of the disclosure, the silencing element is operably linked to a *Nrf4* promoter, variant or fragment thereof. In specific aspects of the disclosure, the silencing element is expressed in a plant ovule primordia, plant ovule, plant female sporocyte, plant female gametophyte, or plant female gamete.

In certain aspects of the disclosure, the methods and compositions include a plant cell that has the modified endogenous *Nrf4* gene or silencing element targeting *Nrf4.* The plant may produce non-reduced, or non-reduced and non-recombined, female gametes. Additionally, the modified *Nrf4* gene or *Nrf4* silencing element can be combined with other genes that are modified and/or other silencing elements that target other genes of interest.

For example, the plant cell having the modified endogenous *Nrf4* gene or silencing element targeting *Nrf4* may be combined or stacked with a silencing element that targets genes that play a role in recombination in order to create plants that produce, or non-reduced and, non-recombined, female gametes. For example, the recombination target genes include but are not limited to *SPO11, PRD1* (De Must et al., (2007) EMBO J. 26:4126-4137), *OSD1, TAM, REC8* (known as *Fad* in maize) *Ago 104* (Singh et al., (2011) Plant Cell. 23:443-458), *AM1, AM2, PAM1, PAM2, AS1, DSY1, DY1, ST1, EL1, DV1, VA1, VA2,* and *PO1* and combinations thereof.

In other aspects of the disclosure, the plant cell having the modified endogenous *Nrf4* gene or silencing element targeting *Nrf4* may be combined with one or more genes involved in recombination that have been modified, for example, by gene-editing technologies, to have decreased recombination activity in order to create plants that produce non-reduced, or non-reduced and non-recombined, female gametes. The non-reduced, or non-reduced and non-recombined, female gametes therefore may be produced using gene-editing techniques or silencing elements or combinations thereof.

The non-reduced, or non-reduced and non-recombined, female gametes can be stacked with traits desirable for disease or herbicide resistance (e.g., fumonisin detoxification genes (U.S. Pat. No. 5,792,931); avirulence and disease resistance genes (Jones et al., (1994) Science 266:789-793; Martin et al., (1993) Science 262:1432-1436; Mindrinos et al., (1994) Cell 78:1089-1099); acetolactate synthase (ALS) mutants that lead to herbicide resistance, such as the S4 and/or Hra mutations; inhibitors of glutamine synthase, such as phosphinothricin or basta (e.g., bar gene); and glyphosate resistance (EPSPS gene); traits desirable for processing or process products such as high oil (e.g., U.S. Pat. No. 6,232,529); modified oils (e.g., fatty acid desaturase genes (U.S. Pat. No. 5,952,544; WO 94/11516)); modified starches (e.g., ADPG pyrophosphorylases (AGPase), starch synthases (SS), starch branching enzymes (SBE), and starch debranching enzymes (SDBE)); and polymers or bioplastics (e.g., U.S. Pat. No. 5,602,321; beta-ketothiolase, polyhydroxybutyrate synthase, and acetoacetyl-CoA reductase (Schubert et al., (1988) J. Bacteriol. 170:5837-5847) facilitate expression of polyhydroxyalkanoates (PHAs)). One could also combine various polynucleotides, for example, polynucleotides providing agronomic traits such as male sterility (e.g., see U.S. Pat. No. 5,583,210), stalk strength, flowering time, or transformation technology traits, such as cell cycle regulation or gene targeting (e.g., WO 99/61619, WO 00/17364, and WO 99/25821).

These stacked combinations can be created by any method including, but not limited to, cross-breeding plants by any conventional or TopCross methodology, or genetic transformation. If the sequences are stacked by genetically transforming the plants, the polynucleotide sequences of interest can be combined at any time and in any order. For example, a transgenic plant comprising one or more desired traits can be used as the target to introduce further traits by subsequent transformation. The traits can be introduced simultaneously in a co-transformation protocol with the polynucleotides of interest provided by any combination of transformation cassettes. For example, if two sequences will be introduced, the two sequences can be contained in separate transformation cassettes (trans) or contained on the same transformation cassette (cis). Expression of the sequences can be driven by the same promoter or by different promoters. In certain cases, it may be desirable to introduce a transformation cassette that will suppress the expression of the polynucleotide of interest. This may be combined with any combination of other suppression cassettes or overexpression cassettes to generate the desired combination of traits in the plant. It is further recognized that polynucleotide sequences can be stacked at a desired genomic location using a site-specific recombination system. See, for example, WO99/25821, WO99/25854, WO99/25840, WO99/25855, and WO99/25853.

A further aspect of the disclosure includes methods of maintaining heterozygosity in a progeny plant that includes regenerating a progeny plant from a parent plant that has non-reduced, non-recombinant clonal gametes and has the same genotype as the parent plant. In some examples, the endogenous *Nrf4* gene and/or gene involved in recombination is disrupted in the parent and progeny plant. In an aspect, the methods disclosed herein can further comprise the step of introducing into the plant genome a disruption of the endogenous *Nrf4* gene, and regenerating a plant having such an altered genome.

In one example, compositions include an isolated polynucleotide comprising a nucleotide sequence comprising at least 19 consecutive nucleotides of any one of SEQ ID NOS: 3, 4, 5, 6, 7, 8, 10, 12, 13, 17, 18, 22, 28 and 29 or a complement thereof, wherein said polynucleotide encodes a silencing element that decreases *Nrf4* activity. In other aspects of the disclosure, compositions include an isolated polynucleotide comprising a nucleotide sequence comprising nucleotide sequence that hybridizes under stringent conditions to the full length complement of the nucleotide sequence of comprising any one of SEQ ID NOS: 3, 4, 5, 6, 7, 8, 10, 12, 13, 17, 18, 22, 28 and 29, wherein said polynucleotide encodes a silencing element that decreases *Nrf4* activity. In some aspects of the disclosure, the silencing element is part of an expression cassette operably linked to a female sporegenesis-related promoter. An expression cassette may express a polynucleotide disclosed herein as a double stranded RNA. An expression cassette comprising a heterologous polynucleotide disclosed herein, wherein said polynucleotide comprises a silencing element, which is expressed as a hairpin RNA.

### XII. Methods of Modulating Nrf4 Activity and/or Concentration

Methods and compositions are provided to modify the expression level and/or activity of a *Nrf4* polynucleotide and/or Nrf4 polypeptide in a plant cell, for example, an ovule primordia, ovule, female sporocyte, female gametophyte, or female gamete plant cell. In some aspects of the disclosure, methods and compositions include decreasing the *Nrf4* activity or expression level of a *Nrf4* polynucleotide, Nrf4 polypeptide, variant or fragment thereof in a plant cell, for example, an ovule primordia, ovule, female sporocyte, female gametophyte, or female gamete. Compositions suitable for this purpose are described elsewhere herein.

In some aspects of the disclosure, methods and compositions include increasing the *Nrf4* activity or expression level of a *Nrf4* polynucleotide, Nrf4 polypeptide, variant, orthologs or fragment thereof in a plant cell, for example, in an ovule primordia, ovule, female sporocyte, female gametophyte, or female gamete.

Biologically active variants of a *Nrf4* polynucleotide will have at least about 70%. 75%, 80%, 85%, 86%, 87%, 88%, 89%, 90%, 91%, 92%, 93%, 94%, 95%, 96%, 97%, 98%, 99% or more sequence identity to any polynucleotide encoding a Nrf4 polypeptide, including the polynucleotide of any one of SEQ ID NO: 3, 4, 5, 6, 7, 8, 10, 12, 13, 17, 18, 22, 28, and 29 as determined by sequence alignment programs and parameters described elsewhere herein.

Biologically active variants of an Nrf4 polypeptide (and the polynucleotide encoding the same) will have at least about 70%. 75%, 80%, 85%, 86%, 87%, 88%, 89%, 90%, 91%, 92%, 93%, 94%, 95%, 96%, 97%, 98%, 99% or more sequence identity to any Nrf4 polypeptide, including but not limited to, the polypeptide of any one identified in Table 1 , for example, as set forth in SEQ ID NOs: 1, 2, 9, 14, 16, 20, 24, 25, or 27 as determined by sequence alignment programs and parameters described elsewhere herein.

As discussed elsewhere herein, methods and compositions are provided which employ polynucleotides and polypeptides having *Nrf4* activity. Fragments and variants of *Nrf4* polynucleotides and Nrf4 polypeptides are also encompassed. By "fragment" is intended a portion of the polynucleotide or a portion of the amino acid sequence and hence protein encoded thereby. Fragments of a polynucleotide may encode protein fragments that retain non-reduction and/or non-recombination activity. Thus, fragments of a nucleotide sequence may range from at least about 20 nucleotides, about 50 nucleotides, about 100 nucleotides and up to the full-length polynucleotide encoding the Nrf4 polypeptides.

A fragment of a *Nrf4* polynucleotide that encodes a biologically active portion of a Nrf4 protein will encode at least 50, 75, 100, 150, 175, 200, 225, 250, 275, 300, 325, 350, 375, 400, 410, 415, 420, 425, 430, 435 or 440 contiguous amino acids or up to the total number of amino acids present in a full-length Nrf4 polypeptide.

Thus, a fragment of a *Nrf4* polynucleotide may encode a biologically active portion of a Nrf4 polypeptide. A biologically active portion of a Nrf4 polypeptide can be prepared by isolating a portion of one of the *Nrf4* polynucleotides, expressing the encoded portion of the Nrf4 polypeptides (e.g., by recombinant expression *in vitro*)*,* and assessing the activity of the portion of the Nrf4 protein. Polynucleotides that are fragments of a *Nrf4* nucleotide sequence comprise at least 16, 20, 50, 75, 100, 150, 200, 250, 300, 350, 400, 450, 500, 550, 600, 650, 700, 800, 900, 1,000, 1,100, 1,200, 1,300 or 1,400 contiguous nucleotides or up to the number of nucleotides present in a full-length *Nrf4* polynucleotide disclosed herein.

"Variant" protein is intended to mean a protein derived from the protein by deletion (i.e., truncation at the 5' and/or 3' end) and/or a deletion or addition of one or more amino acids at one or more internal sites in the native protein and/or substitution of one or more amino acids at one or more sites in the native protein. Variant proteins encompassed are biologically active, that is they continue to possess the desired biological activity of the native protein, that is, have non-reduction activity. Such variants may result from, for example, genetic polymorphism or from human manipulation.

"Variants" is intended to mean substantially similar sequences. For polynucleotides, a variant comprises a polynucleotide having a deletion (i.e., truncations) at the 5' and/or 3' end and/or a deletion and/or addition of one or more nucleotides at one or more internal sites within the native polynucleotide and/or a substitution of one or more nucleotides at one or more sites in the native polynucleotide. As used herein, a "native" polynucleotide or polypeptide comprises a naturally occurring nucleotide sequence or amino acid sequence, respectively. For polynucleotides, conservative variants include those sequences that, because of the degeneracy of the genetic code, encode the amino acid sequence of one of the Nrf4 polypeptides. Naturally occurring variants such as these can be identified with the use of well-known molecular biology techniques, as, for example, with polymerase chain reaction (PCR) and hybridization techniques as outlined below. Variant polynucleotides also include synthetically derived polynucleotides, such as those generated, for example, by using site-directed mutagenesis or gene synthesis but which still encode a Nrf4 polypeptide.

The Nrf4 polypeptides, active variants and fragments thereof may be altered in various ways including amino acid substitutions, deletions, truncations and insertions. Methods for such manipulations are generally known in the art. For example, amino acid sequence variants and fragments of the Nrf4 proteins can be prepared by mutations in the DNA. Methods for mutagenesis and polynucleotide alterations are well known in the art. See, for example, Kunkel, (1985) Proc. Natl. Acad. Sci. USA 82:488-492; Kunkel et al., (1987) Methods in Enzymol. 154:367-382; US Patent Number 4,873,192; Walker and Gaastra, eds. (1983) Techniques in Molecular Biology (MacMillan Publishing Company, New York) and the references cited therein. Guidance as to appropriate amino acid substitutions that do not affect biological activity of the protein of interest may be found in the model of Dayhoff et al., (1978) Atlas of Protein Sequence and Structure (Natl. Biomed. Res. Found. Washington, D.C.). Conservative substitutions, such as exchanging one amino acid with another having similar properties, may be optimal.

Thus, the genes and polynucleotides disclosed herein include both the naturally occurring sequences as well as DNA sequence variants. Likewise, the Nrf4 polypeptides and proteins encompass bothnaturally occurring polypeptides as well as variants and modified forms thereof. Mutations that will be made in the DNA encoding the variant must not place the sequence out of reading frame and optimally will not create complementary regions that could produce secondary mRNA structure. See, EP Patent Application Publication Number 75,444.

Variant polynucleotides and proteins also encompass sequences and proteins derived from a mutagenic and recombinogenic procedure such as DNA shuffling. With such a procedure, one or more different coding sequences can be manipulated to create a new Nrf4 polypeptide possessing the desired properties. In this manner, libraries of recombinant polynucleotides are generated from a population of related sequence polynucleotides comprising sequence regions that have substantial sequence identity and can be homologously recombined *in vitro* or *in vivo.* For example, using this approach, sequence motifs encoding a domain of interest may be shuffled between the *Nrf4* sequences disclosed herein and other known *Nrf4* genes to obtain a new gene coding for a protein with an improved property of interest, such as a decreased Kₘ in the case of an enzyme. Strategies for such DNA shuffling are known in the art. See, for example, Stemmer, (1994) Proc. Natl. Acad. Sci. USA 91:10747-10751; Stemmer, (1994) Nature 370:389-391; Crameri et al., (1997) Nature Biotech. 15:436-438; Moore et al., (1997) J. Mol. Biol. 272:336-347; Zhang et al., (1997) Proc. Natl. Acad. Sci. USA 94:4504-4509; Crameri et al., (1998)Nature 391:288-291, and US Patent Numbers 5,605,793 and 5,837,458.

Thus, constructs are provided comprising a promoter operably linked to a *Nrf4* polynucleotide that when expressed has *Nrf4* activity in a plant female gamete, female gametophyte, female sporocyte, ovule, or ovule primodium. In one aspect of the disclosure, compositions include an isolated polynucleotide comprising a nucleotide sequence comprising polynucleotides 70%,75%, 80%, 85%, 90%, 91%, 92%, 93%, 94%, 95%, 96%, 97%, 98% or 100% identical to of any one of the polynucleotides of SEQ ID NOS: 3, 4, 5, 6, 7, 8, 10, 12, 13, 17, 18, 22, 28 and 29 or a complement thereof.

In still further aspects of the disclosure, the polynucleotide encodes for a polypeptide set forth in SEQ ID NO: 1, 2, 9, 14, 16, 20, 24, 25 and 27 or an *Nrf4* polynucleotide encoding a Nrf4 polypeptide having at least 80%, 85%, 90%, 91%, 92%, 93%, 94%, 95%, 96%, 97%, 98% 99% or 100% sequence identity to the polypeptide set forth in SEQ ID NO: 1, 2, 9, 14, 16, 20, 24, 25 and 27.

The following examples are offered by way of illustration and not by way of limitation.

### EXAMPLES

The embodiments are further defined in the following Examples, in which parts and percentages are by weight and degrees are Celsius, unless otherwise stated. It should be understood that these Examples, while indicating embodiments of the disclosure, are given by way of illustration only.

### Example 1: Genetic Screen and Identification of the Maize (Zea mays L.) nrf4 Mutant

A genetic screen was conducted to identify mutants which form viable, non-reduced female gametes. The screen makes use of the developmental failure of maize endosperm that does not possess the normal 2 maternal to 1 paternal (2m: 1p) genome ratio (Lin, (1984) Genetics 100:475-486). When wild-type diploid plants, which produce normal, reduced (haploid) female gametes, are pollinated with diploid pollen (containing diploid male gametes) from a tetraploid parent, fertilization of the central cells results in imbalanced endosperm (2m:2p), leading to seed abortion. Plants of a mutagenized M₂ population that produce ears with plump seeds, presumably with a 4m:2p genome ratio in the endosperm, may possess a mutation producing non-reduced (diploid), functional female gametes.

Maize germplasm with very active *Mutator* (*Mu*) transposable elements at a high copy number were crossed to the public inbred line Oh43, and the resulting F₁ plants were self-pollinated to create M₂ families, which should segregate 1 homozygous wild-type (no insertion): 2 insertion heterozygous :1 insertion homozygous plant for any given insertion. Screening at the M₂ rather than F₁ stage (Singh *et al.,* (2011) *supra),* allows the discovery of recessive mutants affecting female sporogenesis including meiosis (Grossniklaus, (2001) *supra).* Approximately 3200 M₂ families (-15 to 20 plants each) were subsequently crossed as females with a tetraploid line. The tetraploid tester line contains the dominant embryo/endosperm color marker *R1-navajo* (*R1-nj*) to insure that seed was indeed produced by cross-pollination rather than unintended self- or sib-pollination between or within the female M₂ families. The tetraploid *Rl-nj* line was specifically generated for this purpose by crossing a diploid *Rl-nj* pollen parent to a public tetraploid W23 line that had been generated using the *elongate1* method (Birchler, (1994) in The Maize Handbook, eds. Freeling, Walbot (Springer, Berlin), pp. 394-395). Taking advantage of some diploid pollen that is produced spontaneously, rare tetraploid offspring with a 4m:2p genome ration in the endosperm can be recovered. Subsequent self- and sib-pollinations and selection for high pollen viability led to the isolation of a fully fertile, tetraploid tester line that is tetraplex for the *Rl-nj* allele *(Rl-nj*/ *Rl-nj*/ *Rl-nj*/ *Rl-nj).*

In this screen, four families were identified with plants possessing ears with plump (non-aborted) seeds. One of them, designated *non-reductive in female4 (nrf4*), contained a few ears with a very high proportion of plump seeds (FIG. 1). Progeny growth from a portion of such seeds confirmed viability and tetraploidy. In addition to crossing the *nrf4* mutant as females with the tetraploid line, the M₂ plants were used as males in crosses with the public inbred line W22. Individual plants that produced a high frequency of plump seeds also produced a high frequency of plump seeds in the cross with W22 (diploid), indicating that non-reduction was female-specific. The Fi plants produced from the cross of W22 x highfrequency plump-seeded plants were self-pollinated, and the resulting F₂ plants were crossed as females with tetraploid plants to confirm the initial phenotype. Out of a total of 57 ears evaluated, 14 produced a high frequency of plump seeds up to 85-100%; this fits a model of a monogenic recessive trait (χ², 1 d.f. = 0.94). This frequency (penetrance) of non-reduced, viable female gametes per ear is generally higher than reported for other maize mutants (30-60%, *elongate1* (*el1*; Rhoades and Dempsey, (1966) Genetics 54:505-22); 20-80%, *Dominant non-reduction 4* (*Dnr4*; Singh et al., 2011), *supra).*

### Example 2: Cloning of the nrf4 Allele

Plants producing plump seed in the second evaluation in Example 1 were crossed again as males to the public inbred line W22, the new F₁ was self-pollinated, and the resulting F₂ plants were once again crossed as females with the tetraploid *R1-nj* tester line. Samples of leaf tissue were taken from each pollinated plant. Fifteen plants which were subsequently shown to produce a high percentage of plump seed were evaluated by highthroughput sequencing (HTS) analysis of their *Mu*-flanking regions. This method utilizes the sequence in the terminal-inverted-repeat (TIR) region, which is conserved in all *Mu* elements.

DNA was isolated from the 15 samples described above. DNA was normalized to 20ng/ul, sheared on Covaris E210 (10% Duty Cycle, Intensity of 0.5, 1000cycles/burst for 45 seconds), and processed through the Flanking Sequence library prep method. This library preparation involved repairing the DNA ends using the NEBNext End Repair Enzyme mix; followed by adding an A-base to the 3' end of the DNA with NEBNext Klenow Fragment. An indexed adapter was then ligated using NEBNext Quick T4 DNA ligase (New England Biolabs). Libraries undergo 2 rounds of PCR (PCR1, PCR2) using 2x Phusion DNA Polymerase Mastermix (New England Biolabs), for 18 and 20 rounds of amplification, respectively. The 1^{st} primer in PCR1 (PHN130859-Muint26) and PCR2 (PHN130859-Muint26-PE2.0) has a sequence that matches the common region in all *Mutator* elements, except that PCR2 primers had specific sequences attached to enable Illumina sequencing. The 2^{nd} primer in each reaction (PE1.0) has sequence that matches the ligated adapter. For nucleotide sequences of all primers see Table 2.

**Table 2.**

| **Mu Oligo name** | **Sequence** |
|---|---|
| PHN130859-Muint26 | AGAAGCCAACGCCAWCGCCTCYATTTC (SEQ ID NO: 30) |
| PHN130859-Muint26-PE2.0 | CAAGCAGAAGACGGCATACGAGATAGAAGCCAACGCCAWC GCCTCYATTTC (SEQ ID NO: 31) |
| PE1.0 | AATGATACGGCGACCACCGAGATCTACACTCTTTCCCTACA CGACGCTCTTCCGATCT (SEQ ID NO: 32) |

After PCR amplification, the products were purified using Ampure XP beads and ran on a Fragment Analyzer (Advanced Analytical Technologies Inc.) to check for quality and quantity. Products of all samples were pooled and sequenced on an Illumina HiSeq2000 or GAIIx sequencer with a run configuration of single read 108 bases. Bases 1 to 6 are the barcode to identify the sample, base 7 is a T-base and is removed. This leaves 101bp of sequence for analysis. *nrf4* events had approximately 1.5-10 million reads for each sample.

One *Mu*-flanking sequence was found to be present in all 15 plants, and thus most likely to be the causal insertion of the *nrf4* non-reduction phenotype. Reverse genetics based on TUSC (Meeley and Briggs, (1995) Maize Genetics Cooperation Newsletter 69:67-82) was used to isolate two additional *Mu* insertion alleles in the same gene, and they also showed the non-reductive phenotype. Thus, the correct identity of the isolated *Nrf4* gene was clearly confirmed using additional alleles. The *Mu* insertions are located in locus GRMZM2G148133 (on the world wide web at http://www.maizegdb.org/cgibin/displaygmresults.cgi?term=GRMZM2G148133), which has homologs in several grass species but does not have any functional annotation (SEQ ID NOs #17 - 19).

### Example 3: MicroRNA Experiment to Phenocopy the nrf4 Mutant

Based on the *Nrf4* gene sequence, a micro RNA sequence was designed against the *Nrf4* coding region (see SEQ ID NOs #33 - 37). This sequence was inserted into a transformation vector using the maize ubiquitin promoter to drive expression. Transformed maize plants carrying a single copy of the construct were generated. These plants were used as females in crosses with diploid male plants, which produced normal haploid pollen. As a result of these crosses the transformed plants showed seed set, indicating that they had reduced female gametes as well, and that the *nrf4* non-reduced female gamete phenotype was not achieved with this particular microRNA design. A proprietary software program for gene silencing indicated the microRNA sequence had folding at its 5'end, and without wishing to be bound by this theory, it is possible that this design affected microRNA function.

### Example 4: Expression analysis of Nrf4 transcript using in situ hybridization

Based on the *Nrf4* gene sequence, an antisense gene-specific probe was designed covering the first, second, and part of the third exon of the *Nrf4* gene. This *Nrf4* probe sequence was inserted into the *pDRIVE* vector (Qiagen) suited for *in vitro* transcription. A sense riboprobes labeled with digoxygenin (DIG) was synthesized using the SP6 polymerase.

Immature wild-type ears of inbred line W22 (approximately 3-4 cm in size) were fixed, embedded in paraffin, and sectioned using a microtome. Tissue sections were subjected to RNA *in situ* hybridization with the riboprobe described above. The riboprobe was detected using anti-DIG-antibodies fused with alkaline phosphatase (Roche) and the signal was developed by providing the alkaline phosphatase substrate Western Blue (Roche). Images were taken on a transmission light microscope equipped with a digital color camera (Leica).

The *Nrf4* transcript showed a consistently high expression in a portion of the ovule's nucellus around megaspore mother cell (MMC) and in the integument primordia (FIG. 2A). In the developing and mature MMC, the *Nrf4* signal varied from intermediate to very strong depending on the particular section (FIG. 2B & FIG. 2C). This variation may related to different stages of MMC differentiation that are cytologically not distinguishable in these sections.

### Example 5: Expression Analysis of the Nrf4 gene

A DNA sequence primarily consisting of the putative promoter region of the *Nrf4* gene (nt 1-1737 of SEQ ID NO: 7) was synthesized (GenScript), with the following modifications: 32bp were added 5' in order to add an *EcoRI* restriction site, and STOP codons in all 6 reading frames; nt 521 was changed from G to C to eliminate an *Eco RI* restriction site; nt 841 was changed from A to T to eliminate an ORF; and nt 1737 was changed from A to G to be compatible with a *BamHI* restriction site. The resulting DNA was cloned into a vector cut with *EcoRI* and *BamHI,* creating a plant transcriptional unit (PTU) consisting of the putative *Nrf4* promoter, *Adh1* intron, the ZS-green1 fluorescent protein (Clontech) CDS, and the *pinII* transcription terminator. This entry vector was subsequently cloned into a plant transformation vector using Gateway multisite cloning (Invitrogen), introduced into *Agrobacterium tumefaciens* by electroporation, and transformed into maize by standard methods. Such methods for preparing the constructs and transforming maize are as previously described and known in the art.

Immature ears (approximately 2.5 and 3 cm in size) of regenerated transgenic plants were examined. Hand cross-sections were made through the ear, but horizontal longitudinal through the axis of the ovule primordia. Images were observed on an epi-fluorescence microscope using a GFP filter set for the Zs-green1 and a DAPI filter set for the natural blue auto-fluorescence of the tissue. Ovule-specific Zs-green1-positive fluorescence was observed in the 2.5 cm ear, which is pre-meiotic in development, prior to female sporocyte differentiation (FIG. 3A). Similar expression was also observed in the 3 cm ear sections, at which stage female sporocytes are fully differentiation, but also pre-meiotic (FIG. 3B). Consequently, the *Nrf4* promoter may be utilized in constructs designed to modify or alter the meiotic process, a key step in apomixis.

### Example 6: Production of a Tetraploid "Haploid" Inducer

Marker analysis of female meiotic behavior in non-reduced eggs can be simplified by elimination of the male genome. In maize, some lines (generally referred to as "haploid inducers") produce pollen capable of inducing gynogenesis, resulting in the production of maternal haploid progeny that originate exclusively from the egg cell (Röber et al., (2005) Maydica 50:275-283). A major locus, designated *gynogenesis inducer1* (*ggi1*), controls gynogenesis in maize (Barret et al., (2008) Theor. Appl. Genet. 117:581-594).

Haploid inducers may possess a dominant marker gene to distinguish biparental diploid from maternal haploid progeny. One example is *R1-nj*, which produces a purple scutellum and a purple crown of the aleurone (endosperm) of F₁ kernels crossed with unpigmented females. In haploid induction crosses, kernels with a maternally-derived haploid embryo and a regular triploid endosperm possess a colorless embryo (scutellum) and a purple crown (endosperm), whilst Fi (diploid) kernels have pigmentation in both embryo and endosperm.

In order to eliminate the male genome in mutants with non-reduced egg (and central) cells and produce viable seeds, an inducer must be created which is tetraploid (to preserve the 2m-1p genome ratio in the endosperm). To accomplish this, the fact that normal diploid maize will produce a certain low frequency of diploid eggs (0.5-5/1000; Bauman, (1961) Maize Genet. Coop. Newslet. 35:128-130) was exploited. Consequently, the *R1-nj* tetraploid maize line described above was used as a male in a cross with a diploid inducer line (*R1-nj, ggi1*), and the rare plump kernels were selected. Seeds from these kernels were grown and confirmed to be tetraploid by standard flow cytometry. Such plants would be homozygous for *R1-nj* and heterozygous for *ggi1*; specifically as a duplex tetraploid *(ggi1*/*ggi1*/*Ggi1*/*Ggi1*). These plants were self-pollinated. The progeny were analyzed for markers flanking *ggi1,* and homozygous *ggi1* plants (frequency 1/36) were selected. These plants were also self-pollinated and crossed as a male to a tetraploid line lacking *R1-nj.* Approximately 5% of the progeny possessed a colorless embryo and a purple crown. Flow cytometry confirmed that these progeny were in fact diploid. This tetraploid version of a "haploid" inducer (THI) was used in crosses with *nrf4* homozygous females to generate maternal diploid offspring.

### Example 7: Marker Analysis of nrf4 Meiotic Behavior Demonstrates the Production of Maternal Clones

In order to test for possible background/modifier effects on the *nrf4* phenotype and to insure a high frequency of heterozygous markers for analysis, the *nrf4* mutant allele was crossed 4 times (BC₃) into 4 different inbred lines (Public lines B73 & W22; and 2 Pioneer proprietary lines Line Z & Line Y). The converted inbred lines were crossed to each other, and *nrf4* homozygotes were identified. The F₁ *nrf4* homozygous plants were crossed as females with THI (described in Example 5). Diploid progeny of maternal origin was selected as described in Example 5, with the additional step of confirming the lack of *ggi1-*flanking markers. The key to self-reproducing hybrids (SRH), as in naturally occurring apomicts, is progeny that are identical to the maternal parent. In addition to non-reduction, this requires a lack of recombination and chromosome segregation, resulting in the full retention of heterozygosity. To assess the level at which heterozygosity is retained in the progeny, both the female Fi *nrf4* homozygous maternal plants and their selected diploid progeny were tissue-sampled. Both DNA preparation and single-nucleotide polymorphism (SNP) analysis were conducted using standard methods known to one of ordinary skill in the art. A total of 384 SNP markers, distributed as evenly as possible across the maize genome, were analyzed. Depending on the particular F₁, and the particular plant within a cross, the number of informative (heterozygous in the maternal plant) markers varied (Table 3). In total, 10 out of 227 progeny (4.4%) retained 100% of the heterozygosity present in its maternal parent. Thus, *nrf4* is capable of producing "clonal" gametes, albeit at a low frequency. The differences in the frequency within the individual Fi hybrids may indicate the presence of background effects or modifier gene(s), which may be exploited through selection to produce higher levels of clonal female gametes.

**Table 3.**

| F₁ Pedigree | Number of Parent-Progeny Pairs | Number of Informative Markers Per Pair | Number of Progeny Retaining 100% Heterozygousity |
|---|---|---|---|
| B73 x Line Z | 63 | 117-181 | 4 (6.3%) |
| Line Y x Line Z | 18 | 100-109 | 2(11%) |
| W22 x B73 | 111 | 105-197 | 5 (4.5%) |
| W22 x Line Z | 28 | 122-143 | 0 (0%) |
| W22 x Line Y | 7 | 140 | 0 (0%) |

### Example 8: Combining nrf4 with other Mutants

Depending on the exact mechanisms of non-reduced gamete production, their genetic content may differ substantially (Crismani et al., (2013) J. Exp. Bot. 64:55-65). Two aspects affect the genotype of 2n gametes: (1) segregation of homologous or sister chromatids (centromeres), and (2) the presence or absence of recombination (Crismani *et al.,* (2013) *supra).* In some cases, the genetic products of meiosis show that sister centromeres segregate from each other, but recombination occurs. Consequently, heterozygosity is preserved at centromeres and tends to be reduced away from the centromeres. This is generally referred to as a first division restitution (FDR) mechanism. Examples include *ps1 and jason* mutants in *Arabidopsis* (Crismani *et al.,* (2013) *supra).* In other cases, the opposite effect occurs as sister chromatids (centromeres) migrate together (recombination occurs). Heterozygosity is completely lost at the centromeres, and the resulting homozygosity tends to be reduced with distance from the centromeres. This is sometimes referred to as second division restitution (SDR). Examples include *osd1* and *tam* in *Arabidopsis* (Crismani *et al.,* (2013) *supra).* Thus, beyond non-reduced gamete production, the engineering of apomixis in a species requires a mitotic-like division to replace meiosis - sister chromatids segregating without recombination. This has been accomplished in *Arabidopsis* at a high frequency in the triple mutant *spo11 rec8 osd1* (MiMe; d'Erfurth et al., (2009) PLoS Biology 7:e1000124), although the level of female non-reduced spores in *osd1* mutants is estimated to be -85%.

Despite the low frequency of occurrence, the ability of *nrf4* mutants to produce non-reduced, non-recombined but viable gametes makes *Nrf4* a good candidate for the engineering of apomixis. Indeed, maternal clonal progeny could be produced in crosses with THI. Given its overall very high degree of non-reduced female gamete viability, *nrf4* may be combined with other mutants in order to increase the frequency of non-recombination and replace meiosis with a mitotic-like division. In particular, mutants which completely eliminate recombination, such as *spo11 and prd1* (De Muyt et al., (2007) EMBO J. 26:4126-4137). Also, *nrf4* could serve as a substitute for *osd1* or *tam* in combination with *rec8* (known as *afd1* in maize) and *spo11* in a maize version analogous to *Arabidopsis* MiMe. Other known maize mutants affecting meiosis, which could be combined with *nrf4,* are *ago104* (Singh *et al.,* (2011) *supra)* as well as *am1, am2, pam1, pam2, as1, dsy1, dy1, st1, el1, dv1, va1, va2,* and *po1* (Table 12: Meiotic Mutants, (1997) in Mutants of Maize, eds. Neuffer, Coe, Wessler (Cold Spring Harbor Laboratory Press, New York), p. 314).

All publications and patent applications mentioned in the specification are indicative of the level of those skilled in the art to which this disclosure pertains.

## Claims

1. A method of producing a plant, the method comprising:
disrupting by genome editing, transposon tagging, or mutagenizing an endogenous *Nrf4* gene in a plant or plant cell, wherein the plant cell is an ovule primordia, ovule, female gametophyte, or female gamete, thereby producing viable non-reduced, or viable non-reduced and non-recombined, gametes; and
regenerating a plant having the disruption of the endogenous *Nrf4* gene:
(A) wherein the endogenous *Nrf4* gene comprises a polynucleotide selected from the group consisting of:
(a) a polynucleotide having at least 80% sequence identity, as determined by the GAP algorithm under default parameters, to the full length sequence of a polynucleotide selected from the group consisting of SEQ ID NOs: 3, 4, 5, 6, 7, 8, 10, 12, 13, 17, 18, 22, 28, and 29, and wherein the polynucleotide encodes a polypeptide that has the function of reducing, or reducing and recombining, female sporocytes, female gametophytes, or female gametes during meiosis;
(b) a polynucleotide having at least 90% sequence identity, as determined by the GAP algorithm under default parameters, to the full length sequence of a polynucleotide selected from the group consisting of SEQ ID NOs: 3, 4, 5, 6, 7, 8, 10, 12, 13, 17, 18, 22, 28, and 29, and wherein the polynucleotide encodes a polypeptide that has the function of reducing, or reducing and recombining, female sporocytes, female gametophytes, or female gametes during meiosis;
(c) a polynucleotide selected from the group consisting of SEQ ID NOs: 3, 4, 5, 6, 7, 8, 10, 12, 13, 17, 18, 22, 28, and 29;
(d) a polynucleotide that is a fragment comprising at least 450 contiguous nucleotides of the polynucleotide of (a), (b), or (c), and wherein the polynucleotide encodes a polypeptide that has the function of reducing, or reducing and recombining, female sporocytes, female gametophytes, or female gametes during meiosis; or
(B) wherein the endogenous *Nrf4* gene encodes for a Nrf4 polypeptide selected from the group consisting of:
(a) a polypeptide comprising an amino acid sequence being identical to or having at least 80% identity with SEQ ID NOs: 1, 2, 9, 14, 16, 20, 24, 25 or 27, or an ortholog thereof, and wherein the polypeptide has the function of reducing, or reducing and recombining, female sporocytes, female gametophytes, or female gametes during meiosis;
(b) a polypeptide comprising an amino acid sequence being identical to or having at least 90% identity with SEQ ID NOs: 1, 2, 9, 14, 16, 20, 24, 25 or 27, or an ortholog thereof, and wherein the polypeptide has the function of reducing, or reducing and recombining, female sporocytes, female gametophytes, or female gametes during meiosis;
(c) a polypeptide comprising an amino acid sequence selected from SEQ ID NOs: 1, 2, 9, 14, 16, 20, 24, 25 or 27, or an ortholog thereof; and
(d) a polypeptide comprising an amino acid sequence which is a fragment comprising at least 150 contiguous amino acids of the amino acid sequence of (a), (b), or (c), and wherein the polypeptide has the function of reducing, or reducing and recombining, female sporocytes, female gametophytes, or female gametes during meiosis.

2. The method of claim 1:
(I) wherein said disruption inhibits expression, activity, or both expression and activity of a product of said endogenous *Nrf4* gene compared to a corresponding control plant lacking such a disruption;
(II) further comprising a step of disrupting in the plant, in addition to the *Nrf4* gene, at least one other gene involved in recombination; or
(III) further comprising a step of disrupting in the plant, in addition to the *Nrf4* gene, at least one other endogenous gene involved in meiosis.

3. The method of claim 2, wherein the at least one other endogenous gene involved in meiosis is *REC8* or an ortholog thereof.

4. The method of claim 3, further comprising:
(i) disrupting one of the following genes selected from the group consisting of *SPO11, PRD1, PRD2, PRD3, DFO1,* and any orthologs thereof; or
(ii) disrupting *OSD1* or *TAM1,* or orthologs thereof, or any combination thereof.

5. The method of any one of claims 1 to 4, wherein the female gametophyte or female gamete undergoes parthenogenesis or genome elimination.

6. The method of claim 5, further comprising producing a seed, wherein the seed comprises parthenogenetically-derived clonal embryos.

7. The method of claim 1:
(a) wherein the plant is maize, wheat, rice, sorghum, barley, oat, lawn grass, rye, soybean, Brassica, or sunflower; or
(b) wherein the plant with the disrupted endogenous *Nrf4* gene comprises viable non-reduced, viable or non-reduced and non-recombined, gametes when compared to a control plant.

8. The method of any of claims 1 to 7, wherein the plant produced by said method is a non-naturally occurring plant with developing ovules comprising non-reduced, or non-reduced and non-recombined clonal female gametes.

9. A nucleic acid construct comprising a nucleotide sequence encoding an element that silences *Nrf4* activity in a plant cell selected from the group consisting of an ovule primordia, ovule, female gametophyte, or female gamete; wherein:
(A) the element comprises at least 19 nucleotides of any one of SEQ ID NOs: 3, 4, 5, 6, 7, 8, 10, 12, 13, 17, 18, 22, 28, and 29 or a complement thereof; or
(B) the element comprises a nucleotide sequence that hybridizes under stringent conditions to a full length complement of a nucleotide sequence of any one of SEQ ID NOs: 3, 4, 5, 6, 7, 8, 10, 12, 13, 17, 18, 22, 28, and 29;
wherein the construct further comprises a heterologous promoter functional in plants operably linked to the element.

10. The nucleic acid construct of claim 9, wherein the element is operably linked to a promoter in sense orientation or in antisense orientation.

11. The nucleic acid construct of claim 9 or 10, wherein the promoter is a constitutive, inducible, cell- or tissue-preferred, or cell- or tissue-specific promoter.

12. A plant cell transformed with the construct of claim 9; or a plant comprising said plant cell.

13. The plant cell or plant of claim 12, wherein:
(a) the plant comprises viable non-reduced, or non-reduced and non-recombined, gametes compared to a control plant; or
(b) the plant is Arabidopsis, maize, wheat, rice, sorghum, barley, oat, lawn grass, rye, soybean, Brassica, sunflower, millet, sugarcane, cotton, safflower, tobacco, or alfalfa;
or a seed from said plant.

14. A method of decreasing expression of one or more *Nrf4* genes in the plant by introducing into the plant by transformation the nucleic acid construct of any one of claims 9 to 11, wherein the decreased expression of the one or more *Nrf4* genes is in an ovule primordia, ovule, female sporocyte, female gametophyte, or female gamete of the plant.

15. The method of claim 14, wherein:
(I) the one or more *Nrf4* genes is endogenous to the plant;
(II) the *Nrf4* gene comprises a polynucleotide selected from the group consisting of:
(a) a polynucleotide having at least 80% sequence identity, as determined by the GAP algorithm under default parameters, to the full length sequence of a polynucleotide selected from the group consisting of SEQ ID NOs: 3, 4, 5, 6, 7, 8, 10, 12, 13, 17, 18, 22, 28, and 29, and wherein the polynucleotide encodes a polypeptide that has the function of reducing, or reducing and recombining, female sporocytes, female gametophytes, or female gametes during meiosis;
(b) a polynucleotide having at least 90% sequence identity, as determined by the GAP algorithm under default parameters, to the full length sequence of a polynucleotide selected from the group consisting of SEQ ID NOs: 3, 4, 5, 6, 7, 8, 10, 12, 13, 17, 18, 22, 28, and 29, and wherein the polynucleotide encodes a polypeptide that has the function of reducing, or reducing and recombining, female sporocytes, female gametophytes, or female gametes during meiosis;
(c) a polynucleotide selected from the group consisting of SEQ ID NOs: 3, 4, 5, 6, 7, 8, 10, 12, 13, 17, 18, 22, 28, and 29;
(d) a polynucleotide which is a fragment comprising at least 450 contiguous nucleotides of the polynucleotide of (a), (b), or (c), and wherein the polynucleotide encodes a polypeptide that has the function of reducing, or reducing and recombining, female sporocytes, female gametophytes, or female gametes during meiosis;
(e) a polynucleotide which is complementary to the polynucleotide of (a), (b), (c), or (d); and
(f) a polynucleotide that hybridizes to the polynucleotide of (a), (b), (c), (d), or (e); or
(III) the endogenous *Nrf4* gene encodes a polypeptide selected from the group consisting of:
(a) a polypeptide comprising an amino acid sequence being identical to or having at least 80% identity with SEQ ID NOs: 1, 2, 9, 14, 16, 20, 24, 25 and 27, or an ortholog thereof, and wherein the polypeptide has the function of reducing, or reducing and recombining, female sporocytes, female gametophytes, or female gametes during meiosis;
(b) a polypeptide comprising an amino acid sequence being identical to or having at least 90% identity with SEQ ID NOs: 1, 2, 9, 14, 16, 20, 24, 25 and 27, or an ortholog thereof, and wherein the polypeptide has the function of reducing, or reducing and recombining, female sporocytes, female gametophytes, or female gametes during meiosis;
(c) a polypeptide comprising an amino acid sequence selected from SEQ ID NOs: 1, 2, 9, 14, 16, 20, 24, 25 and 27, or an ortholog thereof; and
(d) a polypeptide comprising an amino acid sequence which is a fragment comprising at least 150 contiguous amino acids of the amino acid sequence of (a), (b), or (c), and wherein the polypeptide has the function of reducing, or reducing and recombining, female sporocytes, female gametophytes, or female gametes during meiosis.

16. An expression cassette comprising in operable linkage to a heterologous nucleotide sequence of interest a nucleic acid molecule comprising a polynucleotide selected from the group consisting of:
(a) a polynucleotide sequence comprising the polynucleotide sequence SEQ ID NOs: 11, 15, 19, 21, 23 and 26;
(b) a polynucleotide sequence comprising a fragment comprising at least 16 contiguous nucleotides of the polynucleotide sequence of SEQ ID NOs: 11, 15, 19, 21, 23 and 26, wherein the polynucleotide sequence initiates transcription in a plant cell;
(c) a polynucleotide which is complementary to the polynucleotide of (a) or (b); and
(d) a polynucleotide that hybridizes under stringent conditions to the polynucleotide of (a), (b) or (c);
wherein said polynucleotide initiates transcription in a cell of an ovule primordia, ovule, female sporocyte, female gametophyte, or female gamete.

17. A plant comprising the expression cassette of claim 16.

18. The expression cassette of claim 16 or the plant of claim 17, wherein:
(a) the plant is maize, wheat, rice, sorghum, barley, oat, lawn grass, rye, soybean, Brassica, sunflower, millet, sugarcane, cotton, safflower, tobacco, or alfalfa; or a transgenic seed of said plant; or
(b) the heterologous nucleotide sequence of interest comprises a gene product that confers non-reduction, non-recombination, parthenogenesis, or any combination thereof.

19. A method for expressing a polynucleotide sequence in a plant or a plant cell, said method comprising introducing into the plant or the plant cell by transformation an expression cassette comprising a promoter operably linked to a polynucleotide sequence of interest, wherein said promoter comprises any of the polynucleotide sequences of claim 16.

20. The method of claim 19, wherein the polynucleotide sequence of interest comprises a gene product that confers non-reduction, non-recombination, parthenogenesis, or any combination thereof.

21. An expression cassette comprising a heterologous promoter operably linked to a polynucleotide comprising a member selected from the group consisting of:
(a) a polynucleotide that encodes the polypeptide of SEQ ID NOs: 1, 2, 9, 14, 16, 20, 24, 25 or 27;
(b) a polynucleotide comprising the sequence set forth in SEQ ID NOs: 3, 4, 5, 6, 7, 8, 10, 12, 13, 17, 18, 22, 28 or 29;
(c) a polynucleotide comprising at least 300 nucleotides in length which hybridizes under stringent conditions to a polynucleotide of (a) or (b), wherein the conditions include hybridization in 40 to 45% formamide, 1 M NaCl, 1% SDS at 37°C and a wash in 0.5 X to 1 X SSC at 55 to 60°C;
(d) a polynucleotide having at least 80% sequence identity to SEQ ID NOs: 3, 4, 5, 6, 7, 8, 10, 12, 13, 17, 18, 22, 28 or 29, wherein the % sequence identity is based on the entire coding region and is determined by BLAST 2.0 under default parameters, wherein the polynucleotide encodes a polypeptide that confers the function of reducing, or reducing and recombining, female sporocytes, female gametophytes, or female gametes during meiosis; and
(e) a polynucleotide fully complementary to a polynucleotide of any one of (a) to (d).

22. An isolated polypeptide selected from the group consisting of:
(a) a polypeptide comprising any one of SEQ ID NOs: 9, 14, 16, 20, 24, 25 or 27, wherein said polypeptide confers the function of reducing, or reducing and recombining, female sporocytes, female gametophytes, or female gametes during meiosis;
(b) a polypeptide that is at least 80% identical to the amino acid sequence of any of SEQ ID NOs: 9, 14, 16, 20, 24, 25 or 27, wherein said polypeptide confers the function of reducing, or reducing and recombining, female sporocytes, female gametophytes, or female gametes during meiosis;
(c) a polypeptide that is encoded by a nucleic acid molecule comprising a nucleotide sequence that is at least 80% identical to any one of SEQ ID NOs: 8, 10, 12, 17, 18, 28 or 29 or a complement thereof, wherein said polypeptide confers the function of reducing, or reducing and recombining, female sporocytes, female gametophytes, or female gametes during meiosis;
(d) a polypeptide that is encoded by a nucleic acid molecule that hybridizes with a nucleic acid probe consisting of the nucleotide sequence of any of SEQ ID NOs: 8, 10, 12, 17, 18, 28 or 29, or a complement thereof following at least one wash in 0.2X SSC at 55°C for 20 minutes, wherein said polypeptide confers the function of reducing, or reducing and recombining, female sporocytes, female gametophytes, or female gametes during meiosis; and
(e) a fragment comprising at least 150 consecutive amino acids of any of SEQ ID NOs: 9, 14, 16, 20, 24, 25 or 27, wherein said polypeptide confers the function of reducing, or reducing and recombining, female sporocytes, female gametophytes, or female gametes during meiosis.

23. A plant cell transformed with the expression cassette of claim 21, or a plant regenerated from said plant cell, wherein said plant comprises the expression cassette.

24. The plant of claim 23, wherein the plant is maize, wheat, rice, sorghum, barley, oat, lawn grass, rye, soybean, Brassica, sunflower, millet, sugarcane, cotton, safflower, tobacco, or alfalfa; or a seed from said plant.

25. A method of increasing the expression of one or more *Nrf4* genes in a plant cell by introducing into the plant by transformation:
(A) a polynucleotide comprising a member selected from the group consisting of:
(a) a polynucleotide that encodes the polypeptide of SEQ ID NOs: 1, 2, 9, 14, 16, 20, 24, 25 or 27;
(b) a polynucleotide comprising the sequence set forth in SEQ ID NOs: 3, 4, 5, 6, 7, 8, 10, 12, 13, 17, 18, 22, 28 or 29;
(c) a polynucleotide comprising at least 300 nucleotides in length which hybridizes under stringent conditions to a polynucleotide of (a) or (b), wherein the conditions include hybridization in 40 to 45% formamide, 1 M NaCl, 1% SDS at 37°C and a wash in 0.5 X to 1 X SSC at 55 to 60°C;
(d) a polynucleotide having at least 80% sequence identity to SEQ ID NOs: 3, 4, 5, 6, 7, 8, 10, 12, 13, 17, 18, 22, 28 or 29, wherein the % sequence identity is based on the entire coding region and is determined by BLAST 2.0 under default parameters, wherein the polynucleotide encodes a polypeptide that confers the function of reducing, or reducing and recombining, female sporocytes, female gametophytes, or female gametes during meiosis; and
(e) a polynucleotide fully complementary to a polynucleotide of any one of (a) to (d);
(B) any of the polypeptides of claim 22; or
(C) the expression cassette of claim 21.

## Patentansprüche

1. Verfahren zur Herstellung einer Pflanze, wobei das Verfahren Folgendes umfasst:
Disruptieren eines endogenen Nrf4-Gens in einer Pflanze oder Pflanzenzelle durch Genomeditierung, Transposon-Tagging oder Mutagenisierung, wobei es sich bei der Pflanzenzelle um ein Samenanlagen-Primordium, eine Samenanlage, einen weiblichen Gametophyten oder einen weiblichen Gameten handelt, wodurch lebensfähige nicht-reduzierte oder lebensfähige nicht-reduzierte und nicht-rekombinierte Gameten produziert werden; und
Regenerieren einer Pflanze, welche die Disruption des endogenen Nrf4-Gens aufweist:
(A) wobei das endogene Nrf4-Gen ein Polynukleotid umfasst, ausgewählt aus der Gruppe bestehend aus:
(a) einem Polynukleotid mit mindestens 80 % Sequenzidentität, wie bestimmt durch den GAP-Algorithmus unter Standardparametern, zu der Volllängensequenz eines Polynukleotids, ausgewählt aus der Gruppe bestehend aus den SEQ ID NOs: 3, 4, 5, 6, 7, 8, 10, 12, 13, 17, 18, 22, 28 und 29, und wobei das Polynukleotid ein Polypeptid codiert, das die Funktion des Reduzierens oder Reduzierens und Rekombinierens weiblicher Sporozyten, weiblicher Gametophyten oder weiblicher Gameten während der Meiose hat;
(b) einem Polynukleotid mit mindestens 90 % Sequenzidentität, wie bestimmt durch den GAP-Algorithmus unter Standardparametern, zu der Volllängensequenz eines Polynukleotids, ausgewählt aus der Gruppe bestehend aus den SEQ ID NOs: 3, 4, 5, 6, 7, 8, 10, 12, 13, 17, 18, 22, 28 und 29, und wobei das Polynukleotid ein Polypeptid codiert, das die Funktion des Reduzierens oder Reduzierens und Rekombinierens weiblicher Sporozyten, weiblicher Gametophyten oder weiblicher Gameten während der Meiose hat;
(c) einem Polynukleotid, ausgewählt aus der Gruppe bestehend aus den SEQ ID NOs: 3, 4, 5, 6, 7, 8, 10, 12, 13, 17, 18, 22, 28 und 29;
(d) einem Polynukleotid, das ein Fragment ist, das mindestens 450 zusammenhängende Nukleotide des Polynukleotids von (a), (b) oder (c) umfasst, und wobei das Polynukleotid ein Polypeptid codiert, das die Funktion des Reduzierens oder Reduzierens und Rekombinierens weiblicher Sporozyten, weiblicher Gametophyten oder weiblicher Gameten während der Meiose hat; oder
(B) wobei das endogene Nrf4-Gen für ein Nrf4-Polypeptid codiert, ausgewählt aus der Gruppe bestehend aus:
(a) einem Polypeptid, umfassend eine Aminosäuresequenz, die zu den SEQ ID NOs: 1, 2, 9, 14, 16, 20, 24, 25 oder 27 oder einem Ortholog davon identisch ist oder mindestens 80 % Identität zu diesen aufweist, und wobei das Polypeptid die Funktion des Reduzierens oder Reduzierens und Rekombinierens weiblicher Sporozyten, weiblicher Gametophyten oder weiblicher Gameten während der Meiose hat;
(b) einem Polypeptid, umfassend eine Aminosäuresequenz, die zu den SEQ ID NOs: 1, 2, 9, 14, 16, 20, 24, 25 oder 27 oder einem Ortholog davon identisch ist oder mindestens 90 % Identität zu diesen aufweist, und wobei das Polypeptid die Funktion des Reduzierens oder Reduzierens und Rekombinierens weiblicher Sporozyten, weiblicher Gametophyten oder weiblicher Gameten während der Meiose hat;
(c) einem Polypeptid, umfassend eine Aminosäuresequenz, ausgewählt aus den SEQ ID NOs: 1, 2, 9, 14, 16, 20, 24, 25 oder 27 oder einem Ortholog davon; und
(d) einem Polypeptid, umfassend eine Aminosäuresequenz, die ein Fragment ist, das mindestens 150 zusammenhängende Aminosäuren der Aminosäuresequenz von (a), (b) oder (c) umfasst, und wobei das Polypeptid die Funktion des Reduzierens oder Reduzierens und Rekombinierens weiblicher Sporozyten, weiblicher Gametophyten oder weiblicher Gameten während der Meiose hat.

2. Verfahren nach Anspruch 1:
(I) wobei die Disruption die Expression, Aktivität oder sowohl die Expression als auch die Aktivität eines Produkts des endogenen Nrf4-Gens im Vergleich zu einer entsprechenden Kontrollpflanze ohne eine solche Disruption hemmt;
(II) ferner umfassend einen Schritt des Disruptierens mindestens eines weiteren Gens, das an der Rekombination beteiligt ist, in der Pflanze zusätzlich zum Nrf4-Gen; oder
(III) ferner umfassend einen Schritt des Disruptierens mindestens eines weiteren endogenen Gens, das an der Meiose beteiligt ist, in der Pflanze zusätzlich zum Nrf4-Gen.

3. Verfahren nach Anspruch 2, wobei das mindestens eine weitere endogene Gen, das an der Meiose beteiligt ist, *REC8* oder ein Ortholog davon ist.

4. Verfahren nach Anspruch 3, ferner umfassend:
(i) Disruptieren eines der folgenden Gene, ausgewählt aus der Gruppe bestehend aus *SPO11, PRD1, PRD2, PRD3, DFOl* und jeglichen Orthologen davon; oder
(ii) Disruptieren von *OSD1* oder *TAM1* oder Orthologen davon, oder einer Kombination davon.

5. Verfahren nach einem der Ansprüche 1 bis 4, wobei der weibliche Gametophyt oder weibliche Gamet eine Parthenogenese oder eine Genom-Eliminierung durchläuft.

6. Verfahren nach Anspruch 5, ferner umfassend die Herstellung eines Samens, wobei der Samen parthenogenetisch abgeleitete klonale Embryonen umfasst.

7. Verfahren nach Anspruch 1:
(a) wobei es sich bei der Pflanze um Mais, Weizen, Reis, Sorghum, Gerste, Hafer, Rasengras, Roggen, Sojabohne, Kohl oder Sonnenblume handelt; oder
(b) wobei die Pflanze mit dem disruptierten endogenen Nrf4-Gen im Vergleich zu einer Kontrollpflanze lebensfähige nicht-reduzierte, lebensfähige oder nicht-reduzierte und nicht-rekombinierte Gameten umfasst.

8. Verfahren nach einem der Ansprüche 1 bis 7, wobei die durch das Verfahren hergestellte Pflanze eine nicht natürlich vorkommende Pflanze mit sich entwickelnden Samenanlagen, umfassend nicht-reduzierte oder nicht-reduzierte und nicht-rekombinierte klonale weibliche Gameten, ist.

9. Nukleinsäurekonstrukt, umfassend eine Nukleotidsequenz, die ein Element codiert, das die Nrf4-Aktivität in einer Pflanzenzelle, ausgewählt aus der Gruppe bestehend aus einem Samenanlagen-Primordium, einer Samenanlage, einem weiblichen Gametophyten oder einem weiblichen Gameten, stummschaltet; wobei:
(A) das Element mindestens 19 Nukleotide einer der SEQ ID NOs: 3, 4, 5, 6, 7, 8, 10, 12, 13, 17, 18, 22, 28 und 29 oder eines Komplements davon umfasst; oder
(B) das Element eine Nukleotidsequenz umfasst, die unter stringenten Bedingungen an ein Volllängen-Komplement einer Nukleotidsequenz einer der SEQ ID NOs: 3, 4, 5, 6, 7, 8, 10, 12, 13, 17, 18, 22, 28 und 29 hybridisiert;
wobei das Konstrukt ferner einen in Pflanzen funktionellen heterologen Promotor umfasst, der mit dem Element funktionsfähig verknüpft ist.

10. Nukleinsäurekonstrukt nach Anspruch 9, wobei das Element mit einem Promotor in Sense-Orientierung oder Antisense-Orientierung funktionsfähig verknüpft ist.

11. Nukleinsäurekonstrukt nach Anspruch 9 oder 10, wobei der Promotor ein konstitutiver, induzierbarer, zell- oder gewebebevorzugter oder zell- oder gewebespezifischer Promotor ist.

12. Pflanzenzelle, die mit dem Konstrukt nach Anspruch 9 transformiert ist; oder Pflanze, die diese Pflanzenzelle umfasst.

13. Pflanzenzelle oder Pflanze nach Anspruch 12, wobei:
(a) die Pflanze lebensfähige, im Vergleich zu einer Kontrollpflanze nicht-reduzierte oder nicht-reduzierte und nicht-rekombinierte Gameten umfasst; oder
(b) die Pflanze Arabidopsis, Mais, Weizen, Reis, Sorghum, Gerste, Hafer, Rasengras, Roggen, Sojabohne, Kohl, Sonnenblume, Hirse, Zuckerrohr, Baumwolle, Saflor, Tabak oder Luzerne ist;
oder ein Samen von dieser Pflanze.

14. Verfahren zur Verringerung der Expression eines oder mehrerer Nrf4-Gene in der Pflanze durch Einbringen des Nukleinsäurekonstrukts nach einem der Ansprüche 9 bis 11 in die Pflanze mittels Transformation, wobei die verringerte Expression des einen oder der mehreren Nrf4-Gene in einem Samenanlagen-Primordium, einer Samenanlage, einem weiblichen Sporozyten, einem weiblichen Gametophyten oder einem weiblichen Gameten der Pflanze vorliegt.

15. Verfahren nach Anspruch 14, wobei:
(I) das eine oder die mehreren Nrf4-Gene hinsichtlich der Pflanze endogen sind;
(II) das Nrf4-Gen ein Polynukleotid umfasst, ausgewählt aus der Gruppe bestehend aus:
(a) einem Polynukleotid mit mindestens 80 % Sequenzidentität, wie bestimmt durch den GAP-Algorithmus unter Standardparametern, zu der Volllängensequenz eines Polynukleotids, ausgewählt aus der Gruppe bestehend aus den SEQ ID NOs: 3, 4, 5, 6, 7, 8, 10, 12, 13, 17, 18, 22, 28 und 29, und wobei das Polynukleotid ein Polypeptid codiert, das die Funktion des Reduzierens oder Reduzierens und Rekombinierens weiblicher Sporozyten, weiblicher Gametophyten oder weiblicher Gameten während der Meiose hat;
(b) einem Polynukleotid mit mindestens 90 % Sequenzidentität, wie bestimmt durch den GAP-Algorithmus unter Standardparametern, zu der Volllängensequenz eines Polynukleotids, ausgewählt aus der Gruppe bestehend aus den SEQ ID NOs: 3, 4, 5, 6, 7, 8, 10, 12, 13, 17, 18, 22, 28 und 29, und wobei das Polynukleotid ein Polypeptid codiert, das die Funktion des Reduzierens oder Reduzierens und Rekombinierens weiblicher Sporozyten, weiblicher Gametophyten oder weiblicher Gameten während der Meiose hat;
(c) einem Polynukleotid, ausgewählt aus der Gruppe bestehend aus den SEQ ID NOs: 3, 4, 5, 6, 7, 8, 10, 12, 13, 17, 18, 22, 28 und 29;
(d) einem Polynukleotid, das ein Fragment ist, das mindestens 450 zusammenhängende Nukleotide des Polynukleotids von (a), (b) oder (c) umfasst, und wobei das Polynukleotid ein Polypeptid codiert, das die Funktion des Reduzierens oder Reduzierens und Rekombinierens weiblicher Sporozyten, weiblicher Gametophyten oder weiblicher Gameten während der Meiose hat;
(e) einem Polynukleotid, das zu dem Polynukleotid von (a), (b), (c) oder (d) komplementär ist; und
(f) einem Polynukleotid, das an das Polynukleotid von (a), (b), (c), (d) oder (e) hybridisiert; oder
(III) das endogene Nrf4-Gen ein Polypeptid codiert, ausgewählt aus der Gruppe bestehend aus:
(a) einem Polypeptid, umfassend eine Aminosäuresequenz, die zu SEQ ID NOs: 1, 2, 9, 14, 16, 20, 24, 25 und 27 oder einem Ortholog davon identisch ist oder mindestens 80 % Identität zu diesen aufweist, und wobei das Polypeptid die Funktion des Reduzierens oder Reduzierens und Rekombinierens weiblicher Sporozyten, weiblicher Gametophyten oder weiblicher Gameten während der Meiose hat;
(b) einem Polypeptid, umfassend eine Aminosäuresequenz, die zu SEQ ID NOs: 1, 2, 9, 14, 16, 20, 24, 25 und 27 oder einem Ortholog davon identisch ist oder mindestens 90 % Identität zu diesen aufweist, und wobei das Polypeptid die Funktion des Reduzierens oder Reduzierens und Rekombinierens weiblicher Sporozyten, weiblicher Gametophyten oder weiblicher Gameten während der Meiose hat;
(c) einem Polypeptid, umfassend eine Aminosäuresequenz ausgewählt aus SEQ ID NOs: 1, 2, 9, 14, 16, 20, 24, 25 oder 27 oder einem Ortholog davon; und
(d) einem Polypeptid, umfassend eine Aminosäuresequenz, die ein Fragment ist, das mindestens 150 zusammenhängende Aminosäuren der Aminosäuresequenz von (a), (b) oder (c) umfasst, und wobei das Polypeptid die Funktion des Reduzierens oder Reduzierens und Rekombinierens weiblicher Sporozyten, weiblicher Gametophyten oder weiblicher Gameten während der Meiose hat.

16. Expressionskassette, die in funktionsfähiger Verknüpfung mit einer interessierenden heterologen Nukleotidsequenz ein Nukleinsäuremolekül umfasst, das ein Polynukleotid umfasst, ausgewählt aus der Gruppe bestehend aus:
(a) einer Polynukleotidsequenz, umfassend die Polynukleotidsequenz SEQ ID NOs: 11, 15, 19, 21, 23 und 26;
(b) einer Polynukleotidsequenz, umfassend ein Fragment, das mindestens 16 zusammenhängende Nukleotide der Polynukleotidsequenz von SEQ ID NOs: 11, 15, 19, 21, 23 und 26 umfasst, wobei die Polynukleotidsequenz die Transkription in einer Pflanzenzelle initiiert;
(c) einem Polynukleotid, das zu dem Polynukleotid von (a) oder (b) komplementär ist; und
(d) einem Polynukleotid, das unter stringenten Bedingungen an das Polynukleotid von (a), (b) oder (c) hybridisiert;
wobei das Polynukleotid die Transkription in einer Zelle eines Samenanlagen-Primordiums, einer Samenanlage, eines weiblichen Sporozyten, eines weiblichen Gametophyten oder eines weiblichen Gameten initiiert.

17. Pflanze, umfassend die Expressionskassette nach Anspruch 16.

18. Expressionskassette nach Anspruch 16 oder Pflanze nach Anspruch 17, wobei:
(a) es sich bei der Pflanze um Mais, Weizen, Reis, Sorghum, Gerste, Hafer, Rasengras, Roggen, Sojabohne, Kohl, Sonnenblume, Hirse, Zuckerrohr, Baumwolle, Saflor, Tabak oder Luzerne; oder einen transgenen Samen dieser Pflanze handelt; oder
(b) die interessierende heterologe Nukleotidsequenz ein Genprodukt umfasst, das Nicht-Reduktion, Nicht-Rekombination, Parthenogenese oder eine beliebige Kombination von diesen verleiht.

19. Verfahren zur Expression einer Polynukleotidsequenz in einer Pflanze oder einer Pflanzenzelle, wobei das Verfahren das Einbringen einer Expressionskassette, umfassend einen Promotor, der funktionsfähig mit einer interessierenden Polynukleotidsequenz verknüpft ist, in die Pflanze oder die Pflanzenzelle mittels Transformation umfasst, wobei der Promotor eine der Polynukleotidsequenzen nach Anspruch 16 umfasst.

20. Verfahren nach Anspruch 19, wobei die interessierende Polynukleotidsequenz ein Genprodukt umfasst, das Nicht-Reduktion, Nicht-Rekombination, Parthenogenese oder eine beliebige Kombination von diesen verleiht.

21. Expressionskassette, umfassend einen heterologen Promotor, der funktionsfähig verknüpft ist mit einem Polynukleotid, umfassend ein Mitglied ausgewählt aus der Gruppe bestehend aus:
(a) einem Polynukleotid, das das Polypeptid von SEQ ID NOs: 1, 2, 9, 14, 16, 20, 24, 25 oder 27 codiert;
(b) einem Polynukleotid, das die in SEQ ID NOs: 3, 4, 5, 6, 7, 8, 10, 12, 13, 17, 18, 22, 28 oder 29 dargelegte Sequenz umfasst;
(c) einem Polynukleotid mit einer Länge von mindestens 300 Nukleotiden, das unter stringenten Bedingungen an ein Polynukleotid von (a) oder (b) hybridisiert, wobei die Bedingungen die Hybridisierung in 40 bis 45 % Formamid, 1 M NaCl, 1 % SDS bei 37°C und eine Waschung in 0,5 X bis 1 X SSC bei 55 bis 60°C umfassen;
(d) einem Polynukleotid mit mindestens 80 % Sequenzidentität zu den SEQ ID NOs: 3, 4, 5, 6, 7, 8, 10, 12, 13, 17, 18, 22, 28 oder 29, wobei die % Sequenzidentität auf der gesamten codierenden Region basiert und mittels BLAST 2.0 unter Standardparametern bestimmt wird, wobei das Polynukleotid ein Polypeptid codiert, das die Funktion des Reduzierens oder Reduzierens und Rekombinierens weiblicher Sporozyten, weiblicher Gametophyten oder weiblicher Gameten während der Meiose verleiht; und
(e) einem Polynukleotid, das zu einem Polynukleotid von einem von (a) bis (d) vollständig komplementär ist.

22. Isoliertes Polypeptid, ausgewählt aus der Gruppe bestehend aus:
(a) einem Polypeptid, umfassend eine der SEQ ID NOs: 9, 14, 16, 20, 24, 25 oder 27, wobei das Polypeptid die Funktion des Reduzierens oder Reduzierens und Rekombinierens weiblicher Sporozyten, weiblicher Gametophyten oder weiblicher Gameten während der Meiose verleiht;
(b) einem Polypeptid, das mindestens 80 % zu der Aminosäuresequenz einer der SEQ ID NOs: 9, 14, 16, 20, 24, 25 oder 27 identisch ist, wobei das Polypeptid die Funktion des Reduzierens oder Reduzierens und Rekombinierens weiblicher Sporozyten, weiblicher Gametophyten oder weiblicher Gameten während der Meiose verleiht;
(c) einem Polypeptid, das von einem Nukleinsäuremolekül codiert wird, umfassend eine Nukleotidsequenz, die mindestens 80 % zu einer der SEQ ID NOs: 8, 10, 12, 17, 18, 28 oder 29 oder einem Komplement davon, identisch ist, wobei das Polypeptid die Funktion des Reduzierens oder Reduzierens und Rekombinierens weiblicher Sporozyten, weiblicher Gametophyten oder weiblicher Gameten während der Meiose verleiht;
(d) einem Polypeptid, das von einem Nukleinsäuremolekül codiert wird, das mit einer Nukleinsäuresonde, bestehend aus der Nukleotidsequenz einer der SEQ ID NOs: 8, 10, 12, 17, 18, 28 oder 29 oder einem Komplement davon, nach mindestens einer Waschung in 0,2X SSC bei 55°C für 20 Minuten hybridisiert, wobei das Polypeptid die Funktion des Reduzierens oder Reduzierens und Rekombinierens weiblicher Sporozyten, weiblicher Gametophyten oder weiblicher Gameten während der Meiose verleiht; und
(e) einem Fragment, das mindestens 150 zusammenhängende Aminosäuren einer der SEQ ID NOs: 9, 14, 16, 20, 24, 25 oder 27 umfasst, wobei das Polypeptid die Funktion des Reduzierens oder Reduzierens und Rekombinierens weiblicher Sporozyten, weiblicher Gametophyten oder weiblicher Gameten während der Meiose verleiht.

23. Pflanzenzelle, die mit der Expressionskassette nach Anspruch 21 transformiert ist, oder Pflanze, die aus der Pflanzenzelle regeneriert wurde, wobei die Pflanze die Expressionskassette umfasst.

24. Pflanze nach Anspruch 23, wobei es sich bei der Pflanze um Mais, Weizen, Reis, Sorghum, Gerste, Hafer, Rasengras, Roggen, Sojabohne, Kohl, Sonnenblume, Hirse, Zuckerrohr, Baumwolle, Saflor, Tabak oder Luzerne handelt; oder ein Samen von dieser Pflanze.

25. Verfahren zur Erhöhung der Expression eines oder mehrerer Nrf4-Gene in einer Pflanzenzelle durch Einbringen in die Pflanze, mittels Transformation:
(A) eines Polynukleotids, umfassend ein Mitglied ausgewählt aus der Gruppe bestehend aus:
(a) einem Polynukleotid, das das Polypeptid von SEQ ID NOs: 1, 2, 9, 14, 16, 20, 24, 25 oder 27 codiert;
(b) einem Polynukleotid, das die in SEQ ID NOs: 3, 4, 5, 6, 7, 8, 10, 12, 13, 17, 18, 22, 28 oder 29 dargelegte Sequenz umfasst;
(c) einem Polynukleotid mit einer Länge von mindestens 300 Nukleotiden, das unter stringenten Bedingungen an ein Polynukleotid von (a) oder (b) hybridisiert, wobei die Bedingungen die Hybridisierung in 40 bis 45 % Formamid, 1 M NaCl, 1 % SDS bei 37°C und eine Waschung in 0,5 X bis 1 X SSC bei 55 bis 60°C umfassen;
(d) einem Polynukleotid mit mindestens 80 % Sequenzidentität zu den SEQ ID NOs: 3, 4, 5, 6, 7, 8, 10, 12, 13, 17, 18, 22, 28 oder 29, wobei die % Sequenzidentität auf der gesamten codierenden Region basiert und mittels BLAST 2.0 unter Standardparametern bestimmt wird, wobei das Polynukleotid ein Polypeptid codiert, das die Funktion des Reduzierens oder Reduzierens und Rekombinierens weiblicher Sporozyten, weiblicher Gametophyten oder weiblicher Gameten während der Meiose verleiht; und
(e) einem Polynukleotid, das zu einem Polynukleotid von einem von (a) bis (d) vollständig komplementär ist;
(B) eines der Polypeptide nach Anspruch 22; oder
(C) der Expressionskassette nach Anspruch 21.

## Revendications

1. Procédé de production d'un végétal, le procédé comprenant :
la perturbation par édition du génome, marquage par transposon ou mutagenèse d'un gène *Nrf4* endogène dans un végétal ou une cellule végétale, la cellule végétale étant un primordia d'ovule, un ovule, un gamétophyte femelle ou un gamète femelle, produisant ainsi des gamètes viables non réduits ou des gamètes viables non réduits et non recombinés ; et
la régénération d'un végétal ayant la perturbation du gène *Nrf4* endogène :
(A) le gène *Nrf4* endogène comprenant un polynucléotide choisi dans le groupe constitué par :
(a) un polynucléotide ayant au moins 80 % d'identité de séquence, comme déterminé par l'algorithme GAP sous des paramètres par défaut, par rapport à la séquence de pleine longueur d'un polynucléotide choisi dans le groupe constitué par les SEQ ID NO: 3, 4, 5, 6, 7, 8, 10, 12, 13, 17, 18, 22, 28 et 29, et le polynucléotide codant pour un polypeptide qui possède la fonction de réduire, ou de réduire et recombiner, des sporocytes femelles, des gamétophytes femelles ou des gamètes femelles pendant la méiose ;
(b) un polynucléotide ayant au moins 90 % d'identité de séquence, comme déterminé par l'algorithme GAP sous des paramètres par défaut, par rapport à la séquence de pleine longueur d'un polynucléotide choisi dans le groupe constitué par les SEQ ID NO: 3, 4, 5, 6, 7, 8, 10, 12, 13, 17, 18, 22, 28 et 29, et le polynucléotide codant pour un polypeptide qui possède la fonction de réduire, ou de réduire et recombiner, des sporocytes femelles, des gamétophytes femelles ou des gamètes femelles pendant la méiose ;
(c) un polynucléotide choisi dans le groupe constitué par les SEQ ID NO: 3, 4, 5, 6, 7, 8, 10, 12, 13, 17, 18, 22, 28 et 29 ;
(d) un polynucléotide qui est un fragment comprenant au moins 450 nucléotides contigus du polynucléotide de (a), (b) ou (c), et, le polynucléotide codant pour un polypeptide qui possède la fonction de réduire, ou de réduire et recombiner, des sporocytes femelles, des gamétophytes femelles ou des gamètes femelles pendant la méiose ; ou
(B) le gène *Nrf4* endogène codant pour un polypeptide Nrf4 choisi dans le groupe constitué par :
(a) un polypeptide comprenant une séquence d'acides aminés qui est identique aux ou possède au moins 80 % d'identité avec les SEQ ID NO: 1, 2, 9, 14, 16, 20, 24, 25 ou 27, ou un orthologue correspondant, et le polypeptide ayant la fonction de réduire, ou de réduire et recombiner, des sporocytes femelles, des gamétophytes femelles ou des gamètes femelles pendant la méiose ;
(b) un polypeptide comprenant une séquence d'acides aminés qui est identique aux ou possède au moins 90 % d'identité avec les SEQ ID NO: 1, 2, 9, 14, 16, 20, 24, 25 ou 27, ou un orthologue correspondant, et le polypeptide ayant la fonction de réduire, ou de réduire et recombiner, des sporocytes femelles, des gamétophytes femelles ou des gamètes femelles pendant la méiose ;
(c) un polypeptide comprenant une séquence d'acides aminés choisie parmi les SEQ ID NO: 1, 2, 9, 14, 16, 20, 24, 25 ou 27, ou un orthologue correspondant ; et
(d) un polypeptide comprenant une séquence d'acides aminés qui est un fragment comprenant au moins 150 acides aminés contigus de la séquence d'acides aminés de (a), (b) ou (c), et, le polypeptide ayant la fonction de réduire, ou de réduire et recombiner, des sporocytes femelles, des gamétophytes femelles ou des gamètes femelles pendant la méiose.

2. Procédé selon la revendication 1 :
(I) ladite perturbation inhibant l'expression, l'activité ou à la fois l'expression et l'activité d'un produit dudit gène *Nrf4* endogène par comparaison avec un végétal témoin correspondant dépourvu d'une telle perturbation ;
(II) comprenant en outre une étape de perturbation dans le végétal, en plus du gène *Nrf4,* d'au moins un autre gène impliqué dans la recombinaison ; ou
(III) comprenant en outre une étape de perturbation dans le végétal, en plus du gène *Nrf4,* d'au moins un autre gène endogène impliqué dans la méiose.

3. Procédé selon la revendication 2, l'au moins un autre gène endogène impliqué dans la méiose étant *REC8* ou un orthologue correspondant.

4. Procédé selon la revendication 3, comprenant en outre :
(i) la perturbation d'un des gènes suivants choisis dans le groupe constitué par *SPO11, PRD1, PRD2, PRD3, DF01,* et de quelconques orthologues correspondants ; ou
(ii) la perturbation de *OSD1* ou *TAM1,* ou d'orthologues correspondants, ou d'une quelconque combinaison correspondante.

5. Procédé selon l'une quelconque des revendications 1 à 4, le gamétophyte femelle ou le gamète femelle subissant une parthénogenèse ou une élimination du génome.

6. Procédé selon la revendication 5, comprenant en outre la production d'une graine, la graine comprenant des embryons clonaux issus parthénogénétiquement.

7. Procédé selon la revendication 1 :
(a) le végétal étant le maïs, le blé, le riz, le sorgho, l'orge, l'avoine, le gazon, le seigle, le soja, Brassica, ou le tournesol ; ou
(b) le végétal comportant le gène *Nrf4* endogène perturbé comprenant des gamètes, viables non réduits, viables ou non réduits et non recombinés, par comparaison avec un végétal témoin.

8. Procédé selon l'une quelconque des revendications 1 à 7, le végétal produit par ledit procédé étant un végétal non d'origine naturelle avec ovules en développement comprenant des gamètes femelles clonaux non réduits ou non réduits et non recombinés.

9. Construction d'acide nucléique comprenant une séquence de nucléotides codant pour un élément qui étouffe l'activité de *Nrf4* dans une cellule végétale choisie dans le groupe constitué par un primordia d'ovule, un ovule, un gamétophyte femelle, ou un gamète femelle ;
(A) l'élément comprenant au moins 19 nucléotides de l'une quelconque parmi les SEQ ID NO: 3, 4, 5, 6, 7, 8, 10, 12, 13, 17, 18, 22, 28 et 29 ou un complément correspondant ; ou
(B) l'élément comprenant une séquence de nucléotides qui s'hybride dans des conditions stringentes à un complément de pleine longueur d'une séquence de nucléotides de l'une quelconque parmi les SEQ ID NO: 3, 4, 5, 6, 7, 8, 10, 12, 13, 17, 18, 22, 28 et 29 ;
la construction comprenant en outre un promoteur hétérologue fonctionnel dans des végétaux lié de manière fonctionnelle à l'élément.

10. Construction d'acide nucléique selon la revendication 9, l'élément étant lié de manière fonctionnelle à un promoteur dans l'orientation sens ou dans l'orientation antisens.

11. Construction d'acide nucléique selon la revendication 9 ou 10, le promoteur étant un promoteur constitutif, inductible, préféré par une cellule ou un tissu, ou spécifique à une cellule ou un tissu.

12. Cellule végétale transformée par la construction selon la revendication 9 ; ou végétal comprenant ladite cellule végétale.

13. Cellule végétale ou végétal selon la revendication 12,
(a) le végétal comprenant des gamètes viables non réduits, ou non réduits et non recombinés, par comparaison avec un végétal témoin ; ou
(b) le végétal étant Arabidopsis, le maïs, le blé, le riz, le sorgho, l'orge, l'avoine, le gazon, le seigle, le soja, Brassica, le tournesol, le millet, la canne à sucre, le coton, le carthame, le tabac ou l'alfalfa ;
ou graine provenant dudit végétal.

14. Procédé de diminution de l'expression d'un ou plusieurs gènes *Nrf4* dans le végétal par introduction dans le végétal par transformation de la construction d'acide nucléique selon l'une quelconque des revendications 9 à 11, l'expression diminuée du ou des gènes *Nrf4* étant dans un primordia d'ovule, un ovule, un sporocyte femelle, un gamétophyte femelle ou un gamète femelle du végétal.

15. Procédé selon la revendication 14,
(I) le ou les gènes *Nrf4* étant endogènes au végétal ;
(II) le gène *Nrf4* comprenant un polynucléotide choisi dans le groupe constitué par :
(a) un polynucléotide ayant au moins 80 % d'identité de séquence, comme déterminé par l'algorithme GAP sous des paramètres par défaut, par rapport à la séquence de pleine longueur d'un polynucléotide choisi dans le groupe constitué par les SEQ ID NO: 3, 4, 5, 6, 7, 8, 10, 12, 13, 17, 18, 22, 28 et 29, et le polynucléotide codant pour un polypeptide qui possède la fonction de réduire, ou de réduire et recombiner, des sporocytes femelles, des gamétophytes femelles ou des gamètes femelles pendant la méiose ;
(b) un polynucléotide ayant au moins 90 % d'identité de séquence, comme déterminé par l'algorithme GAP sous des paramètres par défaut, par rapport à la séquence de pleine longueur d'un polynucléotide choisi dans le groupe constitué par les SEQ ID NO: 3, 4, 5, 6, 7, 8, 10, 12, 13, 17, 18, 22, 28 et 29, et le polynucléotide codant pour un polypeptide qui possède la fonction de réduire, ou de réduire et recombiner, des sporocytes femelles, des gamétophytes femelles ou des gamètes femelles pendant la méiose ;
(c) un polynucléotide choisi dans le groupe constitué par les SEQ ID NO: 3, 4, 5, 6, 7, 8, 10, 12, 13, 17, 18, 22, 28 et 29 ;
(d) un polynucléotide qui est un fragment comprenant au moins 450 nucléotides contigus du polynucléotide de (a), (b) ou (c), et, le polynucléotide codant pour un polypeptide qui possède la fonction de réduire, ou de réduire et recombiner, des sporocytes femelles, des gamétophytes femelles ou des gamètes femelles pendant la méiose ;
(e) un polynucléotide qui est complémentaire au polynucléotide de (a), (b), (c), ou (d) ; et
(f) un polynucléotide qui s'hybride au polynucléotide de (a), (b), (c), (d), ou (e) ; ou
(III) le gène *Nrf4* endogène codant pour un polypeptide choisi dans le groupe constitué par :
(a) un polypeptide comprenant une séquence d'acides aminés qui est identique aux ou possède au moins 80 % d'identité avec les SEQ ID NO: 1, 2, 9, 14, 16, 20, 24, 25 et 27, ou un orthologue correspondant, et le polypeptide ayant la fonction de réduire, ou de réduire et recombiner, des sporocytes femelles, des gamétophytes femelles ou des gamètes femelles pendant la méiose ;
(b) un polypeptide comprenant une séquence d'acides aminés qui est identique aux ou possède au moins 90 % d'identité avec les SEQ ID NO: 1, 2, 9, 14, 16, 20, 24, 25 et 27, ou un orthologue correspondant, et le polypeptide ayant la fonction de réduire, ou de réduire et recombiner, des sporocytes femelles, des gamétophytes femelles ou des gamètes femelles pendant la méiose ;
(c) un polypeptide comprenant une séquence d'acides aminés choisie parmi les SEQ ID NO: 1, 2, 9, 14, 16, 20, 24, 25 et 27, ou un orthologue correspondant ; et
(d) un polypeptide comprenant une séquence d'acides aminés qui est un fragment comprenant au moins 150 acides aminés contigus de la séquence d'acides aminés de (a), (b) ou (c), et, le polypeptide ayant la fonction de réduire, ou de réduire et recombiner, des sporocytes femelles, des gamétophytes femelles ou des gamètes femelles pendant la méiose.

16. Cassette d'expression comprenant en liaison fonctionnelle à une séquence de nucléotides hétérologue d'intérêt, une molécule d'acide nucléique comprenant un polynucléotide choisi dans le groupe constitué par :
(a) une séquence de polynucléotide comprenant la séquence de polynucléotide parmi les SEQ ID NO: 11, 15, 19, 21, 23 et 26 ;
(b) une séquence de polynucléotide comprenant un fragment comprenant au moins 16 nucléotides contigus de la séquence de polynucléotide parmi les SEQ ID NO: 11, 15, 19, 21, 23 et 26, la séquence de polynucléotide initiant la transcription dans une cellule végétale ;
(c) un polynucléotide qui est complémentaire au polynucléotide de (a) ou (b) ; et
(d) un polynucléotide qui s'hybride dans des conditions stringentes au polynucléotide de (a), (b) ou (c) ;
ledit polynucléotide initiant la transcription dans une cellule d'un primordia d'ovule, d'un ovule, d'un sporocyte femelle, d'un gamétophyte femelle ou d'un gamète femelle.

17. Végétal comprenant la cassette d'expression selon la revendication 16.

18. Cassette d'expression selon la revendication 16 ou végétal selon la revendication 17,
(a) le végétal étant le maïs, le blé, le riz, le sorgho, l'orge, l'avoine, le gazon, le seigle, le soja, Brassica, le tournesol, le millet, la canne à sucre, le coton, le carthame, le tabac ou l'alfalfa ; ou une graine transgénique dudit végétal ; ou
(b) la séquence de nucléotides hétérologue d'intérêt comprenant un produit génique qui confère une non réduction, une non recombinaison, une parthénogenèse, ou une quelconque combinaison correspondante.

19. Procédé pour l'expression d'une séquence de polynucléotide dans un végétal ou une cellule végétale, ledit procédé comprenant l'introduction dans le végétal ou la cellule végétale par transformation, d'une cassette d'expression comprenant un promoteur lié de manière fonctionnelle à une séquence de polynucléotide d'intérêt, ledit promoteur comprenant l'une quelconque des séquences de polynucléotide selon la revendication 16.

20. Procédé selon la revendication 19, la séquence de polynucléotide d'intérêt comprenant un produit génique qui confère une non réduction, une non recombinaison, une parthénogenèse, ou une quelconque combinaison correspondante.

21. Cassette d'expression comprenant un promoteur hétérologue lié de manière fonctionnelle à un polynucléotide comprenant un élément choisi dans le groupe constitué par :
(a) un polynucléotide qui code pour le polypeptide de la SEQ ID NO: 1, 2, 9, 14, 16, 20, 24, 25 ou 27 ;
(b) un polynucléotide comprenant la séquence indiquée dans les SEQ ID NO: 3, 4, 5, 6, 7, 8, 10, 12, 13, 17, 18, 22, 28 ou 29 ;
(c) un polynucléotide comprenant au moins 300 nucléotides de longueur qui s'hybride dans des conditions stringentes à un polynucléotide de (a) ou (b), les conditions comprenant une hybridation dans 40 à 45 % de formamide, NaCl 1 M, 1 % de SDS à 37 °C et un lavage dans 0,5 X à 1 X SSC à une température de 55 à 60 °C ;
(d) un polynucléotide ayant au moins 80 % d'identité de séquence avec les SEQ ID NO: 3, 4, 5, 6, 7, 8, 10, 12, 13, 17, 18, 22, 28 ou 29, le % d'identité de séquence étant basé sur la région codante entière et étant déterminé par BLAST 2.0 sous des paramètres par défaut, le polynucléotide codant pour un polypeptide qui confère la fonction de réduire, ou de réduire et recombiner, des sporocytes femelles, des gamétophytes femelles ou des gamètes femelles pendant la méiose ; et
(e) un polynucléotide entièrement complémentaire à un polynucléotide de l'un quelconque parmi (a) à (d).

22. Polypeptide isolé choisi dans le groupe constitué par :
(a) un polypeptide comprenant l'une quelconque parmi les SEQ ID NO: 9, 14, 16, 20, 24, 25 ou 27, ledit polypeptide conférant la fonction de réduire, ou de réduire et recombiner, des sporocytes femelles, des gamétophytes femelles ou des gamètes femelles pendant la méiose ;
(b) un polypeptide qui est au moins 80 % identique à la séquence d'acides aminés de l'une quelconque parmi les SEQ ID NO: 9, 14, 16, 20, 24, 25 ou 27, ledit polypeptide conférant la fonction de réduire, ou de réduire et recombiner, des sporocytes femelles, des gamétophytes femelles ou des gamètes femelles pendant la méiose ;
(c) un polypeptide qui est codé par une molécule d'acide nucléique comprenant une séquence de nucléotides qui est au moins 80 % identique à l'une quelconque parmi les SEQ ID NO: 8, 10, 12, 17, 18, 28 ou 29 ou un complément correspondants, ledit polypeptide conférant la fonction de réduire, ou de réduire et recombiner, des sporocytes femelles, des gamétophytes femelles ou des gamètes femelles pendant la méiose ;
(d) un polypeptide qui est codé par une molécule d'acide nucléique qui s'hybride avec une sonde d'acide nucléique constituée de la séquence de nucléotides de l'une quelconque parmi les SEQ ID NO: 8, 10, 12, 17, 18, 28 ou 29, ou un complément correspondant à la suite d'au moins un lavage dans 0,2X SSC à 55 °C pendant 20 minutes, ledit polypeptide conférant la fonction de réduire, ou de réduire et recombiner, des sporocytes femelles, des gamétophytes femelles ou des gamètes femelles pendant la méiose ; et
(e) un fragment comprenant au moins 150 acides aminés consécutifs de l'une quelconque parmi les SEQ ID NO: 9, 14, 16, 20, 24, 25 ou 27, ledit polypeptide conférant la fonction de réduire, ou de réduire et recombiner, des sporocytes femelles, des gamétophytes femelles ou des gamètes femelles pendant la méiose.

23. Cellule végétale transformée par la cassette d'expression selon la revendication 21, ou végétal régénéré à partir de ladite cellule végétale, ledit végétal comprenant la cassette d'expression.

24. Végétal selon la revendication 23, le végétal étant le maïs, le blé, le riz, le sorgho, l'orge, l'avoine, le gazon, le seigle, le soja, Brassica, le tournesol, le millet, la canne à sucre, le coton, le carthame, le tabac ou l'alfalfa ; ou graine dudit végétal.

25. Procédé d'augmentation de l'expression d'un ou plusieurs gènes *Nrf4* dans une cellule végétale par introduction dans le végétal par transformation :
(A) d'un polynucléotide comprenant un élément choisi dans le groupe constitué par :
(a) un polynucléotide qui code pour le polypeptide de la SEQ ID NO: 1, 2, 9, 14, 16, 20, 24, 25 ou 27 ;
(b) un polynucléotide comprenant la séquence indiquée dans les SEQ ID NO: 3, 4, 5, 6, 7, 8, 10, 12, 13, 17, 18, 22, 28 ou 29 ;
(c) un polynucléotide comprenant au moins 300 nucléotides de longueur qui s'hybride dans des conditions stringentes à un polynucléotide de (a) ou (b), les conditions comprenant une hybridation dans 40 à 45 % de formamide, NaCl 1 M, 1 % de SDS à 37 °C et un lavage dans 0,5 X à 1 X SSC à une température de 55 à 60 °C ;
(d) un polynucléotide ayant au moins 80 % d'identité de séquence avec les SEQ ID NO: 3, 4, 5, 6, 7, 8, 10, 12, 13, 17, 18, 22, 28 ou 29, le % d'identité de séquence étant basé sur la région codante entière et étant déterminé par BLAST 2.0 sous des paramètres par défaut, le polynucléotide codant pour un polypeptide qui confère la fonction de réduire, ou de réduire et recombiner, des sporocytes femelles, des gamétophytes femelles ou des gamètes femelles pendant la méiose ; et
(e) un polynucléotide entièrement complémentaire à un polynucléotide de l'un quelconque parmi (a) à (d)
(B) d'un quelconque parmi les polypeptides selon la revendication 22 ; ou
(C) de la cassette d'expression selon la revendication 21.
